# EUROPEAN PATENT APPLICATION

(11) **EP 3 012 326 A1**
(43) Date of publication of application: **27.04.2016**
(21) Application number: 14775157.2
(22) Date of filing: 26.03.2014
(51) Int. Cl.: C12Q 1/68, C12N 15/09

(54) **PRIMER AND PROBE SET USED IN IDENTIFYING GENE POLYMORPHISM, AND USE THEREOF**

(30) Priority: 26.03.2013 JP 2013064226
(71) Applicant: Nippon Gene Co., Ltd, Tokyo 10110054 (JP)
(72) Inventor: KITANI, Masakazu, Toyama-shi Toyama 930-0982 (JP); MAKI, Fuminori, Toyama-shi Toyama 930-0982 (JP); IZAWA, Masaki, Toyama-shi Toyama 930-0982 (JP); KANAYAMA, Shinji, Toyama-shi Toyama 930-0834 (JP); YONEDA, Yuko, Toyama-shi Toyama 930-0982 (JP)
(74) Representative: Vossius & Partner Patentanwälte Rechtsanwälte mbB
(86) International application number: PCT/JP2014/058624
(87) International publication number: WO 2014/157377

(57) **Abstract**

By establishing a simple method for extracting a nucleic acid from a human specimen, and using the LAMP method, which is an isothermal gene amplification method showing superior quickness and convenience, with a concept of measurement different from the conventional ones, there is established a test method including steps up to detection with a measurement apparatus. There is provided a method for detecting a target site, which is an LAMP method using four kinds of primers, FIP, F3 primer, BIP, and B3 primer, which are designed on the basis of six regions of a template polynucleotide, F1 region, F2 region, F3 region, B1 region, B2 region, and B3 region, wherein: the template polynucleotide contains the target site, the four kinds of primers are designed so that the target site exists between the F1 region and the F2 region, or between the B1 region and the B2 region; a loop primer designed on the basis of a region between the F 1 region and the F2 region or between the B 1 region and the B2 region on the side on which the target site exists, and an oligonucleotide probe that can associate with a region including the target site are used, the loop primer and the probe are designed so that the 5' end of the loop primer and the 3' end of the probe associate with the template polynucleotide at positions close to each other, one of the loop primer and the probe is modified with a fluorescent molecule around the 5' end in the case of the loop primer or the 3' end in the case of the probe, and the other is modified with a quenching molecule.

## Description

### Technical Field

The present invention relates to detection of a specific gene sequence. More precisely, the present invention relates to a set of primers and probes for detection of gene polymorphism, especially single nucleotide polymorphism (SNP), of, for example, human or an animal, a test method and test kit using it, as well as a measurement apparatus therefor.

### Background Art

In recent new drug development, a concept of individualized medicine is directed, in which optimal therapeutic policy is chosen for each patient by preliminarily inspecting gene information of a patient to be treated. As a part thereof, there is suggested importance of companion diagnostic drugs for preliminarily investigating so-called gene information for nucleic acids and proteins of a patient concerning efficacy and adverse reactions of new drugs. Namely, although therapeutic treatments and drugs given to patients with diseases have conventionally been determined for each disease to a certain extent, individual differences are observed in curative effects or adverse reactions thereof. Therefore, genetic type is identified for each patient so as to choose optimal therapeutic treatment and drug for each group of patients. Such a strategy is called individualized medicine, or it is also called tailor-made medicine.

Human viral hepatitis is a systemic infectious disease of which main lesion is formed in the liver due to infection of hepatitis virus and immunological response of host. Hepatitis C accounts for 70% of chronic liver diseases in Japan, and it is a serious disease that develops into hepatic cancer via hepatic cirrhosis.

At present, the three-agent combined therapy using peginterferon, ribavirin, and telaprevir is used for therapeutic treatment of human hepatitis C as the most effective therapeutic treatment, and it is considered that interferon induces expression of antiviral genes of cells to promote reduction of virus amount. However, about 70 to 80 percents of severe hepatitis C patients in Japan are infected with the hepatitis C virus type 1b, which causes intractable hepatitis, and it has been proven that the combined therapy using peginterferon and ribavirin is not effective for about 50 percents of such patients who have increased amount of the virus. On such a background, many researches have been conducted, and it has successively been reported in 2009 by Japanese and foreign research groups that gene polymorphisms (SNPs) relating to the efficacy exist in flanking regions of the human interleukin 28B (IL28B) gene (Patent document 1, Non-patent documents 1 to 4).

For example, it is known that, among the SNPs existing in the IL28B gene, type of rs8099917 (TT, TG, GG) is one of the potent factors for predicting effectiveness of the combined therapy using peginterferon and ribavirin. rs8099917 was discovered through massive analysis of gene information of Japanese hepatitis C patients based on GWAS (genome-wide association study). It has been found that the combined therapy using peginterferon and ribavirin is not effective on the group of patients having a risk allele of TG or GG (Patent document 2, Non-patent documents 5 and 6).

Therefore, by identifying SNP in advance, a patient for whom radical cure can be expected can be selected with high probability. Moreover, also for a patient for whom it is judged that high effect cannot be expected, the identified SNP can be used as an index for determination of treatment period and treatment policy. Furthermore, if it is judged that it is desirable for a patient to wait for a next-generation therapy on the basis of the SNP judgment, the patient can avoid unnecessary expenses or suffering from adverse drug reactions, and it is considered that this also leads to reduction of the national medical expenses (2 to 6 million yens per one person). The usefulness of rs8099917 is considered important also in Japan, and genetic screening before start of treatment is recommended in the Ministry of Health, Labor and Welfare "Guideline for treatment of chronic hepatitis B and C and hepatic cirrhosis", 2012, and the Japan Society of Hepatology, "Guideline for treatment of hepatitis C". These references describe that it is desirable to use also rs11881222 and rs8103142 in addition to rs8099917 for prediction of effectiveness of the combined therapy, and rs12979860 is also known to be a major factor for determination of effect of the therapy in various countries, although not so many Japanese people have that SNP.

Ribavirin used for the combined therapy is a purine nucleoside analogue, and hemolytic anemia is known as an adverse drug reaction thereof. As in the case of peginterferon, SNPs relevant to the adverse drug reaction such as rs1127354 and rs7270101 have been discovered also for this agent (Non-patent document 7).

For identification of SNP, in addition to PCR-RFLP (polymerase chain reaction-restriction fragment length polymorphism), the Exo-proofreading method, and the TDI method (template directed dye-terminator incorporation), analysis by the direct sequencing method (Non-patent document 8) has been used so far to date as a highly reliable method. Moreover, in recent years, in connection with the spread or PCR apparatuses that enable real time monitoring of fluorescence, the TaqMan method (Non-patent document 9), quenching probe method, hybridization probe method, molecular beacon method (Non-patent document 10), invader method, invader-plus method, and so forth are used by researchers, which are conducted by associating a third or fourth probe consisting of a nucleic acid labeled with a compound that emits fluorescence or causes quenching of fluorescence (Patent document 3), and different from the primers with a genetic region. As a method not using any probe, there is a method of identifying a target nucleotide by melting profile analysis using an intercalator that binds to a double-stranded nucleic acid.

Furthermore, there has also been developed a gene amplification method, in which four kinds of primers (FIP (Forward Inner Primer), F3 primer, BIP (Backward Inner Primer), and B3 primer) are set for six regions (F1, F2, F3, B1, B2, and B3) of a target gene, and the reaction is allowed at a constant temperature using a strand displacement reaction (Patent document 4, and Non-patent document 11). This method is called LAMP (Loop-Mediated Isothermal Amplification) method. This method further uses loop primers (loop primer F and loop primer B) having sequences complementary to the single-strand moieties (between the F1 region and the F2 region, and between the B1 region and the B2 region) of the loop on the 5' end side of the dumbbell structure of the amplification product to increase origin of DNA synthesis, and thereby enables quicker amplification (Non-patent document 12).

### Prior art references

### Patent documents

Patent document 1: Japanese Patent Unexamined Publication (Kokai) No. 2011-41526 (Japanese Patent Application No. 2009-192615)
Patent document 2: Japanese Patent Unexamined Publication (Kokai) No. 2011-193786 (Japanese Patent Application No. 2010-63622)
Patent document 3: Japanese Patent Unexamined Publication (Kokai) No. 05-211872 (Japanese Patent Application No. 3-7777)
Patent document 4: WO2000/028082

### Non-patent documents

Non-patent document 1: Enomoto N., Comparison of full length sequences of interferon-sensitive and resistant hepatitis C virus 1b - Sensitivity to interferon is conferred by amino acid substitutions in the NS5A region, J. Clin. Invest, 96, 224
(1995) Non-patent document 2: Akuta N., Association of amino acid substitution pattern in core protein of hepatitis C virus genotype 1b high viral load and non-virological response to interferon-ribavirin combination therapy, Intervirology, 48, 372 (2005)
Non-patent document 3: Dongliang Ge et al., Genetic variation in IL28B predicts hepatitis C treatment-induced viral clearance, Nature, 2009, 461:399-40
Non-patent Document 4: Yasuhito Tanaka et al., Genome-wide association of IL28B with response to pegylated interferon-α and ribavirin therapy for chronic hepatitis C, Nature Genetics, 2009;40 (Issue 10): 1105-1109
Non-patent document 5: Hidenori Ochi et al., ITPA Polymorphism Affects Ribavirin-Induced Anemia and Outcomes of Therapy - A Genome-Wide Study of Japanese HCV Virus Patients, Gastroenterology, 2010, 139:1190-1197
Non-patent document 6: Fumitaka Suzuki et al., Influence of ITPA Polymorphisms on Decreases of Hemoglobin During Treatment with Pegylated Interferon, Ribavirin, and Telaprevir, Hepatology, 2011;53:415-421
Non-patent document 7: Jacques Fellay et al., ITPA gene variants protect against anaemia in patients treated for chronic hepatitis C, Nature, 2010, 464:405-408
Non-patent document 8: Kwok, P.-Y., 1994, Comparative analysis of human DNA variations by fluorescence-based sequencing ofPCR products, Genomics, 23:138-144
Non-patent document 9: Livak, Allelic discrimination using fluorogenic probes and the 5' nuclease assay, Genetic Analysis, 14, 143-149 (1999)
Non-patent document 10: Belinda A.J. Giesendorf, Molecular beacons: a new approach for semiautomated mutation analysis, Clinical Chemistry, 44, 482-486 (1998)
Non-patent document 11: Notomi T., Loop-mediated isothermal amplification of DNA, Nucleic Acids Research, Vol. 28, No. 12, e63, 2000
Non-patent document 12: Nagamine K., Accelerated reaction by loop-mediated isothermal amplification using loop primers, Molecular and Cellular Probes, 16, 3, 223-229, 2002

### Summary of the Invention

### Object to be Achieved by the Invention

Among the conventional SNP identification methods, the PCR-RFLP method is useful. However, it requires, after obtaining a single PCR product, a restriction enzyme treatment and electrophoresis analysis. These operations require time, and erroneous determination due to design of the test and handling, and contamination of the test environment by the amplification product are concerned for this method. For both the Exo-proofreading method and TDI method, the amplification and detection must be performed with separate instruments, and sufficient effect could not be obtained. The method of using an intercalator to identify a target nucleotide on the basis of melting profile analysis without using a probe requires highly precise temperature control in order to identify difference of one nucleotide. Although the analysis using a microarray is technically matured, it requires instruments for exclusive use, knowledge, and experience.

In addition, for all the methods mentioned above, for example, highly accurate design of primers or probe is extremely important, and requires much labor. In addition, they essentially require PCR, and therefore require expensive instruments, and reagent kits. Thus, they cannot necessarily be considered methods suitable for routinely performed for the identification on clinical sites or at general practitioner level. Therefore, it is considered that many of the tests are entrusted to external specialized agencies, and such practice involves problems concerning protection of personal information, ethical procedures, time and cost required for them, and so forth.

### Means for Achieving the Object

The inventors of the present invention conducted various researches in order to develop a quick and convenient technique for detecting SNP, aiming mainly at promoting realization of individualized medicine using human gene information, and aiding development of companion diagnostic drugs. Specifically, by establishing a simple method for extracting a nucleic acid from a human specimen, and using the LAMP method, which is an isothermal gene amplification method showing superior quickness and convenience, with a concept of measurement different from the conventional ones, they established a test method including steps up to detection with a measurement apparatus. The results obtained by this technique agreed with the results obtained by the direct sequencing method as the conventional method, and thus accuracy thereof was demonstrated.

Specifically, the inventors of the present invention noted use of the 5' end of primer or *in vitro* transcription product that is to be the end of gene amplification product for identification of a target nucleotide by associating it with an amplification region. Namely, in the LAMP method, the loop primer is introduced into almost all of the 5' ends of the amplification products, and amplification products obtained by the LAMP method are most characterized in that they are replicated while ligating specific units of gene regions. Therefore, they estimated that a complementary strand that constituted base pairs with the loop primer was not necessarily contained in the 3' end thereof, and could form a complex higher structure, and by applying a specific energy transfer system to the 5' end of the loop primer that can be disposed on the amplification region, or an *in vitro* transcription product, they realized high sensitivity and detection efficiency without decreasing the amplification reaction rate, and thus accomplished the present invention.

The present invention provides the followings.
[1] An LAMP-FLP method, which is an LAMP method using four kinds of primers, FIP, F3 primer, BIP, and B3 primer, which are designed on the basis of six regions of a template polynucleotide, F1 region, F2 region, F3 region, B1 region, B2 region, and B3 region:
   wherein:
      the template polynucleotide contains a target site, the four kinds of primers are designed so that the target site exists between the F1 region and the F2 region, or between the B1 region and the B2 region;
      a loop primer designed on the basis of a region between the F1 region and the F2 region or between the B1 region and the B2 region on the side on which the target site exists, and an oligonucleotide probe that can associate with a region including the target site are used,
      the loop primer and the probe are designed so that the 5' end of the loop primer and the 3' end of the probe associate with the template polynucleotide at positions close to each other,
      one of the loop primer and the probe is modified with a fluorescent molecule around the 5' end in the case of the loop primer or the 3' end in the case of the probe, and the other is modified with a quenching molecule.
[2] The method according to [1], wherein the target site is designed so as to locate 3 to 20 nucleotides upstream from the 5' end of the associated loop primer.
[3] The method according to [1] or [2], wherein the target site is a single nucleotide polymorphism (SNP).
[4] The method according to [3], wherein SNP is selected from the group consisting of rs8099917, rs12978960, rs11881222, rs8103142, rs1127354, rs7270101, rs1229984, rs671, rs1800592, rs1042713, rs4994, rs1057910, rs9934438, and rs9923231.
[5] The method according to any one of [1] to [4], which comprises the step of changing temperature of system within a temperature range where association or dissociation of the probe occurs, and monitoring change of fluorescence occurring during the temperature change.
[6] The method according to [5], wherein, after an isothermal reaction, temperature of the system is raised and then lowered, during which occurring change of fluorescence is monitored.
[7] The method according to [5] or [6], which comprises the step of calculating ratio of change of fluorescence by primary differentiation.
[8] The method according to [7], which comprises the step of further differentiating a value of ratio of change of fluorescence calculated by the primary differentiation.
[9] The method according to any one of [1] to [8], wherein the probe is modified with the fluorescence molecule around the 3' end thereof, and the loop primer is modified with the quenching molecule around the 5' end thereof.
[10] The method according to any one of [1] to [9], wherein DNA contained in a sample obtained by treating a specimen derived from a living organism with a protease is used as the template polynucleotide.
[11] The method according to [10], wherein the sample is subjected to a treatment for inactivating the protease.
[12] The method according to [10] or [11], wherein the specimen is selected from the group consisting of hair, blood, oral mucosa, nail, bloodstain, and umbilical cord.
[13] The method according to any one of [1] to [12], wherein DNA synthetase used for the amplification reaction is a DNA polymerase I derived from a *Geobacillus* bacterium, or a Csa DNA polymerase or 96-7 DNA polymerase isolated from compost obtained by fermentation at a temperature of 85°C or higher.
[14] A kit for carrying out the method according to any one of [1] to [13], which comprises:
   four kinds of primers (FIP, F3 primer, BIP, and B3 primer) designed on the basis of six regions of a template polynucleotide (F1 region, F2 region, F3 region, B1 region, B2 region, and B3 region);
   a loop primer designed on the basis of a region between the F1 region and the F2 region or between the B1 region and the B2 region on the side on which a target site exists, and
   an oligonucleotide probe that can associate with a region including the target site, and
   wherein:
   the four kinds of the primers are designed so that the target site locates between the F1 region and the F2 region or between the B1 region and the B2 region;
   the loop primer and the probe are designed so that the 5' end of the loop primer and the 3' end of the probe associate with the template polynucleotide at positions close to each other; and
   one of the loop primer and the probe is modified with a fluorescent molecule around the 5' end in the case of the loop primer or the 3' end in the case of the probe, and the other is modified with a quenching molecule.
[15] The kit according to [14], which is for detecting rs8099917, and is used for predicting effect of the combined therapy of peginterferon and ribavirin in a therapeutic treatment of hepatitis C.
[16] A method for treating a specimen for a gene amplification reaction, which comprises the step of treating the specimen with a protease.
[17] The method according to [16], wherein the gene amplification reaction is performed by the PCR method, SDA method, TMA method, NASBA method, TRC method, ICAN method, SmartAmp method, or RCA method.

### Effect of the Invention

The present invention provides a quick, highly efficient and accurate genetic test, and it shows superior flexibility, and contributes to realization of individualized medicine and development of drugs and companion diagnostic agents. It also enables quick test on clinical sites or at general practitioner level. Furthermore, it can be used not only in the field of development of drugs and diagnostic agents, but also for prophylaxis of lifestyle-related diseases, giving indices for tobacco use and drinking, and so forth through providing information on body constitution on site.

### Brief Description of the Drawings

[Fig. 1] Fig. 1 shows an exemplary scheme for determining primer, probe, salt concentrations, etc. for favorably performing the LAMP-FLP method.
[Fig. 2] Fig. 2 shows IL28B rs8099917 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.
[Fig. 3-1] Fig. 3-1 shows IL28B rs8099917 typing based on the LAMP-FLP method. The left graph shows a curve obtained by plotting fluorescence intensity against temperature lowered at a rate of 0.05°C/second, and the right graph shows a curve obtained by primarily differentiating the obtained fluorescence intensity curve (left graph) for the temperature.
[Fig. 3-2] Fig. 3-2 shows IL28B rs8099917 typing based on the LAMP-FLP method. A template DNA artificially synthesized so as to have T as the SNP concerned was used (Major). The obtained fluorescence intensity curve was primarily differentiated for the temperature.
[Fig. 4] Fig. 4 shows IL28B rs12978960 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.

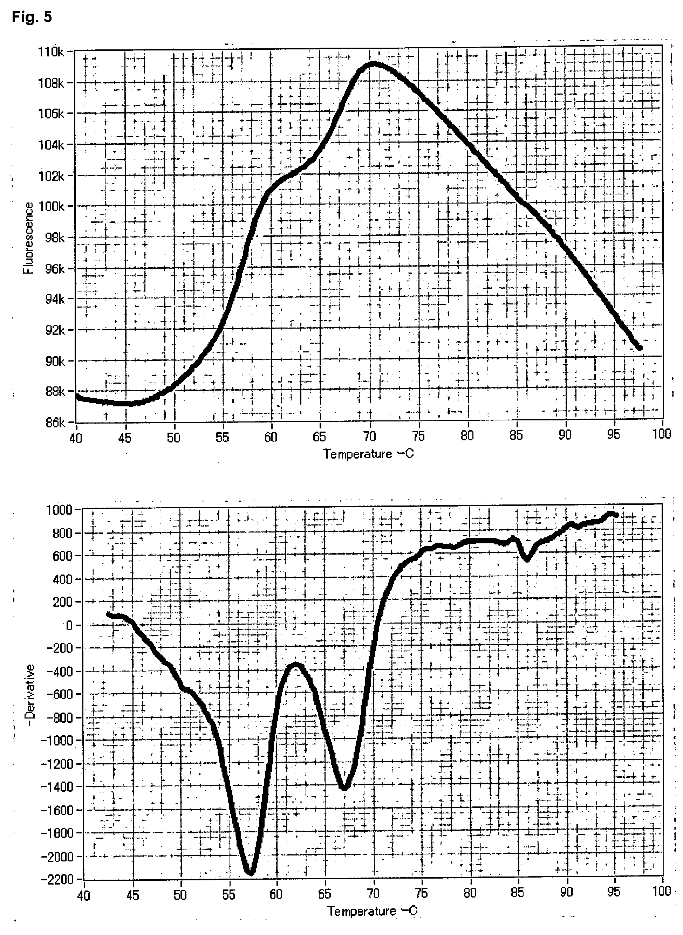

[Fig. 5] Fig. 5 shows IL28B rs12978960 typing based on the LAMP-FLP method. The left graph shows a curve obtained by plotting fluorescence intensity against temperature lowered at a rate of 0.05°C/second, and the right graph shows a curve obtained by primarily differentiating the obtained fluorescence intensity curve (left graph) for the temperature.
[Fig. 6] Fig. 6 shows IL28B rs11881222 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.
[Fig. 7] Fig. 7 shows IL28B rs11881222 typing based on the LAMP-FLP method. The left graph shows a curve obtained by plotting fluorescence intensity against temperature lowered at a rate of 0.05°C/second, and the right graph shows a curve obtained by primarily differentiating the obtained fluorescence intensity curve (left graph) for the temperature.
[Fig. 8] Fig. 8 shows IL28B rs8103142 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.
[Fig. 9] Fig. 9 shows IL28B rs8103142 typing based on the LAMP-FLP method. The left graph shows a curve obtained by plotting fluorescence intensity against temperature lowered at a rate of 0.05°C/second, and the right graph shows a curve obtained by primarily differentiating the obtained fluorescence intensity curve (left graph) for the temperature.
[Fig. 10] Fig. 10 shows ITPA rs1127354 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.
[Fig. 11] Fig. 11 shows ITPA rs1127354 typing based on the LAMP-FLP method. The left graph shows a curve obtained by plotting fluorescence intensity against temperature lowered at a rate of 0.05°C/second, and the right graph shows a curve obtained by primarily differentiating the obtained fluorescence intensity curve (left graph) for the temperature.
[Fig. 12] Fig. 12 shows ITPA rs7270101 typing based on the LAMP-QP method. The left graph shows a curve obtained by plotting fluorescence intensity against temperature raised at a rate of 0.2°C/second, and the right graph shows a curve obtained by primarily differentiating the obtained fluorescence intensity curve (left graph) for the temperature.
[Fig. 13] Fig. 13 shows ITPA rs7270101 typing based on the LAMP-FLP method. The left graph shows a curve obtained by plotting fluorescence intensity against temperature lowered at a rate af 0.05°C/second, and the right graph shows a curve obtained by primarily differentiating the obtained fluorescence intensity curve (left graph) for the temperature.
[Fig. 14] Fig. 14 shows comparison of performances of probes (5' end side portions were deleted).
[Fig. 15] Fig. 15 shows comparison of performances of probes (3' end side portions were deleted).
[Fig. 16] Fig. 16 shows investigation of region for which a probe can be designed.
[Fig. 17] Fig. 17 shows influence of mismatch.

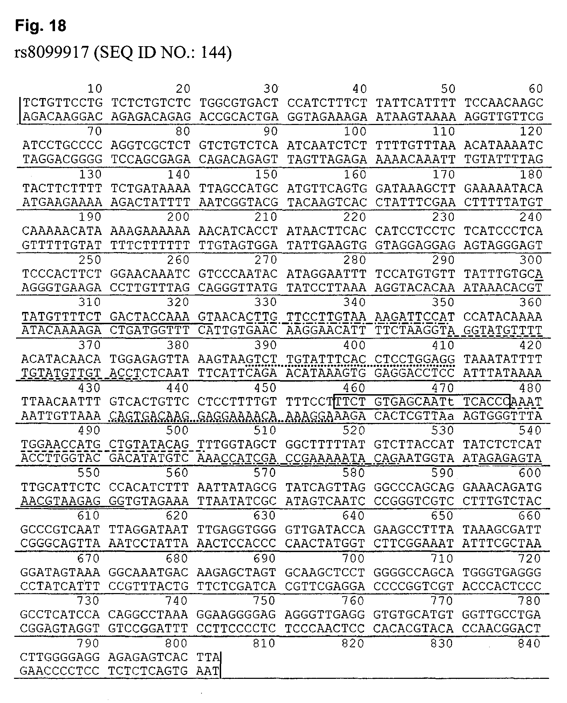

[Fig. 18] Fig. 18 shows positional relationship of the region artificially synthesized as a template DNA for rs8099917 typing, the regions used in the LAMP method, and the probe. The range between the vertical bars was cloned as the template DNA. The underlines indicate regions relevant to the primers. More precisely, the solid lines indicate the F3 region and the B3 region, the dashed dotted line indicates the F2 region or the B2 region, the dotted lines indicate the F1 region or the B1 region, and the dashed lines indicate the region with which the LF primer associates, or the region with which the LB primer associates. The box indicates the region with which the probe associates, and the small letter in the box indicates the target SNP (the same shall apply to the following drawings).
[Fig. 19] Fig. 19 shows positional relationship of the region artificially synthesized as a template DNA for rs12979860 typing, the regions used in the LAMP method, and the probe.
[Fig. 20] Fig. 20 shows positional relationship of the region artificially synthesized as a template DNA for rs11881222 typing, the regions used in the LAMP method, and the probe.
[Fig. 21] Fig. 21 shows positional relationship of the region artificially synthesized as a template DNA for rs8103142 typing, the regions used in the LAMP method, and the probe.
[Fig. 22] Fig. 22 shows positional relationship of the region artificially synthesized as a template DNA for rs1127354 typing, the regions used in the LAMP method, and the probe.
[Fig. 23] Fig. 23 shows positional relationship of the region artificially synthesized as a template DNA for rs7270101 typing, the regions used in the LAMP method, and the probe.
[Fig. 24] Fig. 24 shows positional relationship of the region artificially synthesized as a template DNA for ADH1B (human alcohol dehydrogenase 1B) rs1229984 typing, the regions used in the LAMP method, and the probe.
[Fig. 25] Fig. 25 shows positional relationship of the region artificially synthesized as a template DNA for ALDH2 (human aldehyde dehydrogenase 2) rs671 typing, the regions used in the LAMP method, and the probe.
[Fig. 26] Fig. 26 shows positional relationship of the region artificially synthesized as a template DNA for UCP1 (human uncoupling protein 1) rs1800592 typing, the regions used in the LAMP method, and the probe.
[Fig. 27] Fig. 27 shows positional relationship of the region artificially synthesized as a template DNA for ADRB2 (human adrenoreceptor beta2) rs1042713 typing, the regions used in the LAMP method, and the probe.
[Fig. 28] Fig. 28 shows positional relationship of the region artificially synthesized as a template DNA for ADRB3 (human adrenoreceptor beta3) rs4994 typing, the regions used in the LAMP method, and the probe.
[Fig. 29] Fig. 29 shows positional relationship of the region artificially synthesized as a template DNA for CYP2C9*3 (cytochrome p450 2c9*3) rs1057910 typing, the regions used in the LAMP method, and the probe.
[Fig. 30] Fig. 30 shows positional relationship of the region artificially synthesized as a template DNA for VKORC1 #1 (vitamin K epoxide reductase complex 1 #1) rs9934438 typing, the regions used in the LAMP method, and the probe.
[Fig. 31] Fig. 31 shows positional relationship of the region artificially synthesized as a template DNA for VKORC1 #2 (vitamin K epoxide reductase complex 1 #2) rs9923231 typing, the regions used in the LAMP method, and the probe.

### Modes for Carrying out the Invention

In the present invention, if a numerical range is represented as "m to n", the values of m and n are included in the range, unless especially indicated.

### [LAMP-FLP method]

According to the method of the present invention, in the LAMP (Loop-Mediated Isothermal Amplification) method, a loop primer and oligonucleotide probe modified with a fluorescent molecule or quenching molecule are used in addition to the four kinds of the conventional primers. The method of the present invention can be called LAMP-FLP method.

The LAMP method is an amplification method utilizing a strand displacement reaction with a combination of four kinds of primers for six regions selected from a sequence of a target gene. The primers used for the LAMP method are designed by using the six regions called F3 region, F2 region, F1 region, B1 region, B2 region, and B3 region from the 5' end side in the region as the object of amplification (in the present invention, it may also be referred to as "template (nucleotide or DNA)". The regions complementary to these six regions are referred to as F3c region, F2c region, F1c region, B1c region, B2c region, and B3c region, respectively. In the basic LAMP method, four kinds of primers, more precisely, two kinds of inner primers, i.e., FIP and BIP, and two kinds of outer primers, i.e., F3 primer and B3 primer, are used. The inner primers are constituted by ligating the F1 c (region complementary to the F1 region) and the F2 region, and the B1c region (region complementary to the B1 region) and B2 region. The LAMP method is generally characterized in that it does not require a reaction for denaturing a double strand into single strands, unlike the PCR method, the reaction advances at a constant temperature of 60 to 65°C, and it does not require such an apparatus as thermal cycler. Moreover, it also provides high amplification rate and high specificity.

The terms of F3 region, F2 region, F1 region, B1 region, B2 region, B3 region, FIP, F3 primer, BIP, and B3 primer used in the present invention have the same meanings as those of these terms used in the general LAMP method (see the following diagram), unless especially indicated.

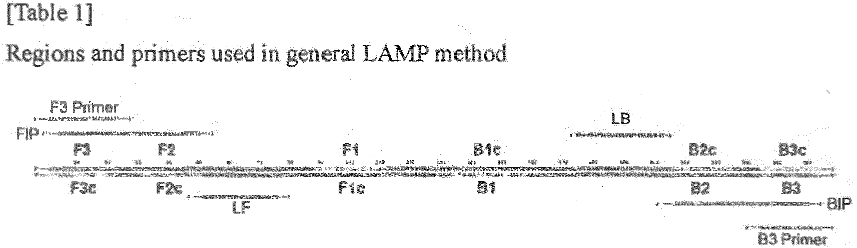

In the method of the present invention (also referred to as the LAMP-FLP method), the template polynucleotide contains a target site, and four kinds of the primers are designed so that the target site exists between the F1 region and the F2 region or between the B1 region and the B2 region. The "target site" referred to in the present invention is a site existing on the template nucleotide, and serving as the object of the detection according to the method of the present invention, unless especially indicated. Examples of the target site include single nucleotide polymorphism (SNP), and deletion, substitution, or addition of one or several nucleotides. The present invention may be explained for the case where the target site includes SNP, and the LAMP-FLP method of the present invention is performed in order to detect SNP as an example, but those skilled in the art can appropriately understand application of the same for detecting a sequence other than SNP.

In the LAMP-FLP method of the present invention, at least one loop primer is used. The LAMP method using a loop primer is well known to those skilled in the art. The loop primer is usually designed as one having a sequence complementary to a single strand moiety of the loop on the 5' end side of the dumbbell structure of the amplification product of the LAMP method (between the B1 region and the B2 region, or between the F1 region and the F2 region). Each primer is called loop primer B (LB) or loop primer F (LF) (refer to the above descriptions). By using the loop primer, origin of DNA synthesis can be increased. In a typical amplification product of the LAMP method having six loops, four loops are not used in the case of the original method using four primers. However, use of the loop primers enables use of all the loops. The term loop primer used in the present invention is used with the same meaning as that used in the general LAMP method using a loop primer, unless especially indicated.

At least one of loop primer is used in the present invention, and it is a loop primer designed on the basis of a region on the side on which the target site exists, among a region between F1 region and the F2 region or a region between the B1 region and the B2 region. In the present specification, explanations may be made by exemplifying the case where the target site exists in a region between the B1 region and the B2 region, but those skilled in the art can easily understand the case where the target site exists in a region between the F1 region and the F2 region by differently reading such explanations. According to the present invention, two or more loop primers may exist.

According to the present invention, an oligonucleotide probe that can associate with a region including the target site is also used. The probe is typically designed on the basis of a major allele or minor allele of the region including the target site. Therefore, in the LAMP-FLP method of the present invention, the difference of Tm values generated due to difference of full match and mismatch of the probe and the region with which the probe associates is revealed as difference of changes of fluorescence intensity as described later.

In the LAMP method of the present invention, the aforementioned at least one loop primer and the probe are designed so that the 5' end of the loop primer and the 3' end of the probe associate with the template polynucleotide at positions close to each other. The expression that the 5' end of the loop primer and the 3' end of the probe associate with the template polynucleotide "at positions close to each other" means that the nucleotides to which the ends associate are contiguous to each other (gap is 0), or there is a gap of several nucleotides between them. The gap preferably consists of 0 to 5 nucleotides, more preferably 0 to 3 nucleotides, still more preferably 0 or 1 nucleotide.

As for the positional relationship with respect to the target site, the loop primer and the probe are designed so that the target site locates 3 to 20 nucleotides, preferably 5 to 15 nucleotides, upstream from the 5' end of the associated loop primer. The probe is preferably designed so that it associates with the target site substantially at the center, more specifically, at a position within 5 nucleotides upstream or downstream from the center thereof, irrespective of the loop primer. When the loop primer and the probe are designed so that the probe and the loop primer are totally included in a region between the B1 region and the B2 region or a region between the F1 region and the F2 region (the region with which FIP or BIP associates does not overlap with the region with which the probe associates), the region between the B1 region and the B2 region (the regions B1 and B2 themselves are not included) or the region between the F1 region and the F2 region (the regions F1 and F2 themselves are not included) generally consists of about 40 to 50 nucleotides in many cases, since both the probe and the loop primer consists of about 20 nucleotides.

In the LAMP-FLP method of the present invention, one of the loop primer and the probe is modified with a fluorescent molecule around the 5' end thereof in the case of the loop primer or around the 3' end thereof in the case of the probe, and the other is modified with a quenching molecule. Therefore, when the probe associates at a position close to the position at which the loop primer associates, FRET (fluorescence resonance energy transfer) occurs, and fluorescence intensity changes. In the LAMP-FLP method of the present invention, the 5' end of loop primer usually used in the conventional LAMP method is modified with a fluorescent molecule or a quenching molecule and used, and this is for utilizing the property of the loop primer that it is necessarily disposed at the end portion of the amplification product in the LAMP method. Moreover, since the fluorescent molecule or the quenching molecule is added around the 3' end of the probe, the probe does not function as a primer.

In the LAMP-FLP method of the present invention, as the fluorescent molecule, there can be used, for example, N-(3-fluoranthyl)maleimide (FAM), fluorescein, dansyl, cascade yellow, fluorescamine, Oregon green, pyrene, Texas red, Pacific blue, marine blue, Alexa, lucifer yellow, BODIPY, coumarin, PyMPO, TET, JOE, Cy3, Cy5, Cy5.5, Cy7, ROX, VIC, HEX, TAMRA, SYBR Green, NBD, and so forth. As the quenching molecule, there may be used a substance that does not emit fluorescence (called dark quencher) such as 4-dimethylaminoazobenzene-4'-carboxylic acid (dabcyl), QSY-7, QSY-21, QSY-35, BHQ-0, BHQ-1, BHQ-2, BHQ-3, and Eclipse, or a fluorescent substance that shows an absorption band in a wavelength range of fluorescence emitted by any of the aforementioned fluorescent molecules may be used as the quenching molecule. Methods for binding the fluorescent molecule and the quenching molecule to an oligonucleotide and methods for detecting change of fluorescence intensity are well known in this technical field.

### [Design of primers and probes for LAMP-FLP method]

The primers and probes used for the LAMP-FLP method of the present invention can be designed as required on the basis of the aforementioned principle of the LAMP-FLP method, and with reference to the methods for designing primers for the conventional LAMP method and methods for designing other probes. For designing LAMP primers, software is marketed, and it can be used also for the LAMP-FLP method of the present invention.

Hereafter, the designing method will be explained by exemplifying a case where the target site is SNP.
(1) The probe region (region of the template nucleotide with which the probe associates) is preferably determined so that SNP is complementary to a nucleotide close to the central part of the probe. Specifically, it is preferably designed so that SNP is complementary to 8 to 10 nucleotide of the central part of the probe. The probe is designed in a length of 15 to 30 nucleotides, preferably 18 to 25 nucleotides, because such a length is effective for specific association with an intended complementary sequence.
(2) The set of the four basic primers for the LAMP method are designed so that the SNP concerned of the probe region exists in a loop region between the B1 region and the B2 region (not including the B1 and B2 regions themselves) or between the F1 region and the F2 region (not including the F1 and F2 regions themselves). All the primers are designed so as not to include SNP, but the probe may overlap with FIP or BIP. The lengths of the F3 primer and the B3 primer are independently 15 to 30 nucleotides, preferably 18 to 25 nucleotides. The lengths of FIP and BIP are independently 30 to 60 nucleotides, preferably 35 to 55 nucleotides.
(3) Concerning the relation to SNP, the loop primer is designed so that SNP exists 3 to 20 nucleotides, preferably 5 to 15 nucleotides, upstream from the 5' end of the associated loop primer. Each loop primer independently has a length of 15 to 30 nucleotides, preferably 18 to 25 nucleotides.

The four primers for the LAMP method are generally designed with paying attentions usually to four of factors, i.e., Tm value, stability of end of each primer region, GC content, and secondary structure. The same shall apply to the LAMP-FLP method of the present invention.

Tm values of the regions may be 60 to 68°C, or 64 to 66°C as the case may be, for the F1c and B1c regions, 55 to 63°C, or 59 to 61°C as the case may be, for the F2 region, B2 region, F3 region, and B3 region, and 64 to 66°C for the loop primer. They can be finely adjusted as required. Since the end of each primer region serves as an origin of the synthesis of nucleic acid, stability is required for it. The primers are preferably designed so that free energies of the 3' ends of F2/B2, F3/B3, and LF/LB and the 5' end of F1c/B1c are not higher than -4 kcal/mol. Since the 5' end of F1c corresponds to the 3' end of the F1 region after replication, stability thereof is important. The primers can be designed so as to have a GC content of 35 to 75%, or 40 to 65% as the case may be. They can also be preferably designed so as to have a GC content of 50 to 60%. As for the secondary structure, FIP and BIP, in particular, are preferably designed so as not to have any extreme secondary structure. They are also designed so that the 3' ends of them are not complementary to each other, in order to prevent generation of a primer dimer.

As for the distance between the primers, they can be designed so that the distance from the outside end of the F2 region to the outside end of the B2 region (corresponding to the region to be amplified by the LAMP method) is 120 to 160 nucleotides. They may be designed with increasing the distance by about 20 nucleotides for each time as required, and they may be designed so that the distance is 120 to 220 nucleotides. They can also be designed so that the distance from the 5' end of the F2 region to the 5' end of the F1 region (portion that forms a loop) is 40 to 80 nucleotides, and the distance may be increased up to 100, or up to 120, if needed. They can be designed so that the distance between the F2 region and the F3 region is 0 to 20 nucleotides, or 0 to 60 nucleotides, if needed.

More specifically, the primers can be designed as follows.

### (1) Design of four basic primers

Commercially available software for designing LAMP primers such as Primer Explorer V4 (FUJITSU, http://primerexplorer.jp/v4#manual/pdf/PEver4.pdf) is started, and the primers are designed with the default setting. The sequence of template nucleotide is read, and the position of the probe to be designed is first roughly determined. If appropriate primers are not designed with the default setting, the values of Distances may be changed. When the design is still difficult, levels of the chain length and the Tm value are eased.

### (2) Design of loop primer

The designed primer set is read, and the loop primer is designed with the default setting. When any appropriate loop primer is not designed with the default setting, the parameters of chain length, Tm, and GC content are changed.

### (3) Determination of probe sequence

According to the loop primer, the probe region is finely adjusted. As the nucleotide on the probe corresponding to the SNP concerned, there can be chosen a corresponding nucleotide of an allele that more easily generates Tm difference between the cases of full match and mismatch among the major allele and minor allele.

The designed primers and probes can be synthesized by existing methods well known to those skilled in the art.

### [Implementation of LAMP-FLP method]

The LAMP-FLP method to be carried out by using the aforementioned primers and probes typically comprises the isothermal amplification step according to the LAMP method, warming step, and step of observing change of fluorescence with slowly lowering temperature, or slowly raising the temperature once lowered.

For the isothermal amplification step, an enzyme that catalyzes any of the various strand displacement reactions for the LAMP method can be used. More specifically, examples include a DNA polymerase 1 derived from a *Geobacillus* bacterium, Bst DNA polymerase (part of the DNA polymerase complex derived from *Bacillus stearothermophilus,* refer to Japanese Patent Nos. 3046142, 4220557, and 4446498), Csa DNA polymerase, and 96-7 DNA polymerase isolated from compost obtained by fermentation at 85°C or higher (refer to Japanese Patent Application No. 2011-539375 (PCT/JP2010/069560)). For all of these, commercial products can be used.

The temperature and time of the isothermal amplification step can be appropriately determined depending on the type of enzyme to be used, etc. The temperature may be, for example, 55 to 70°C, more specifically 60 to 68°C, and the time may be 15 to 80 minutes, more specifically 20 to 40 minutes.

The subsequent warming step can also be appropriately designed according the type of enzyme to be used, Tm value of the probe to be used, etc. The temperature may be, for example, 90 to 100°C, more specifically 95 to 99°C, and the time may be 1 to 10 minutes, more specifically 3 to 7 minutes.

After the warming step, temperature of the system is changed within a temperature range in which association or dissociation of the probe occurs, and change of the fluorescence occurring during the temperature change is monitored. The changing of the temperature within a temperature range in which association or dissociation of the probe occurs includes lowering the temperature as required after performing the isothermal amplification step and the subsequent warming step from the temperature of fhe warming step to a temperature at which association or dissociation of the probe occurs, and then to a further lower temperature (for example, 63°C -> 94°C -> lowering temperature), and raising temperature as required from the temperature once lowered after performing the isothermal amplification step and the subsequent warming step to a temperature at which association or dissociation of the probe occurs, and then to a further higher temperature (for example, 63°C -> 94°C -> 35°C -> raising temperature). In the former case, the probe gradually associates, and in the latter case, it gradually dissociates. In a preferred embodiment of the LAMP-FLP method, with lowering the temperature, change of the fluorescence occurring during the temperature decrease is monitored, and the probe gradually associates in connection with the temperature decrease. In the typical LAMP-QP method, temperature is raised, and change of the fluorescence occurring during the temperature raise is monitored.

According to the investigations of the inventors of the present invention, data obtained at the times of association and dissociation might differ. In the aforementioned preferred embodiment of the LAMP-FLP method, dissociation of the primers is allowed, while the enzyme is inactivated in the warming step, and change of fluorescence is monitored while association of the probe is allowed by lowering the temperature. Therefore, the time for the detection is shortened, and the mechanism for the measurement can be made simple.

When the temperature is lowered in the step of monitoring change of fluorescence, the temperature is preferably slowly lowered at a substantially constant rate of, for example, 0.005 to 2.0°C/second, preferably 0.01 to 1.0°C/second, more preferably 0.025 to 0.075°C/second. The LAMP-FLP method utilizes difference of Tm values for the cases of full match and mismatch of the probe and the nucleotides of the target site, and change of fluorescence intensity during the temperature-lowering process is monitored to obtain fluorescence intensity changing rate, and thereby enables identification of specific nucleotides of the target site.

The fluorescence intensity changing rate can be calculated by primary differentiation of a curve obtained by plotting the fluorescence intensity against the temperature. The values obtained by the primary differentiation may be further differentiated.

The primary differentiation values may form different peaks for the cases of full match and mismatch of the probe and the region with which the probe associates. When both full match and mismatch exist, both peaks may be formed. Therefore, in the case of SNP, whether two alleles of chromosomal polymorphism derived from a specimen are homozygous or heterozygous can also be judged on the basis of the shape of peak. Since the Tm value of the probe may change depending on the presence of salt or contaminant, presence of SNP around the target SNP, etc., it may be difficult to pinpoint the position of peak, when many specimens are examined. However, comparison of the examination results enables appropriate judgment.

The LAMP-FLP method can be implemented on a scale of about several tens of microliters, like the usual LAMP method. Amounts of the template nucleotide, primers, and probe to be used can be appropriately determined by those skilled in the art. For example, the template nucleotide can be used in the range of 0.005 to 0.50 ng/µL, FIP and BIP can be used in the range of 0.1 to 10 µM, the F3 primer and B3 primer can be used in the range of 0.02 to 2.0 µM, and the loop primer not modified with a fluorescent molecule or quenching molecule can be used in the range of 0.08 to 8.0 µM. Although concentrations of the loop primer and probe modified with a fluorescent molecule or quenching molecule may depend on the type of the molecule to be modified, and type of apparatus used for the measurement, the loop primer B modified with FAM can be used in the range of 0.02 to 2.0 µM, and the probe modified with dabcyl used together can be used in the range of 0.04 to 4.0 µM.

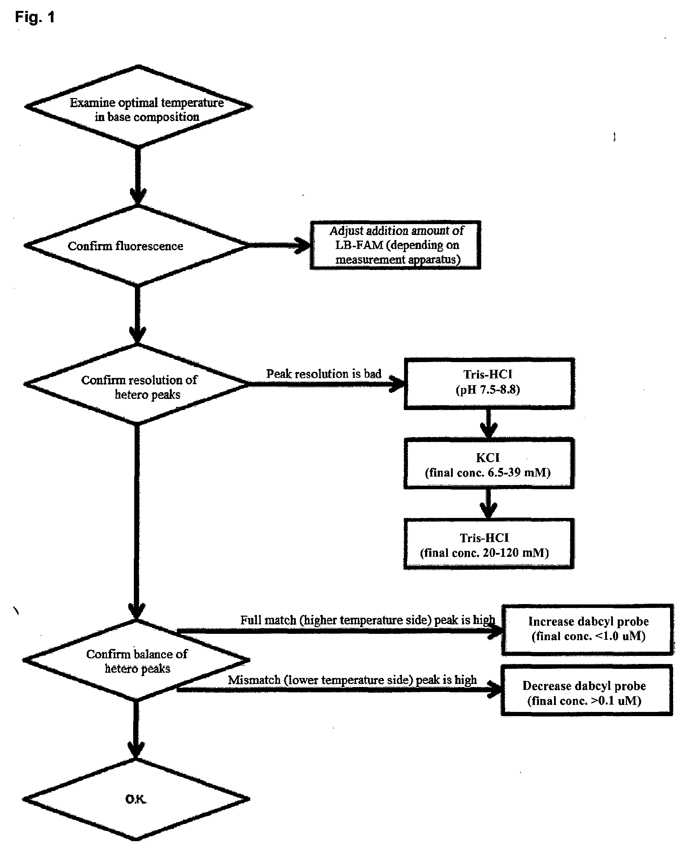

As for the composition of the system, it can contain the aforementioned amounts or concentrations of template, primers, and probe, as well as the four kinds of dNTPs, buffering agent, salt, surfactant, and enzyme that catalyzes the strand displacement reaction for the LAMP method, as apparent to those skilled in the art. Concentrations of the reagents contained in the system can be appropriately determined so that peak can be clearly observed, and judgment can be satisfactory performed. An example of adjustment scheme of primer, probe, and salt concentrations for favorably carrying out the LAMP-FLP method is shown in Fig. 1.

### [Advantages of LAMP-FLP method]

The advantages of the LAMP-FLP method of the present invention will be explained by comparison with the LAMP-QP method. The LAMP-QP method is a method for identifying a nucleotide by using difference of Tm value observed for the cases of full match and mismatch of a fluorescent probe and a target nucleotide, like the LAMP-FLP method. In the LAMP-QP method, a loop region of an LAMP method product is used as a target, and a quenching probe is designed for this position. The 5' end or 3' end of the quenching probe is labeled with a fluorescent substance, and a phenomenon that when the probe binds to a target nucleic acid, the fluorescent molecule interacts with guanine (G) in the target nucleic acid sequence, and the fluorescence is thereby quenched is utilized. If the target nucleic acid exists, fluorescence quenches, and if the target nucleic acid does not exist, fluorescence does not quench. Therefore, presence or absence of the target nucleic acid can be determined. Although the probe functions independently, it must be designed so that C is positioned at a position for the interaction with G in the target nucleic acid sequence, i.e., near the fluorescent molecule at the end, and therefore it suffers from a problem that the degree of freedom of the design is restricted. Depending on the case, LAMP primers that also allow amplification of regions other than the intended template nucleotide must be designed on account of the probe design, and this may lead erroneous judgment.

In contrast, the LAMP-FLP method of the present invention utilizes the property of the loop primer used in the LAMP method that it is necessarily positioned at the end of the amplification product. The 5' end of the loop primer usually used for the LAMP method is modified with a fluorescent molecule or a quenching molecule. Any particular restrictions are not imposed on the design of the probe. Since the probe can be used in a comparatively large amount depending on the amount of the loop primer, it is possible to visually judge emission and quenching of fluorescence under ultraviolet ray or black light irradiation. It is considered that the LAMP-FLP method is theoretically more sensitive compared with the LAMP-QP method. According to the investigations of the inventors of the present invention, the LAMP-FLP method was actually far more sensitive than the LAMP-QP method. Further, since the specificity for the target site depends on not only the probe, but also positional relationship with respect to the loop primer, it is considered that the LAMP-FLP method is more accurate.

The LAMP-FLP method also has an advantage that it allows various modifications. The typical LAMP-FLP method uses one pair of a loop primer having a fluorescent molecule and an oligonucleotide probe having a quenching molecule. However, a multiplex system using a polychromatic fluorescent molecule can also be designed in order to simultaneously detect two or more target sites. For example, in order to simultaneously determine rs8099917 of the IL28B gene, and rs1127354 of the ITPA gene, it is contemplated that a TAMRA-Eclipse Dark quencher system is used for determining rs8099917, and a FAM-Dabcyl system is used for determining rs1127354.

It is also possible to add a quenching molecule to the loop primer, and use two kinds of fluorescent probes. For example, a probe that fully matches G is labeled with FAM, a probe that fully matches A is labeled with TAMRA, and they are used for the LAMP-FLP method. According to this modified method, judgment based on change of color tone, not based on the change rate of fluorescence intensity (waveform of melting profile obtained by plotting fluorescence change against temperature change), can be expected.

### [Use]

The LAMP-FLP method of the present invention can be used for typing of various SNPs. SNPs as the target of typing are not particularly limited For example, in order to predict therapeutic effect for hepatitis C, it can be chosen from rs12043519, rs1986953, rs13392065, rs16987149, rs16987155, rs2374341, rs2374342, rs7566701, rs12713624, rs10208788, rs10208883, rs9876122, rs2036081, rs13122167, rs4488950, rs13150115, rs7663113, rs1379606, rs913500, rs6458003, rs16906840, rs2224068, rs9390164, rs4512312, rs757844, rs4876271, rs7942675, rs17787293, rs917334, rs6489019, rs6489020, rs6489021, rs11610391, rs9528038, rs341554, rs2325219, rs1353348, rs2076954, rs12907066, rs8075713, rs4795794, rs955155, rs12972991, rs12980275, rs8105790, rs11881222, rs8103142, rs28416813, rs4803219, rs8099917, rs7248668, rs10853728, rs4803223, rs2980602, rs4803224, rs11881222, rs8103142, and rs12979860.

The inventors of the present invention designed primers and probes for the LAMP-FLP method used for typing of rs8099917, rs12979860, rs11881222, rs8103142, rs1127354, and rs7270101, actually used them for the typing, and confirmed that they were effective.

The inventors of the present invention also designed LAMP-FLP primers and probes for ADH1B (human alcohol dehydrogenase 1B) rs1229984, ALDH2 (human aldehyde dehydrogenase 2) rs671, UCP1 (human uncoupling protein 1) rs1800592, ADRB2 (human adrenoreceptor beta2) rs1042713, ADRB3 (human adrenoreceptor beta3) rs4994, CYP2C9*3 (cytochrome p450 2c9*3) rs1057910, VKORC1 #1 (vitamin K epoxide reductase complex 1 #1) rs9934438, and VKORC1 #2 (vitamin K epoxide reductase complex 1 #2) rs9923231. Also for other SNPs, or target sites other than SNPs, primers and probes for the LAMP-FLP method aiming at detection or typing thereof can be designed on the basis of the descriptions of this specification.

The method of the present invention can be used for judging whether a test subject who is likely to suffer from a disease or a test subject who already has a disease can enjoy benefit of a treatment with a drug, and for predicting response of a test subject who is receiving a treatment (for example, those for hepatitis C etc.). That is, the present invention also provides companion diagnostic methods and products. Specifically, according to the present invention, there are provided companion diagnostic methods and products therefor (probe, set or kit of probe and primers, etc.) for chemotherapy of diseases and conditions relating to at least rs8099917, rs2979860, rs11881222, rs8103142, rs1127354, rs7270101, rs1229984, rs671, rs1800592, rs1042713, rs4994, rs1057910, rs9934438 and rs9923231.

For example, the type of rs8099917 (TT, TG, GG) among SNPs existing in the IL28B gene is known as one of the leading factors for prediction of effect of the combined therapy using peginterferon and ribavirin. It has already been found that the combined therapy using peginterferon and ribavirin is not effective on patients who have a risk allele of TG or GG (Patent document 2, Non-patent documents 5 and 6). Further, rs1127354 and rs7270101 have been reported as SNPs involved in hemolytic anemia known as adverse drug reaction of ribavirin (Non-patent document 7). Therefore, the present invention provides a companion diagnostic method and product therefor (probe, set or kit of probe and primers, etc.) for the combined therapy using peginterferon and ribavirin for a person having a risk of hepatitis C or a hepatitis C patient.

The present invention provides a probe, set of probe and primers, and kit for performing the LAMP-FLP method. Specific examples of the probe provided by the present invention include an oligonucleotide having a quenching molecule or fluorescent molecule around the 3' end thereof, in which the oligonucleotide consistd of the sequence of SEQ ID NO: 13 or 143 (preferably SEQ ID NO: 143) for rs8099917, SEQ ID NO: 26 for rs12979860, SEQ ID NO: 39 for rs11881222, SEQ ID NO: 52 for rs8103142, SEQ ID NO: 65 for rs1127354, SEQ ID NO: 78 for rs7270101, SEQ ID NO: 85 rs1229984, SEQ ID NO: 92 for rs671, SEQ ID NO: 99 for rs1800592, SEQ ID NO: 106 for rs1042713, SEQ ID NO: 113 for rs4994, SEQ ID NO: 120 for rs1057910, SEQ ID NO: 127 for rs9934438, or SEQ ID NO: 134 for rs9923231. Any of these probes can be modified by depleting or adding one or several nucleotides (for example, 1 to 4, 1 to 3, or 1 or 2 nucleotides) at an end. When the probe is extended, the extended portion may have a sequence complementary to the region for the association. These probes can also be modified (including modifications by deletion and extension) so that the sequence corresponding to the target site is complementary to the other allele. For example, when a major allele is chosen and a probe complementary thereto is designed for a certain SNP, a probe in which the nucleotide corresponding to SNP is replaced with a nucleotide complementary to a minor allele can be designed as a modified version of the probe. The present invention also provides such a modified probe.

Specific examples of the set of primers and probes provided by the present invention include a set of oligonucleotides of the sequences of SEQ ID NOS: 7 to 13 or SEQ ID NOS: 7 to 12 and 143 (preferably SEQ ID NO: 7 to 12 and 143) for rs8099917, SEQ ID NOS: 20 to 26 for rs12979860, SEQ ID NOS: 33 to 39 for rs11881222, SEQ ID NOS: 46 to 52 for rs8103142, SEQ ID NOS: 59 to 65 for rs1127354, SEQ ID NOS: 72 to 78 for rs7270101, SEQ ID NOS: 79 to 85 for rs1229984, SEQ ID NOS: 86 to 92 for rs671, SEQ ID NO: 93 to 99 for rs1800592, SEQ ID NOS: 100 to 106 for rs1042713, SEQ ID NOS: 107 to 113 for rs4994, SEQ ID NO: 114 to 120 for rs1057910, SEQ ID NO: 121 to 127 for rs993443 8, or SEQ ID NOS: 128 to 134 for rs9923231. In these sets, a quenching molecule is provided on one of a primer corresponding to the loop primer B around the 5' end thereof, and the probe around the 3' end thereof, and a fluorescent molecule is provided on the other. Any of the primers may be modified by deletion or addition of one or several (for example, 1 to 4, 1 to 3, or 1 or 2) nucleotides at an end. When a primer is extended, the extended portion may have a sequence complementary to the region for the association. Any probe may be modified by deletion, addition, or substitution, as described above. The present invention also provides a set consisting of arbitrary modified primers and modified probe selected from those mentioned above.

The kit provided by the present invention comprises:
- four kinds of primers (FIP, F3 primer, BIP, and B3 primer) designed on the basis of six regions of a template polynucleotide (F1 region, F2 region, F3 region, B1 region, B2 region, and B3 region):
- a loop primer designed on the basis of a region between the F1 region and the F2 region or between the B1 region and the B2 region on the side on which a target site exists, and
- an oligonucleotide probe that can associate with a region including the target site.

The kit may also comprise an instruction for performing companion diagnostics by the LAMP-FLP method. The set of primers and probes used for the kit can be constituted as described above (the set includes one comprising a modified primer or a modified probe). The kit can be designed as one for detecting rs8099917, and such a kit can be used for predicting effect of the combined therapy using peginterferon and ribavirin for a treatment of hepatitis C.

### [Preparation method of nucleic acid]

In the LAMP-FLP method of the present invention, origin of the template nucleic acid serving as the object of gene amplification is not particularly limited. Further, the method for preparing the template nucleic acid is not also particularly limited. It may be prepared from hair root, blood, or tissue by a known method. Typical examples include those purified from nail, oral mucosa, blood, urine, cerebrospinal fluid, sputum, and pathological tissue using a kit. As existing nucleic acid purification methods, the sodium iodide method, alkali extraction method, and phenol-chloroform method, and so forth are known, and as kits, Genomix (Talent SRL), MagExtractor (Toyobo), QIAamp DNA Blood Mini Kit (QIAGEN), and so forth are known.

A solution obtained by treating a specimen with proteinase K can be used as it is as a solution containing a template nucleic acid for the amplification reaction. The method using a proteinase K is specifically as follows. To an extraction solution containing 0.5 mg/ml of proteinase K, 37.5 mM dithiothreitol, 10 mM Tris-HCl (pH 8.0), 1 mM EDTA (pH 8.0), and 100 mM NaCl, a specimen is added, and the resulting mixture is heated at 55°C for 20 minutes or longer. The reaction mixture is further heated at 94°C for 10 minutes or longer to inactivate the proteinase K. When hair (hair root) is used as the specimen, 1 to 3 hairs having a length of about 1 cm are added to 50 µl of the aforementioned extraction solution. Conditions of the hairs as the specimen such as presence or absence of hair dyeing and being canities or not are not particularly limited so long as they include hair roots. Further, the hair is not limited to a head of hair, and they may be body hair containing hair roots.

A supernatant obtained as described above can be directly used as the template DNA for gene amplification.

This method can be used as a method for preparing a nucleic acid used as a template for use in not only the LAMP-FLP method of the present invention, but also other nucleic acid amplification methods such as the LAMP method, PCR method, SDA method, TMA method, NASBA method, TRC method, ICAN method, SmartAmp method, and RCA method.

Amplification of a nucleic acid is performed mainly for genetic tests. Genetic tests include pathogen genetic test for investigating foreign genes of viruses, bacteria, and so forth that cause infectious diseases, somatic cell genetic test for investigating acquired mutation and abnormal expression of genes of somatic line in tumor cells, and genetics test or germ line genetic test for clarifying genetic information that is inherently possessed and does not change over the whole life. The method for preparing a nucleic acid of the present invention can be used for these tests. Although type of the specimen used for preparing a nucleic acid differs depending on purpose and object of the test, the method of the present invention can be used for preparation of a nucleic acid from various specimens. Specimens to which the present invention can be applied include peripheral blood white blood cell, oral cavity cell, hair (hair root cell), nail, bloodstain, umbilical cord, organ tissue, blood serum, plasma, blood (white blood cell), bone marrow, bone marrow mononuclear cell, cerebrospinal fluid, lesion swab, lesional scraping, aqueous humor, lymphonodus, bronchoalveolar lavage fluid, pleural effusion, ascites, pericardial fluid, pancreatic juice, sputum, bone, pus, urine, feces, vomit, stomach juice, lymphonodus, skin, intestinal tissue, solid tissue (biopsy), paraffin-embedded block, embedded block for frozen sections, autopsy specimen (tissue), and cultured cell.

### Examples

The present invention will be explained in more detail with reference to the following examples. However, the present invention is not limited to the following examples.

### [Methods for preparing samples]

To an extraction solution containing 0.5 mg/ml of proteinase K, 37.5 mM dithiothreitol, 10 mM Tris-HCl (pH 8.0), 1 mM EDTA (pH 8.0), and 100 mM NaCl, 3 of 1 cm human hairs including hair roots were added, and the resulting mixture was heated to 55°C for 20 minutes or longer. The reaction mixture was further heated at 94°C for 10 minutes or longer to inactivate the proteinase K. The obtained supernatant was directly used as a template DNA for gene amplification by the LAMP method etc.

### [Design of primer set and probe]

In this example, the LAMP-QP method and the LAMP-FLP method of the present invention were compared. The primer set and the probe used in the LAMP-FLP method were designed as follows.
(1) The probe position was determined so as to include SNP.
(2) In the case of the LAMP method, a set of the four basic primers was designed so that the SNP concerned in the probe region should locate in the loop region between F1 and F2 or between B1 and B2. All the primers were designed so as not to include SNP, but overlap of the probe and FIP, or the probe and BIP was allowed (refer to Comparison Evaluation Example 1, IL28B rs8099917 typing).
(3) Then, the loop primer was designed. In the LAMP-FLP method, when SNP was located between B1 and B2, the 5' end of the loop primer B was labeled with the FAM molecule, and the donor was incorporated on the side of the product of the LAMP method, so that the Tm value thereof became higher than that of the probe.

More precisely, the primer set and the probe were designed according to the following procedures.

### (1) Design of four basic primers

The LAMP method primer design-supporting software, PrimerExplorer V4 (Fujitsu Systems East), was started, and designing was performed with the default setting. In this procedure, the sequence was read, and the position at which the probe was designed was first temporally determined. When appropriate primers were not designed with that setting, the values of Distances were changed. When the design was still difficult, the conditions for the chain length and Tm value were eased.

### (2) Design of loop primer

The primers of the set designed with the aforementioned software were read, and the loop primer was designed with the default setting. When appropriate primer was not designed with that setting, the values of the parameters of chain length, Tm value, and GC content were changed.

### (3) Determination of probe sequence

According to the loop primer, the position of the probe was finely adjusted. As the nucleotide corresponding to the SNP concerned, there was chosen a nucleotide corresponding to an allele that more easily generated Tm difference between the cases of full match and mismatch among the major allele and minor allele.

### [Synthesis of oligonucleotide]

All the oligonucleotides used in the experiments were synthesized by Nippon Gene Material. In the evaluations, all the regions used as the template (commonly used for the LAMP-FLP method and the LAMP-QP method) were cloned by using Cloning Vector pTS1 DNA (Nippon Genetech).

### [Concentrations of template, primers and probes in evaluation system]

In the following evaluation examples, an evaluation system in a volume of 25 µL was used. As the template DNA, in the case of a real specimen extracted from hair roots, 4 µL of the aforementioned solution was used, and in the case of an artif cial template DNA, 1.6 ng of the template DNA was used per 25 µL of the system. The primers and the probe were used at the following concentrations.

| LAMP-QP method | |
|---|---|
| 1.6 µM | FIP |
| 1.6 µM | BIP |
| 0.2 µM | F3 |
| 0.2 µM | B3 |
| 0.8 µM | LF |
| 0.4 µM | Q probe |

| LAMP-FLP method | |
|---|---|
| 1.6 µM | FIP |
| 1.6 µM | BIP |
| 0.2 µM | F3 |
| 0.2 µM | B3 |
| 0.8 µM | LF |
| 0.2 µM | LB-FAM |
| 0.4 µM | Dabcyl probe |

### [Comparison Evaluation Example 1: IL28B rs8099917 typing]

The sequences of the used primers and probes are shown below.

**[Table 2]**

| Probe and primer set for IL28B rs8099917 typing | |
|---|---|
| LAMP-QP method | |
| SEQ ID NO: 1 rs8099917 FIP | TACCTCCAGG AGGTGAAATA CAAGAGTTCC TTGTAAAAGA TTCCATCCAT |
| SEQ ID NO: 2 rs8099917 BIP | TGTCACTGTT CCTCCTTTTG TTTTCCCAAA CTGTATACAG CATGGTTC |
| SEQ ID NO: 3 rs8099917 F3 Primer | TTGTGCATAT GTTTTCTGAC TACC |
| SEQ ID NO: 4 rs8099917 B3 Primer | ATGGTAAGAC ATAAAAAGCC AGC |
| SEQ ID NO: 5 rs8099917 Loop Primer F | AACTCTCCAT GTTGTATGTT TTTGT |
| SEQ ID NO: 6 rs8099917 Quenching Probe | CTGTGAGCAA TgTCACCC |

| LAMP-FLP method | |
|---|---|
| SEQ ID NO: 7 rs8099917 FIP | CCTCCAGGAG GTGAAATACA AGACCTTGTT CCTTGTAAAA GATTCCA |
| SEQ ID NO: 8 rs80999917 BIP | TCACTGTTCC TCCTTTTGTT TTCCTGACAT AAAAAGCCAG CTACC |
| SEQ ID NO: 9 rs8099917 F3 Primer | ATATGTTTTC TGACTACCAA AGT |
| SEQ ID NO: 10 rs8099917 B3 Primer | GGGAGAATGC AAATGAGAGA |
| SEQ ID NO: 11 rs8099917 Loop Primer F | TCCATGTTGT ATGTTTTTGT ATGGA |
| SEQ ID NO: 12 rs8099917 Loop Primer FB | AAATTGGAAC CATGCTGTAT ACAGT |
| SEQ ID NO: 13 rs8099917 Quencher Probe | TTCTGTGAGC AATgTCACCC |

### LAMP-QP method

As the primer set and probe for identifying rs8099917 SNP, oligonucleotides consisting of the sequences of SEQ ID NOS: 1 to 6 were prepared. The 3' end of the quenching probe (SEQ ID NO: 6) was modified with the BODIPY FL molecule.

A total volume of 25 µL containing the template DNA, set of primers, probe, 0.42 mM each dNTPs, 20 mM Tris-HCl (pH 8.8), 10 mM KCl, 10 mM (NH₄)₂SO₄, 2.4 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate of 0.2°C per second from 40°C to 80°C. Change of the fluorescence value was monitored, and the change curve was primarily differentiated so that a part showing large change of fluorescence value is represented as a peak.

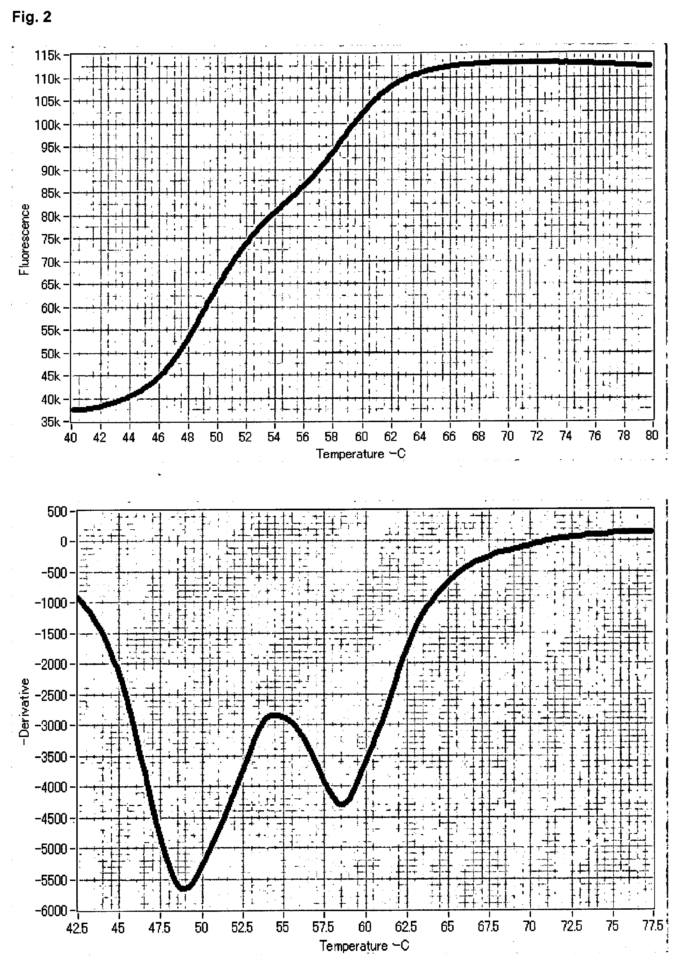

When the template extracted from the hair roots was used, a specific peak or peaks were formed around 49°C or 58°C, or around both the temperatures. Exemplary results are shown in Fig. 2. The results obtained by the LAMP-QP method for the template DNA extracted from the hair roots agreed with the results obtained by the direct sequencing method.

When the template DNA artificially synthesized so as to have T as the SNP concerned was used, a peak was formed around 49°C, and when the template DNA synthesized so as to have G as the SNP concerned was used, a peak was formed around 58°C. Further, when a mixture of these artificial template DNAs was used, peaks were formed around both the temperatures,

### LAMP-FLP method

As the primer set and probe for identifying rs8099917 SNP, oligonucleotides consisting of the sequences of SEQ ID NOS: 7 to 13 were prepared. The 5' end of the loop primer FB (SEQ ID NO: 12) was modified with the FAM molecule, and the 3' end of the quencher probe (SEQ ID NO: 13, full match probe) was modified with the DABCYL molecule. Further, positional relationship of the region artificially synthesized as the template DNA, the regions used for the LAMP method, and the probe is shown in Fig. 18.

A total volume of 25 µL containing the template DNA, set of primers, probe, 1.4 mM each dNTPs, 75 mM Tris-HCl (pH 8.0), 24 mM KCl, 8 mM MgSO₄, 0.1% Tween 20, and 8 units ofBst DNA Polymerase (Nippon Gene) was incubated on Genie (registered trademark) II (OptiGene) for 30 minutes, and then a probe association curve analysis was perfonmed. Specifically, after treatments at 63°C for 30 minutes and 98°C for 5 minutes, change of fluorescence intensity was measured with lowering the temperature at a rate of 0.05°C per second from 98°C to 30°C.

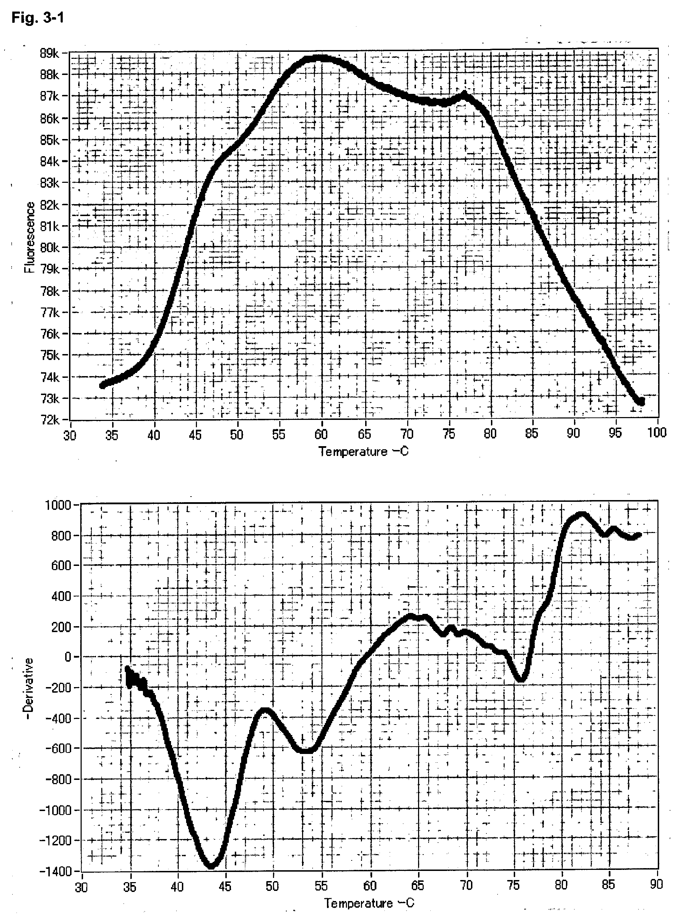

When the template extracted from the hair roots was used with the full match probe, a specific peak or peaks were formed around 50°C or 58°C, or around both the temperatures. When the mismatch probe (SEQ ID NO: 143 mentioned later) was used, a specific peak or peaks were formed around 43°C or 53°C, or around both the temperatures. Exemplary results (for the mismatch probe) are shown in Fig. 3-1.

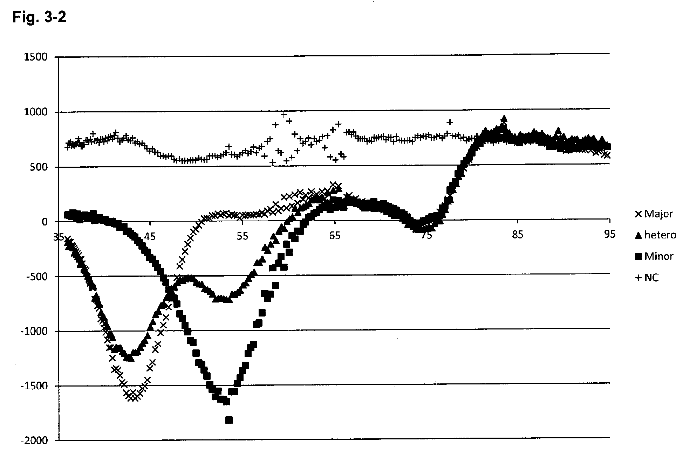

When the template DNA synthesized so as to have T as the SNP concerned was used (Major), a specific peak was formed around 43°C, and when the template DNA synthesized so as to have G as the SNP concerned was used (Minor), a specific peak was formed around 53°C. When a mixture of these artificial template DNAs was used (hetero), peaks were formed around both the temperatures mentioned above (Fig. 3-2).

### Coincidence ratio with respect to results of direct sequencing method

Coincidence ratio of the results with respect to the results obtained by the direct sequencing method was confirmed for the IL28B rs8099917 typing. As a result, the results of the LAMP-FLP method for the template DNA extracted from the hair roots agreed with the results of the direct sequencing method.

### [Comparison Evaluation Example 2: IL2.8B rs12978960 typing]

The sequences of the used primers and probes are shown below.

**[Table 3]**

| Probe and primer set for IL28B rs12978960 typing | |
|---|---|
| LAMP-QP method | |
| SEQ ID NO: 14 rs12979860 FIP | GGAATCCCAG ACTGTGCAGA GGCGGCGCTT ATCGCATACG G |
| SEQ ID NO: 15 rs12979860 BIP | CACGGTCGTG CCTGTCGTGG AGCGCGGAGT GCAATT |
| SEQ ID NO: 16 rg12979860 F3 Primer | TCCTCCTGCG GGACAAG |
| SEQ ID NO: 17 rs12979860 B3 Primer | ACTCGGCTCC AGCTCG |
| SEQ ID NO: 18 rs12979860 Loop Primer F | TTAGGGGCCC TGGCGAGG |
| SEQ ID NO: 19 rs12919860 Quenching Probe | TCCCCGAAGG CGcGAACC |

| LAMP-FLP method | |
|---|---|
| SEQ ID NO: 20 rs12979860 FIP | CCAGTACCCA TCCACgTCCA GGGGCGCTTA TCGCATACCG |
| SEQ ID NO: 21 rs12979860 BIP | GCACGCTCGT GCCTGTCGTG CGAGGGGCTT TGCTG |
| SEQ ID NO: 22 rs12979860 F3 Printer | TCCTCCTGCG GGACAAG |
| SEQ ID NO: 23 rs12979860 B3 Primer | ACTCGGCTCC AGGTCG |
| SEQ ID NO: 24 rs12979860 Loop Primer F | TGTGCAGAGG TTAGGGCC |
| SEQ ID NO: 25 rs12979860 Loop Primer FB | AATTGCACTC CGCGCTCC |
| SEQ ID NO: 26 ras12979860 Quencher Probe | GAAGGCGcGA ACCAGGGTT |

### LAMP-QP method

As the primer set and probe for identifying rs12979860 SNP, oligonucleotides consisting of the sequences of SEQ ID NOS: 14 to 19 were prepared. The 3' end of the quenching probe (SEQ ID NO: 19) was modified with the BODIPY FL molecule.

A total volume of 25 µL containing the template DNA, set of primers, probe, 1.4 mM each dNTPs, 20 mM Tris-HCl (pH 8.8), 10 mM KCl, 10 mM (NH₄)₂SO₄, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate of 0.2°C per second from 40°C to 80°C.

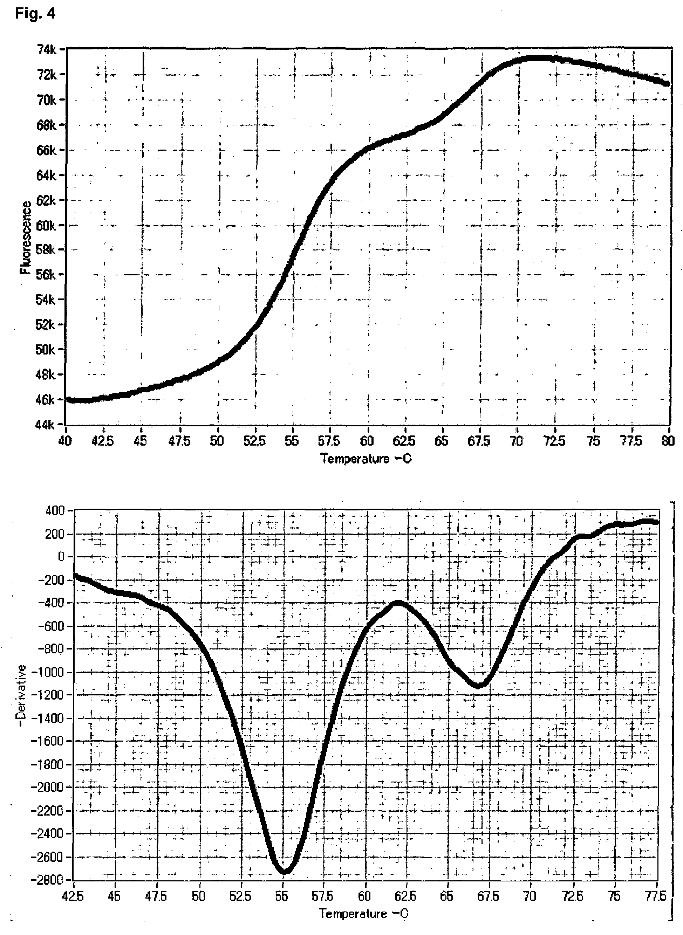

When the template extracted from the hair roots was used, a specific peak or peaks were formed around 55°C or 67°C, or around both the temperatures. Exemplary results are shown in Fig. 4. When the template DNA artificially synthesized so as to have C as the SNP concerned was used, a peak was formed around 67°C, and when the template DNA synthesized so as to have T as the SNP concerned was used, a peak was formed around 55°C. Further, when a mixture of these artificial template DNAs was used, peaks were formed around both the temperatures.

### LAMP-FLP method

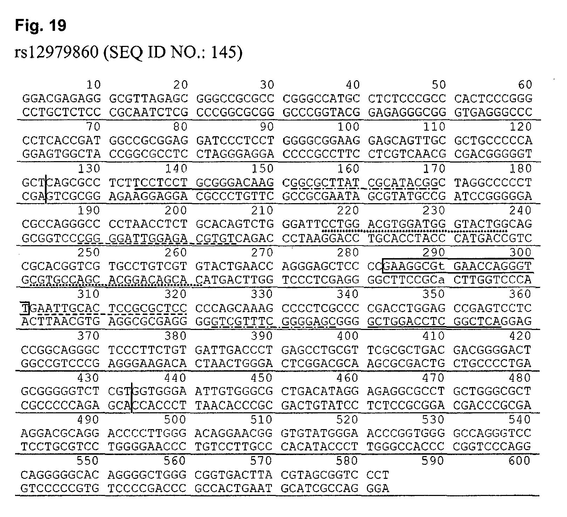

As the primer set and probe for identifying rs 12979860 SNP, oligonucleotides consisting of the sequences of SEQ ID NOS: 20 to 26 were prepared. The 5' end of the loop primer FB (SEQ ID NO: 25) was modified with the FAM molecule, and the 3' end of the quencher probe (SEQ ID NO: 26) was modified with the DABCYL molecule. Further, positional relationship of the region artificially synthesized as the template DNA, the regions used for the LAMP method, and the probe is shown in Fig. 19.

A total volume of 25 µL containing the template DNA, set of primers, probe, 1.4 mM each dNTPs, 75 mM Tris-HCl (pH 8.0), 13 mM KCl, 8 mM MgS0₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with lowering the temperature at a rate of 0.05°C per second from 98°C to 30°C.

When the template extracted from the hair roots was used, a specific peak or peaks were formed around 56°C or 67°C, or around both the temperatures. Exemplary results are shown in Fig, 5. When the template DNA artificially synthesized so as to have C as the SNP concerned was used, a peak was formed around 67°C, and when the template DNA synthesized so as to have T as the SNP concerned was used, a peak was formed around 56°C. Further, when a mixture of these artificial template DNAs was used, peaks were formed around both the temperatures.

### [Comparison Evaluation Example 3: IL28B rs11881222 typing]

The sequences of the used primers and probes are shown below.

**[Table 4]**

| Probe and primer set for IL28B rs11881222 typing | |
|---|---|
| LAMP-QP method | |
| SEQ ID NO: 27 rs118811-22 FIP | TTCCTCTCTA TCCTCCTCCC CCATGAGGAG AGGTGAGAAA CAGC |
| SEQ ID NO: 28 rs11881222 BIP | TGACAGGCAC AGGGGAGAGC TCCCCACACC TGCTACC |
| SEQ ID NO: 29 rs11881222 F3 Primer | GCCTCCAAAG CCACGG |
| SEQ ID NO: 30 rs11881222 B3 Primer | CCCCTTCACC TTCTCCTTCT |
| SEQ ID NO: 31 rs11881222 Loop Primer F | ACCTGTTCCC CTCACCTC |
| SEQ ID NO: 32 rs11881222 Quenching Probe | CTCTGCTCCc ACCTGACCAC |

| LAMP-FLP method | |
|---|---|
| FLP IL28B rs11881222 | |
| SEQ ID NO: 33 rs11881222 FIP | CAGGGACAAT GGAGAAGGAG AAGGCTCCCA GCCCTGCTCA |
| SEQ ID NO: 34 rs11881222 BIP | CCCCACACCT GCTACCCCTT CGAGGAGGAT AGAGAGGAAC AAGT |
| SEQ ID NO: 36 rs11881222 F3 Primer | GGAGTCAGGC CCACC |
| SEQ ID NO: 36 rs11881222 B3 Primer | GAGGTGAGGG GAACAGG |
| SEQ ID NO: 37 rs11881222 Loop Primer F | GGGCCACTAC AGAGCCAGG |
| SEQ ID NO: 38 rs11881222 Loop Primer FB | GCTGTGCCCT CTCCCCTGT |
| SEQ ID NO: 39 rs11881222 Quencher Probe | TGCTCCcACC TGACCACACT |

### LAMP-QP method

As the primer set and probe for identifying rs 11881222 SNP, oligonucleotides consisting of the sequences of SEQ ID NOS: 27 to 32 were prepared. The 3' end of the quenching probe (SEQ ID NO: 32) was modified with the BODIPY FL molecule.

A total volume of 25 µL containing the template DNA, set of primers, probe, 1.4 mM each dNTPs, 20 mM Tris-HCl (pH 8.8), 10 mM KCl, 10 mM (NN₄)₂SO₄, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate of 0.2°C per second from 40°C to 80°C.

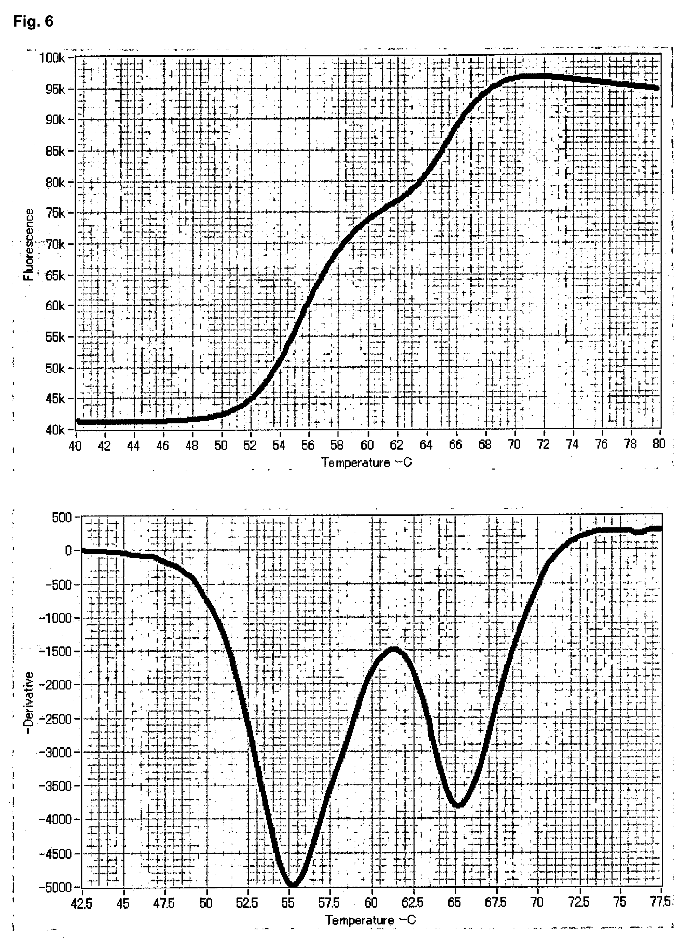

When the template extracted from the hair roots was used, a specific peak or peaks were formed around 54°C or 64°C, or around both the temperatures. Exemplary results are shown in Fig. 6. When the template DNA artificially synthesized so as to have A as the SNP concerned was used, a peak was formed around 54°C, and when the template DNA synthesized so as to have G as the SNP concerned was used, a peak was formed around 64°C. Further, when a mixture of these artificial template DNAs was used, peaks were formed around both the temperatures.

### LAMP-FLP method

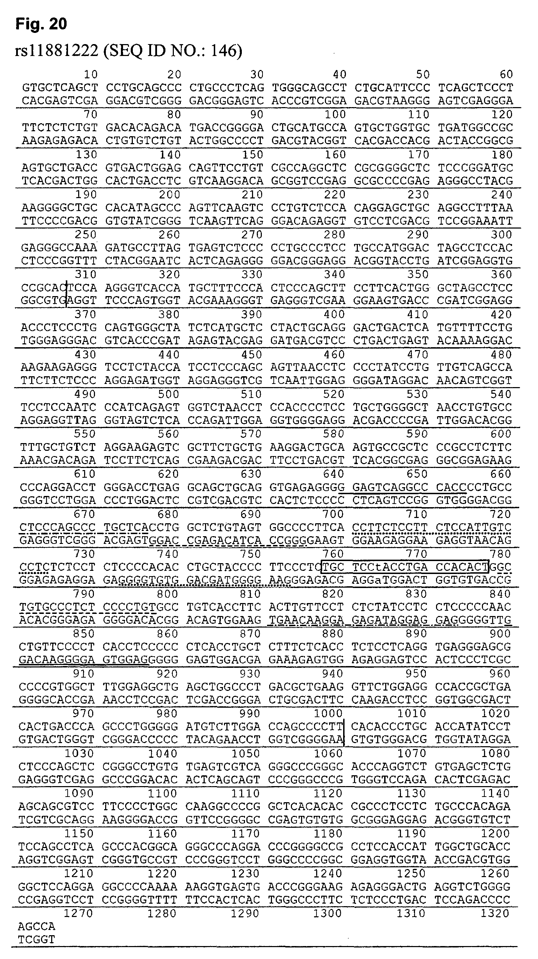

As the primer set and probe for identifying ras11881222 SNP, oligonucleotides consisting of the sequences ofSEQ ID NOS: 33 to 39 were prepared. The 5' end of the loop primer FB (SEQ ID NO: 38) was modified with the FAM molecule, and the 3' end of the quencher prohe (SEQ ID NO: 39) was modified with the DABCYL molecule. Further, positional relationship of the region artificially synthesized as the template DNA, the regions used for the LAMP method, and the probe is shown in Fig. 20.

A total volume of 25 µL containing the template DNA, set of primers, probe, 1.4 mM each dNTPs, 75 mM Tris-HCl (pH 8.0), 13 mM KCl, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with lowering the temperature at a rate of 0.05°C per second from 98°C to 30°C.

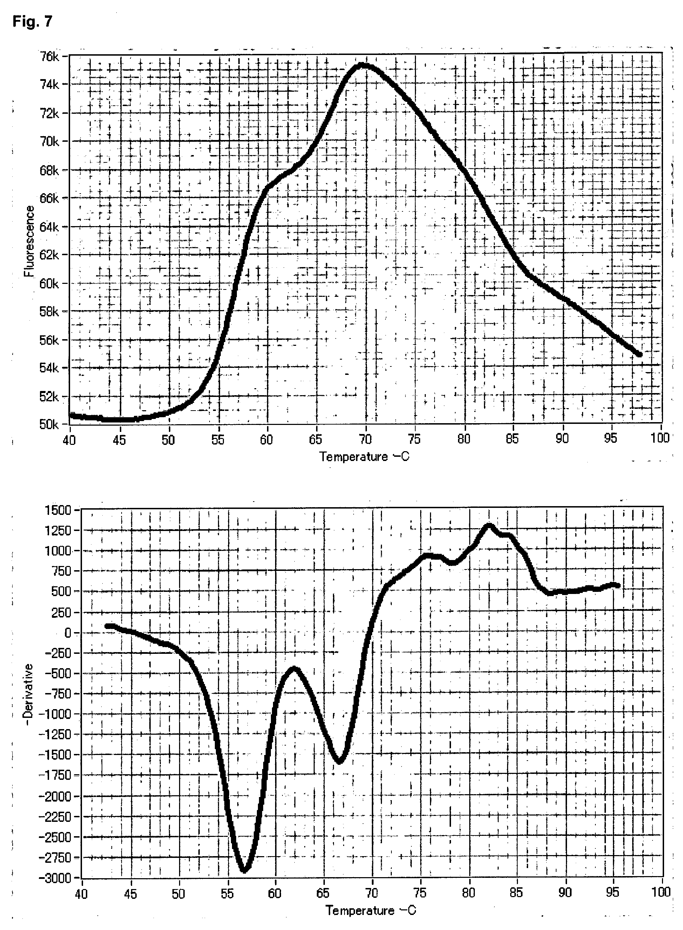

When the template extracted from the hair roots was used, a specific peak or peaks were formed around 57°C or 67°C, or around both the temperatures. Exemplary results are shown in Fig. 7. When the template DNA artificially synthesized so as to have A as the SNP concerned was used, a peak was formed around 57°C, and when the template DNA synthesized so as to have G as the SNP concerned was used, a peak was formed around 67°C.

### [Comparison Evaluation Example 4: IL28B rs8103142 typing]

The sequences of the used primers and probes are shown below.

**[Table 5]**

| Probe and primer set for IL28B rs81103142 typing | |
|---|---|
| LAMP-QP method | |
| SEQ ID NO: 40 rs8103142 FIP | TTAGCCCCAG CAGGAGGGCT CCCCTATCCT GTTGTCA |
| SEQ ID NO: 41 rs8103142 BIP | CCTTTGCTGT CTAGGAAGAG TCGTCAGGTC CCAGGTCCT |
| 5EQ ID NO: 42 rs8103142 F3 Primer | CCATCCTCCC AGCAGTT |
| SEQ ID NO: 43 rs8103142 B3 Primer | GACTCCCCCT CTCACC |
| SEQ ID NO: 44 rs8103142 Loop Primer F | TGATGGGATT GGAGGATGGC |
| SEQ ID NO: 45 rs8103142 Quenching Probe | AGCGGCACcT GCAGTCCTTC |

| LAMP-FLP method | |
|---|---|
| SEQ ID NO: 46 rs8103142 FIP | AGTGGCCCCT TCACCTTCTC CTTAGAGGGA AGGGGTAGCA |
| SEQ ID NO: 47 rs8103142 BIP | GGTGGGCCTG ACTCCCCCGG CTAACCTGTG GCTTTGC |
| SEQ ID NO: 48 rs8103142 F3 Primer | CAGCCAGTGT GGTCAG |
| SEQ ID NO: 49 rs8103142 B3 Primer | TCCCATCAGA GTGGTCTAAC C |
| SEQ ID NO: 50 rs8103142 Loop Primer F | CCATTGTCCC TCTCTCCTCT C |
| SEQ ID NO: 51 rs8103142 Loop Primer FB | AGCAGAAGCG ACTCTTCCTA G |
| SEQ ID NO: 52 rs8103142 Quencher Probe | AGCGGCACcT GCAGTCCTTC |

### LAMP-QP method

As the primer set and probe for identifying rs8103142 SNP, oligonucleotides consisting of the sequences of SEQ ID NOS: 40 to 45 were prepared. The 3' end of the quenching probe (SEQ ID NO: 45) was modified with the BODIPY FL molecule.

A total volume of 25 µL containing the template DNA, set of primers, probe, 1.4 mM each dNTPs, 20 mM Tris-HCl (pH 8.8), 10 mM KCl, 10 mM (NH₄)₂SO₄, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate af 0.2°C per second from 40°C to 80°C.

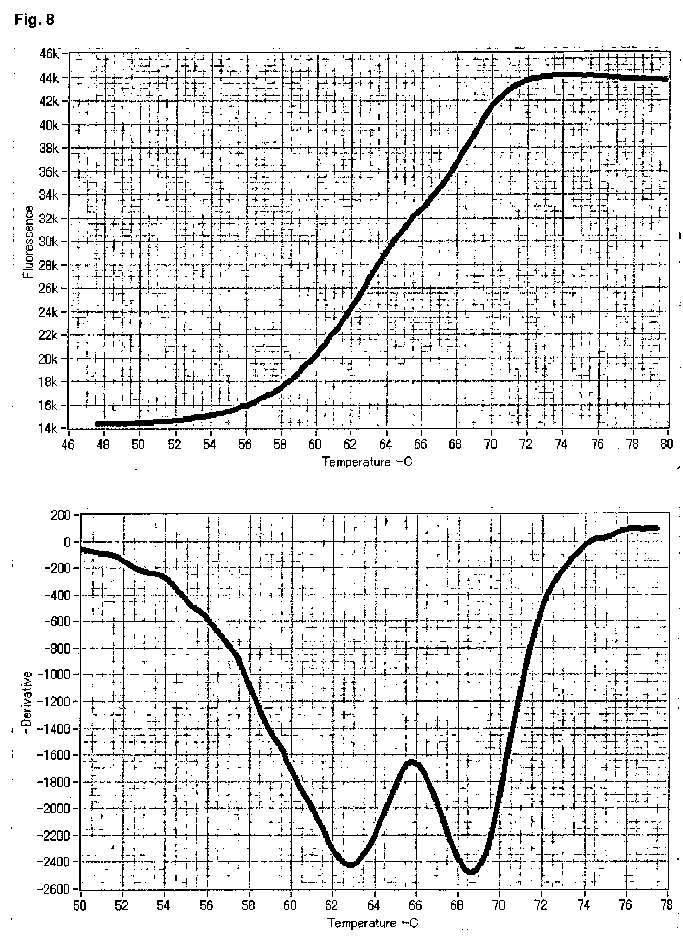

When the template extracted from the hair roots was used, a specific peak or peaks were formed around 62°C or 68°C, or around both the temperatures. Exemplary results are shown in Fig. 8. When the template DNA artificially synthesized so as to have T as the SNP concerned was used, a peak was formed around 62°C, and when the template DNA synthesized so as to have C as the SNP concerned was used, a peak was formed around 68°C.

### LAMP-FLP method

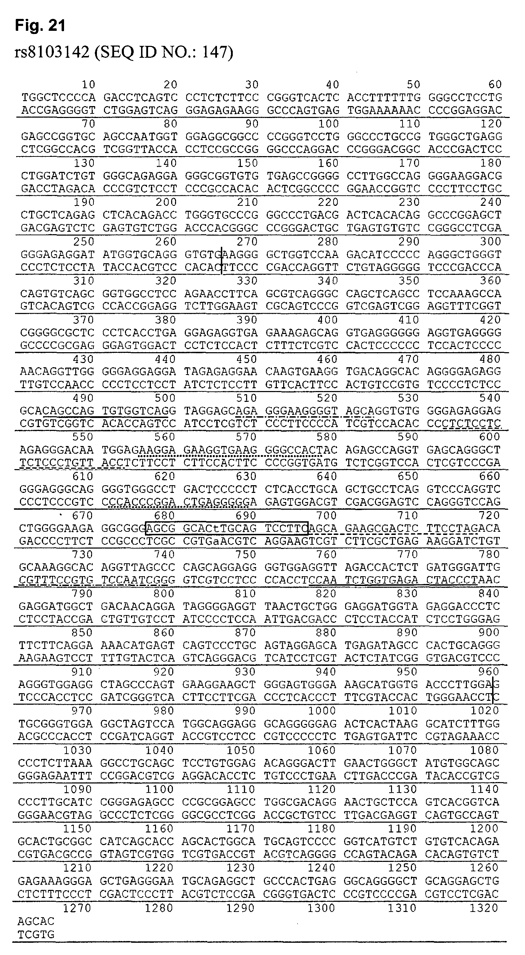

As the primer set and probe for identifying rs8103142 SNP, oligonucleotides consisting of the sequences of SEQ ID NOS: 46 to 52 were prepared. The 5' end of the loop primer FB (SEQ ID NO: 51) was modified with the FAM molecule, and the 3' end of the quencher probe (SEQ ID NO: 52) was modified with the DABCYL molecule. Further, positional relationship of the region artificially synthesized as the template DNA, the regions used for the LAMP method, and the probe is shown in Fig. 21.

A total volume of 25 µL containing the template DNA, set of primers, probe, 1.4 mM each dNTPs, 75 mM Tris-HCl (pH 8.0), 13 mM KCl, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with lowering the temperature at a rate of 0.05°C per second from 98°C to 30°C.

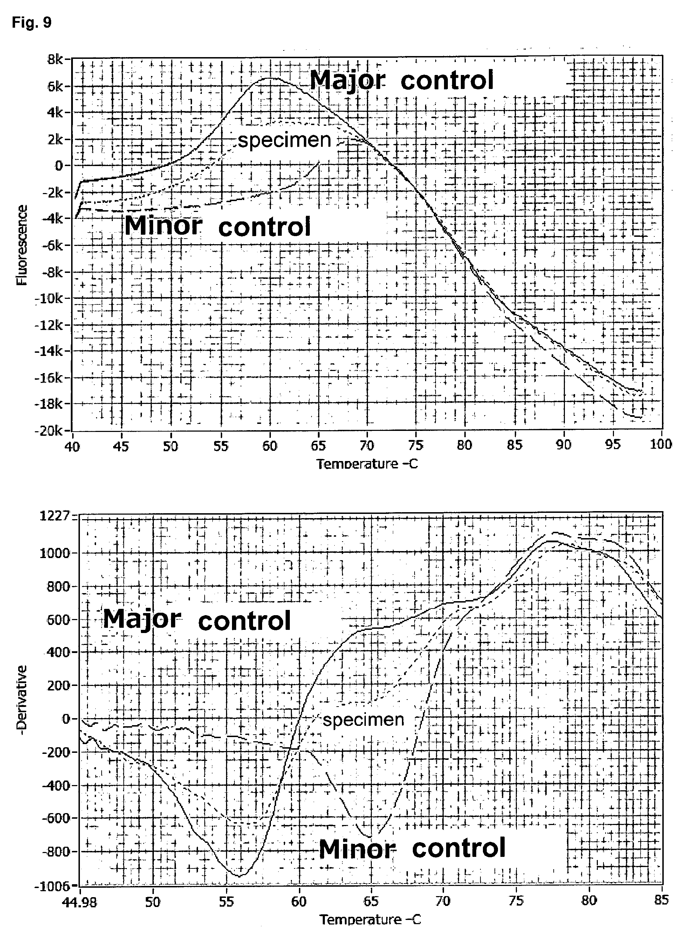

When the template extracted from the hair roots was used, a specific peak or peaks were formed around 55 to 58°C or 65°C, or around both the temperature range and temperature. Exemplary results are shown in Fig. 9. When the template DNA artificially synthesized so as to have T as the SNP concerned was actually used, a peak was formed around 58°C (Major control shown in Fig. 9), and when the template DNA synthesized so as to have C as the SNP concerned was used, a peak was formed around 67°C (Minor control shown in Fig. 9).

### [Comparison Evaluation Example 5: ITPA rs1127354 typing]

The sequences of the used primers and probes are shown below.

**[Table 6]**

| Probe and primer set for IL28B rs127354 typing | |
|---|---|
| LAMP-QP method | |
| SEQ ID NO: 53 rs1127354 FIP | GGTTGGATTT CTCTGTCTTC CTGTGTCCGG CACTTATCAG GGAA |
| SEQ ID NO: 54 rs1127354 BIP | TACGGTCAAT TTTCTGTGCC ACCACTCTTG GAACAGGTCG TTCA |
| SEQ ID NO: 55 rs1127354 F3 Primer | TGGAAATCTC ATCCGGCTC |
| SEQ ID NO: 56 rs1127354 B3 Primer | TCACATGGAG AATCACTAGA TGG |
| SEQ ID NO: 57 rs1127354 Loop Primer F | ACCTGACTTT CTGTGTGTCT GT |
| SEQ ID NO: 58 rs1127354 Quenching Probe | GGAGATAAGT TTcCATGCAC |
| | |

| LAMP-FLP method | |
|---|---|
| SEQ ID NO: 59 rs1127354 FIP | TCTCCATGTG AGCACGTGAG CGCCTGAGTT GGTAAGCT |
| SEQ ID NO: 60 rs1127354 BIP | CTCTTGGAAC AGGTCGTTCA GATTCAACAA AACAGAGACA TACGG |
| SEQ ID NO: 61 rs1127354 F3 Primer | CTGAAAGCCG GGGTGAG |
| SEQ ID NO: 62 rs1127354 B3 Primer | ACAGAGAAAT CCAACCATCT |
| SEQ ID NO: 63 rs1127354 Loop Primer F | TCTGCTGCCC ATCTCC |
| SEQ ID NO: 64 rs1127354 Loop Primer FB | TTTGGTGGCA CAGAAAATTG |
| SEQ ID NO: 65 rs1127354 Quencher Probe | GGAGATAAGT TTcCATGCAC |

### LAMP-OP method

As the primer set and probe for identifying rs1127354 SNP, oligonucleotides consisting of the sequences of SEQ ID NOS: 53 to 58 were prepared. The 3' end of the quenching probe (SEQ ID NO: 58) was modified with the BODIPY FL molecule.

A total volume of 25 µL containing the template DNA, set of primers, probe, 1.4 mM each dNTPs, 20 mM Tris-HCl (pH 8.8), 10 mM KCl, 10 mM (NH₄)₂SO₄, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate of 0.2°C per second from 40°C to 80°C.

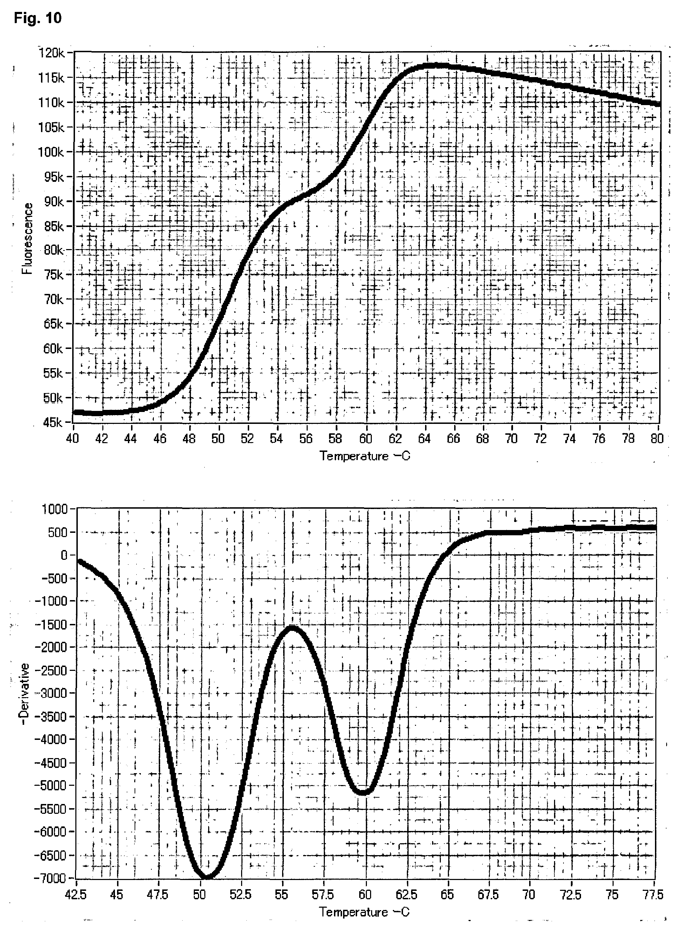

When the template extracted from the hair roots was used, a specif peak or peaks were formed around 49 to 51°C or 58 to 60°C, or around both the temperature ranges. Exemplary results are shown in Fig. 10. When the template DNA artificially synthesized so as to have C as the SNP concerned was used, a peak was formed around 58°C, and when the template DNA synthesized so as to have A as the SNP concerned was used, a peak was formed around 49°C. Further, when a mixture of these artificial template DNAs was used, peaks were formed around both the temperatures.

### LAMP-FLP method

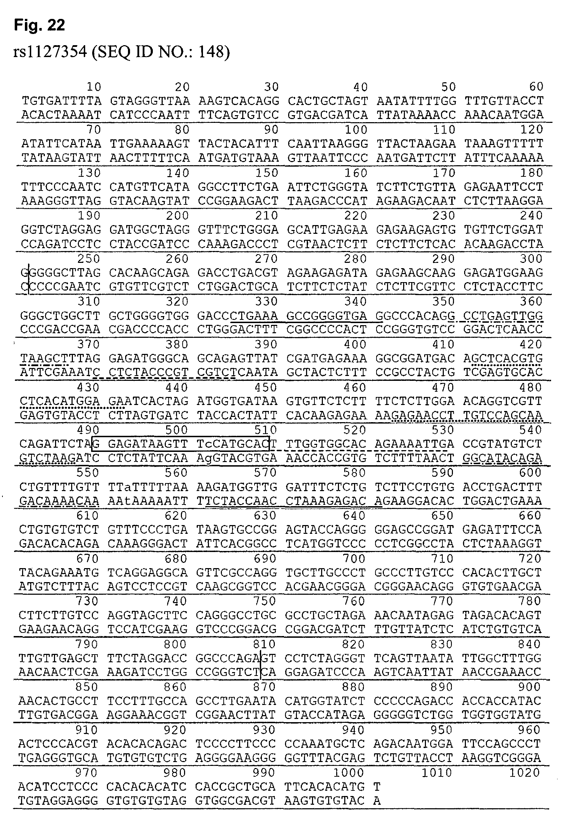

As the primer set and probe for identifying rs1127354 SNP, oligonucleotides consisting of the sequences of SEQ ID NOS: 59 to 65 were prepared. The 5' end of the loop primer FB (SEQ ID NO: 64) was modified with the FAM molecule, and the 3' end of the quencher probe (SEQ ID NO: 65) was modified with the DABCYL molecule. Further, positional relationship of the region artificially synthesized as the template DNA, the regions used for the LAMP method, and the probe is shown in Fig. 22.

A total volume of 25 µL containing the template DNA, set of primers, probe, 1.4 mM each dNTPs, 75 mM Tris-HCl (pH 8.0), 13 mM KCl, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with lowering the temperature at a rate of 0,05°C per second from 98°C to 30°C.

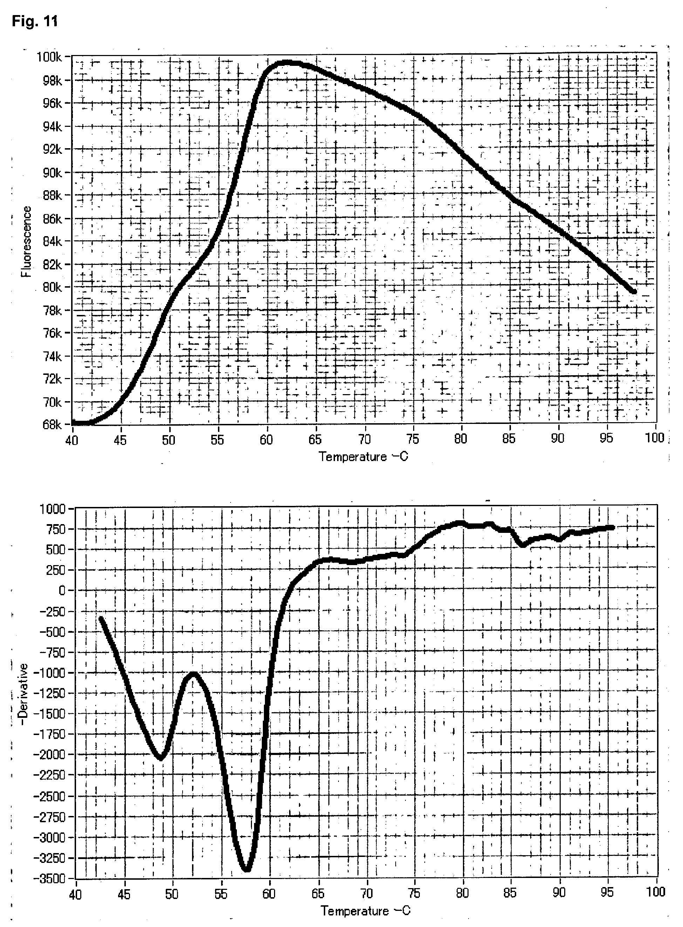

When the template extracted from the hair roots was used, a specific peak or peaks were formed around 48 to 51°C, or 56 to 59°C, or around the both temperature ranges. Exemplary results are shown in Fig. 11. When the template DNA artificially synthesized so as to have C as the SNP concerned was used, a peak was formed around 61°C, and when the template DNA synthesized so as to have A as the SNP concerned was used, a peak was formed around 51°C. Further, when a mixture of these artificial template DNAs was used, peaks were formed around both the temperatures. Also in the case of the identification with the template DNA extracted from hair roots, similar peak formation was observed, and the results agreed with the results obtained by the quenching probe method and the direct sequencing method.

### [Comparison Evaluation Example 6: ITPA rs7270101 typing]

The sequences of the used primers and probes are shown below.

**[Table 7]**

| Probe and primer set for ITPA rs7270101 typing | |
|---|---|
| LAMP-QP method | |
| SEQ ID NO: 66 rs7270101 FIP | AGTGCATGGA AACTTATCTC CTAGAGGAGA ATCACTAGAT GGTGA |
| SEQ ID NO: 67 ras7270101 BIP | GTOGCACAGA AAATTGACCG TATGTCAGGT CACAGGAAGA CAG |
| SEQ ID NO: 68 rs7270101 F3 Printer | GATGAGAAM GCGGATGA |
| SEQ ID NO: 69 rs7270101 B3 Primer | GGGAAACAGA CACACAGA |
| SEQ ID NO: 70 rs7270101 Loop Primer F | ACCACCTGTT CCAAGAGAAA AGAGA |
| SEQ ID NO: 71 rs7270101 Quenching Probe | CTTTTAAAAA gAAAACAAAA C |

| LAMP-FLP method | |
|---|---|
| SEQ ID NO: 72 rs7270101 FIP | TCCCTGATAA GTGCCGGAGT ACCCGAACTG CCTCCTCACA TT |
| SEQ ID NO: 73 rs7270101 BIP | AGTCAGGTCA CACGAMACA GAGATAAGTT TCCATGCACT TTGGTG |
| SEQ ID NO: 74 rs7270101 F3 Primer | GAGCAAGTGT GGGACAAGG |
| SEQ ID NO: 75 rs7270101 B3 Primer | AACAGGTCGT TCAGATTCTA GG |
| SEQ ID NO: 70 rs7270101 Loop Primer F | GCCGGATGAG ATTTCCATAC AGA |
| SEQ ID NO: 77 rs7270101 Loop Primer FB | ATACGGTCAA TTTTCTGTGC |
| SEQ ID NO: 78 rs7270101 Quencher Probe | AAAAAcAAAA CAAAACAGAG AC |

### LAMP-QP method

As the primer set and probe for identifying rs7270101 SNP, oligonucleotides consisting of the sequences of SEQ ID NOS: 66 to 71 were prepared. The 3' end of the quenching probe (SEQ ID NO: 71) was modified with the BODIPY FL molecule.

A total volume of 25 µL containing the template DNA, set of primers, probe, 1.4 mM each dNTPs, 20 mM Tris-HCl (pH 8.8), 10 mM KCl, 10 mM (NH₄)₂SO₄, 8 mM MgSO₄, 0.1% Tween 20, and 8 units of Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with raising the temperature at a rate of 0.2°C per second from 37.5°C to 65°C.

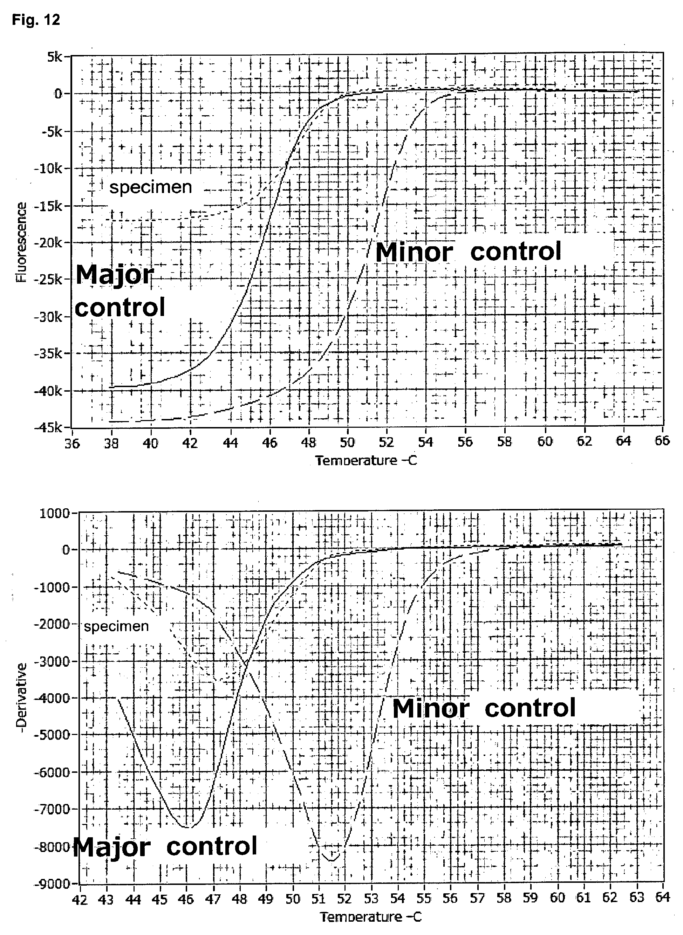

When the template extracted from the hair roots was used, a specific peak or peaks were formed around 46°C or 52°C, or around both the temperatures. Exemplary results are shown in Fig. 12. When the template DNA artificially synthesized so as to have A as the SNP concerned was used, a peak was formed around 46°C (Major control shown in Fig. 12), and when the template DNA synthesized so as to have C as the SNP concerned was used, a peak was formed around 52°C (Minor control shown in Fig. 12).

### LAMP-FLP method

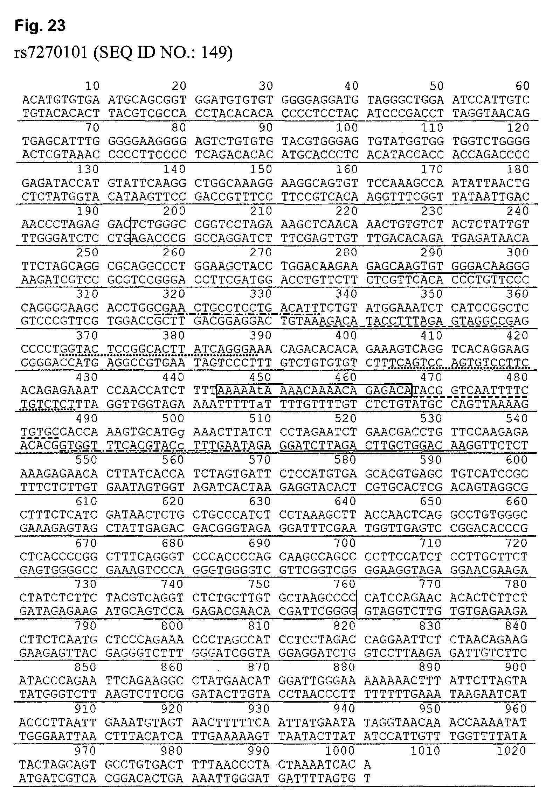

As the primer set and probe for identifying rs7270101 SNP, oligonucleotides consisting of the sequences of SEQ ID NOS: 72 to 78 were prepared. The 5' end of the loop primer FB (SEQ ID NO: 77) was modified with the FAM molecule, and the 3' end of the quencher probe (SEQ ID NO: 78) was modified with the DABCYL molecule. Further, positional relationship of the region artificially synthesized as the template DNA, the regions used for the LAMP method, and the probe is shown in Fig. 23.

A total volume of 25 µL containing the template DNA, set of primers, probe, 1.4 mM each dNTPs, 75 mM Tris-HCl (pH 8.0), 13 mM KCl, 8 mM MgSO₄, 0.1% Tween 20, and 8 units af Bst DNA Polymerase (Nippon Gene) was incubated on Genie (registered trademark) II (OptiGene) for 30 minutes, and then a probe association curve analysis was performed. Specifically, after treatments at 63°C for 30 minutes and 98°C for 5 minutes, change of fluorescence intensity was measured with lowering the temperature at a rate of 0.05°C per second from 98°C to 30°C.

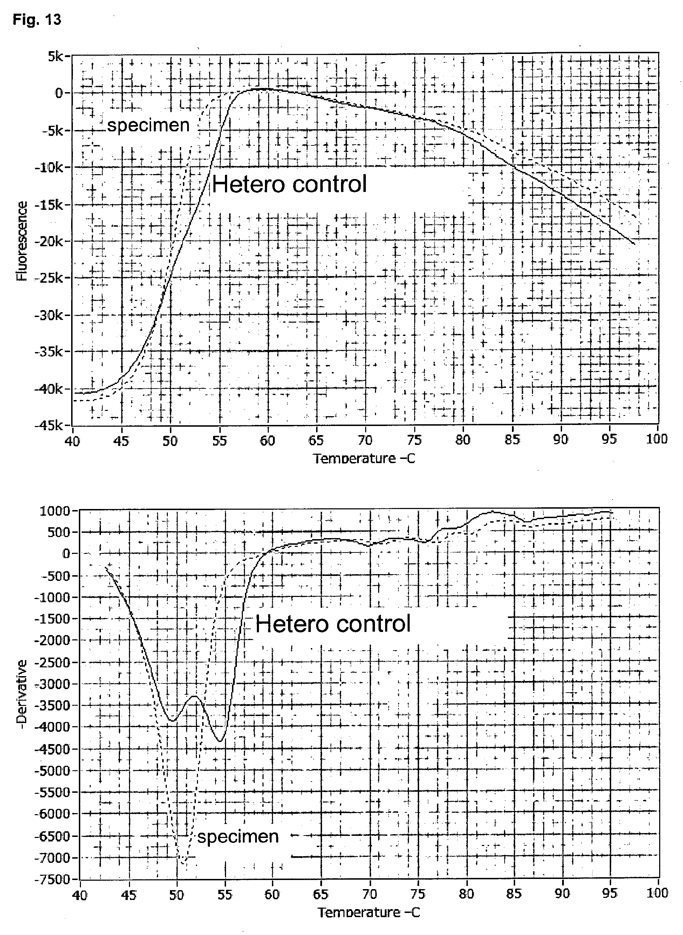

When the template extracted from the hair roots was used, a specific peak or peaks were formed around 50°C or 55°C, or around both the temperatures. Exemplary results are shown in Fig. 13. When a mixture of the template DNA artificially synthesized so as to have A as the SNP concerned, and the template DNA synthesized so as to have C as the SNP concerned was used, peaks were formed around 50°C and 55°C (Hetero control shown in Fig. 13).

### [Design Examples 7 to 13]

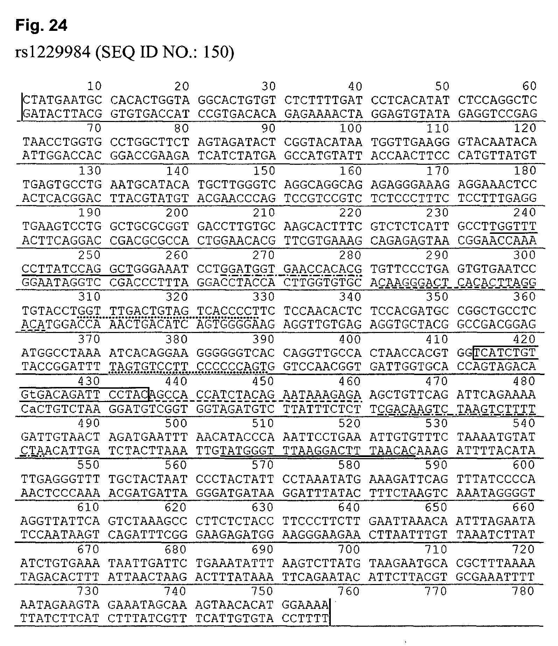

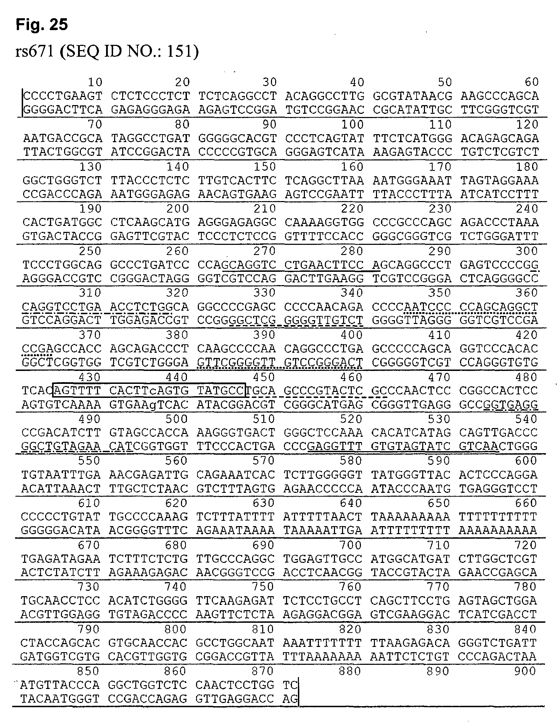

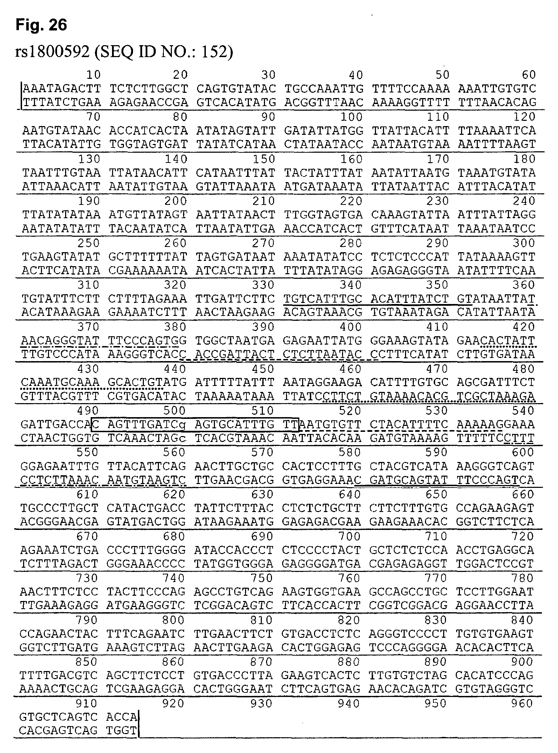

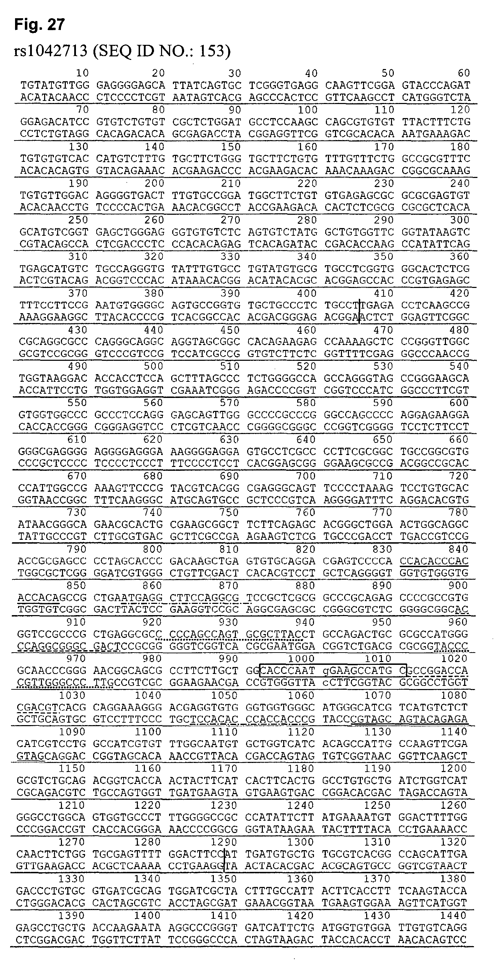

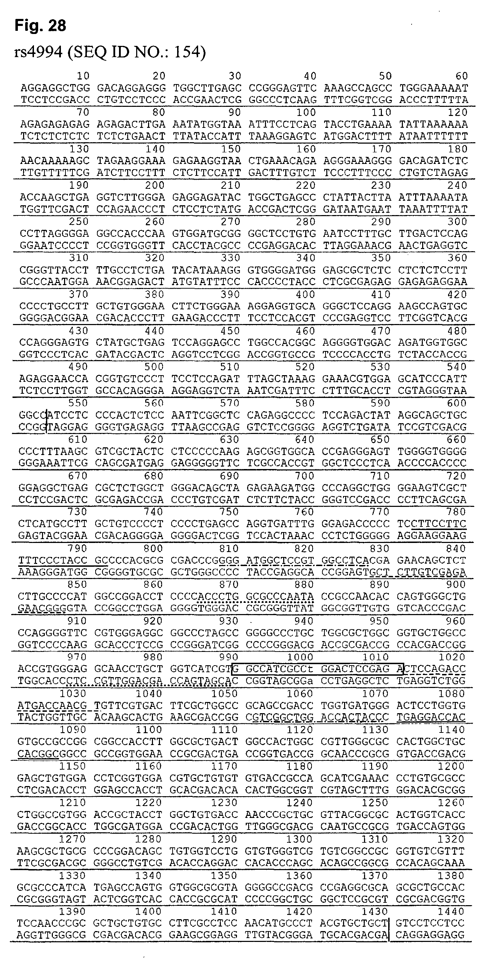

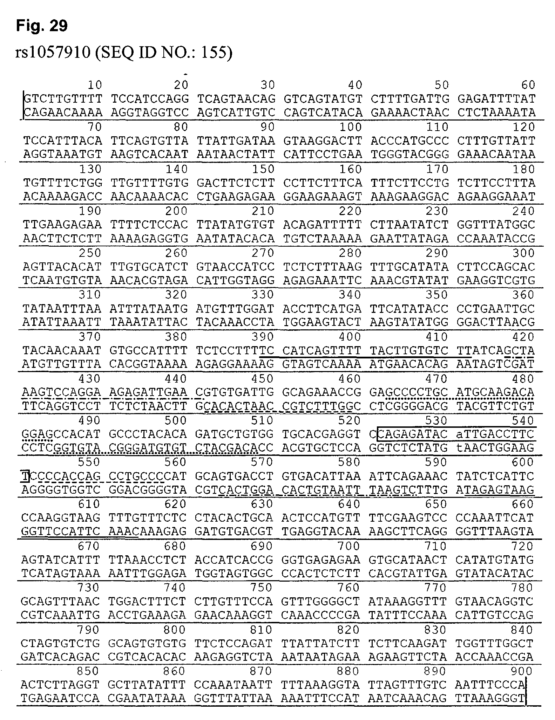

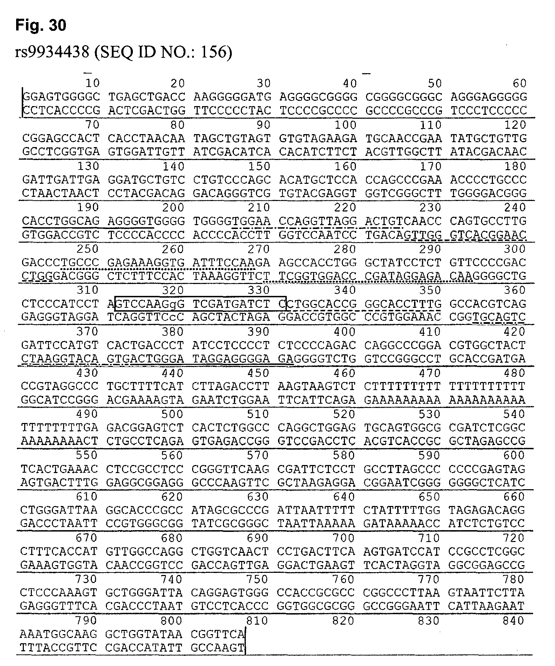

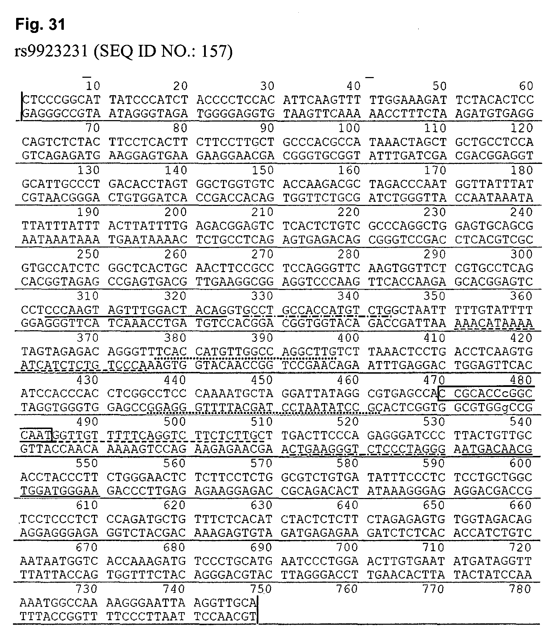

In the same manner as those of the aforementioned comparison evaluation examples, there were designed LAMP-FLP primers and probes for ADH1B (human alcohol dehydrogenase 1B) rs 1229984, ALDH2 (human aldehyde dehydrogenase 2) rs671, UCP1 (human uncoupling protein 1) rs1800592, ADRB2 (human adrenoreceptor beta2) rs1042713, ADRB3 (human adrenoreceptor beta3) rs4994, CYP2C9*3 (cytochrome p450 2c9*3) rs1057910, VKORC1 #1 (vitamin K epoxide reductase complex 1 #1) ras9934438, and VKORC1 #2 (vitamin K epoxide reductase complex 1 #2) rs9923231, as shown in the following table. Further, positional relationships of the regions cloned as the template DNA, the regions used for the LAMP method, and the probes are shown in Figs. 24 to 31, respectively.

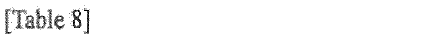

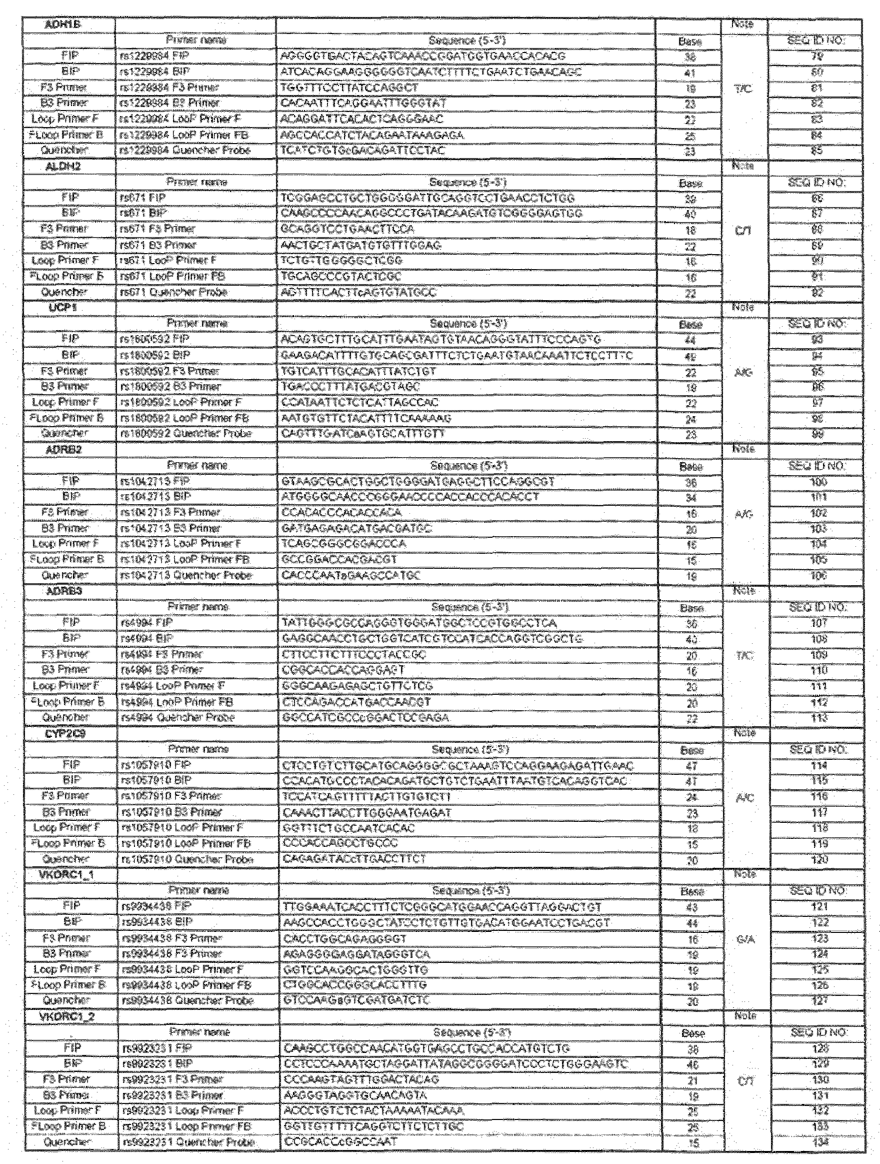

### [Comparison of probe performances]

Performances of probes of which 5' end portions were deleted, and probes of which 3' end portions were deleted were compared. The following probes were prepared, and evaluation was performed in the same manner as that of Comparison Evaluation Example 1 except for the probe.

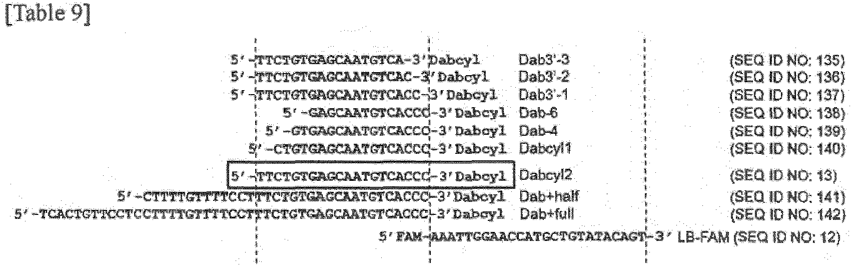

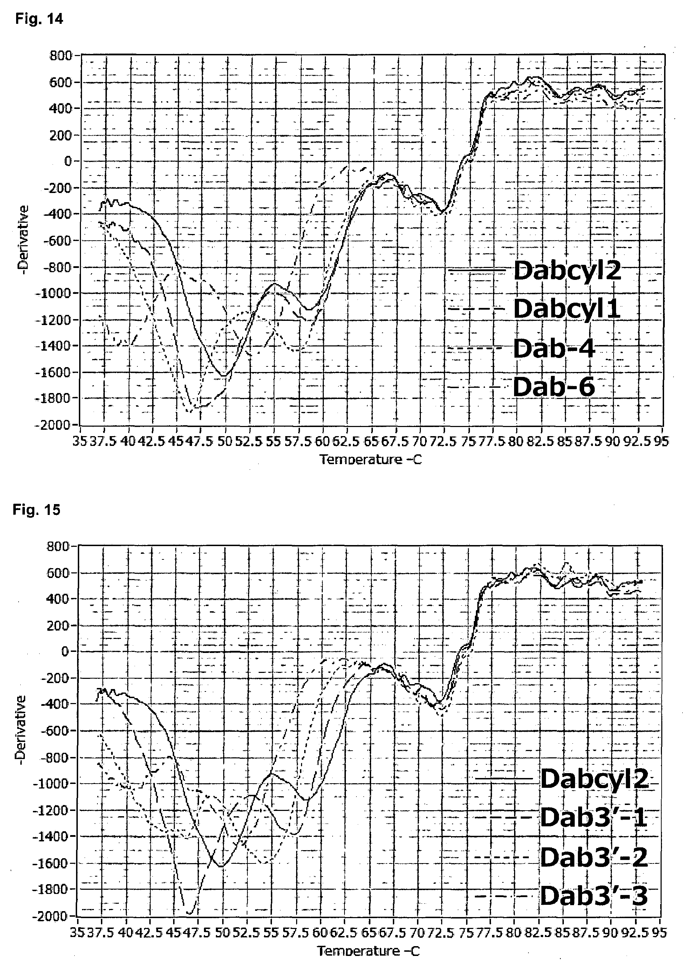

In order to investigate the influence of the deletion of 5' end portion, Dabcyl 2 and those corresponding to Dabcyl 2 of which 5' end portion was deleted, Dabcyl 1, Dab-4, and Dab-6, were compared. The results are shown in Fig. 14. When the probe chain length became shorter, the Tm value generally lowered, but there was observed a tendency that a shorter probe provided larger Tm difference for the cases of full match and mismatch. Shorter chain length did not provide improved peak resolution. It is considered that the improved resolution obtained with Dab-6 was provided by elimination of the formation of the stem-loop structure or primer multi-mer.

In order to investigate the influence of deletion of 3' end portion, Dabcyl 2 and those corresponding to Dabcyl 2 of which 3' end portion was deleted, Dab3'-1, Dab3'-2 and Dab3'-3, were compared. The results are shown in Fig. 15. The gap of one nucleotide did not affect the detection, but the gaps of 2 and 3 nucleotides provided turbulence of peaks. It was considered that it might result from the gaps or shorter chain lengths.

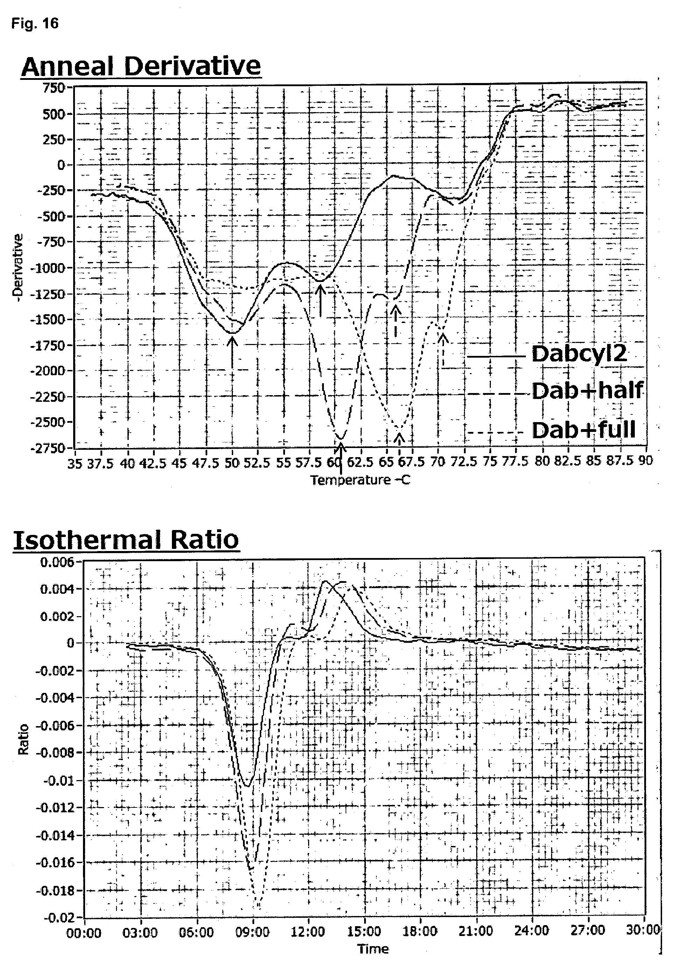

In order to investigate the region for which the probe can be designed, Dabcyl 2, Dab+half (corresponding to Dabcyl 2 extended on the 5' end side for the half of the B1c region), and Dab+full (corresponding to Dabcyl 2 extended on the 5' end side for the whole H1c region) were compared. The results are shown in Fig. 16. A longer probe chain length provided a higher Tm value, but provided a smaller Tm difference between the cases of full match and mismatch. It was found that inclusion of the B1c region in the probe did not affect the amplification reaction on the basis of the isothermal ratios.

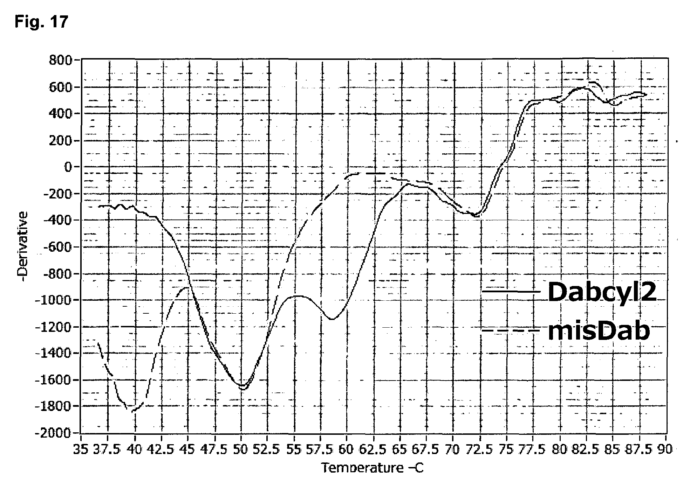

The influence of mismatch was investigated. The 7th G of Dabcyl 2 (SEQ ID NO: 13 mentioned above) was changed to T to prepare misDab (SEQ ID NO: 143), and it was used. The results are shown in Fig. 23. Introduction of the common mismatch markedly reduced the Tm value, but markedly improved the peak resolution (Fig. 17).

**[Table 10]**

| **Dabcyl2** | | **misDab** |
|---|---|---|
| 10 A A C T G G A T G C T A T T C T G G C C 5' 3' | | 3'      A — A C         T G         G A — T Changed to "T"   G — C T — A T   T   C   T   G — C   C   C 5'                              5' |
| Sequence Listing Free Text | | |
| [0133] | | |
| SEQ ID NO: 1 | rs8099917 FIP (LAMP-QP) | |
| SEQ ID NO: 2 | rs8099917 BIP (LAMP-QP) | |
| SEQ ID NO: 3 | rs8099917 F3 primer (LAMP-QP) | |
| SEQ ID NO: 4 | rs8099917 B3 primer (LAMP-QP) | |
| SEQ ID NO: 5 | rs8099917 Loop primer F (LAMP-QP) | |
| SEQ ID NO 6 | rs8099917 Quenching probe (LAMP-QP) | |
| SEQ ID NO: 7 | rs8099917 FIP (LAMP-FLP) | |
| SEQ ID NO: 8 | rs8099917 BIP (LAMP-FLP) | |
| SEQ ID NO: 9 | rs8099917 F3 primer (LAMP-FLP) | |
| SEQ ID NO: 10 | rs8099917 B3 primer (LAMP-FLP) | |
| SEQ ID NO: 11 | rs8099917 Loop primer F (LAMP-FLP) | |
| SEQ ID NO: 12 | rs8099917 Loop primer FB (LAMP-FLP) | |
| SEQ ID NO: 13 | rs8099917 Quencher probe (LAMP-FLP), Dabeyl 2 | |
| SEQ ID NO: 14 | ras12979860 FIP (LAMP-QP) | |
| SEQ ID NO: 15 | rs129798G0 BIP (LAMP-QP) | |
| SEQ ID NO: 16 | rs12979860 F3 primer (LAMP-QP) | |
| SEQ ID NO: 17 | rs12979860 B3 primer (LAMP-QP) | |
| SEQ ID NO: 18 | rs12979860 Loop primer F (LAMP-QP) | |
| SEQ ID NO: 19 | ras12979860 Quenching probe (LAMP-QP) | |
| SEQ ID NO: 20 | rs12979860 FIP (LAMP-FLP) | |
| SEQ ID NO: 21 | rs12979860 BIP (LAMP-FLP) | |
| SEQ ID NO: 22 | rs12979860 F3 primer (LAMP-FLP) | |
| SEQ ID NO: 23 | rs12979860 B3 primer (LAMP-FLP) | |
| SEQ ID NO: 24 | rs12979860 Loop primer F (LAMP-FLP) | |
| SEQ ID NO: 25 | rs12979860 Loop primer FB (LAMP-FLP) | |
| SEQ ID NO: 26 | rs12979860 Quencher probe (LAMP-FLP) | |
| SEQ ID NO: 27 | rs11881222 FIP (LAMP-QP) | |
| SEQ ID NO: 28 | rs11881222 BIP (LAMP-QP) | |
| SEQ ID NO: 29 | rs 11881222 F3 primer (LAMP-QP) | |
| SEQ ID NO: 30 | rs11881222 B3 primer (LAMP-QP) | |
| SEQ ID NO: 31 | rs11881222 Loop primer F (LAMP-QP) | |
| SEQ ID NO: 32 | rs1881222 Quenching probe (LAMP-QP) | |
| SEQ ID NO: 33 | rs11881222 FIP (LAMP-FLP) | |
| SEQ ID NO: 34 | rs11881222 BIP (LAMP-FLP) | |
| SEQ ID NO: 35 | rs11881222 F3 primer (LAMP-FLP) | |
| SEQ ID NO: 36 | rs11881222 B3 primer (LAMP-FLP) | |
| SEQ ID NO: 37 | rs11881222 Loop primer F (LAMP-FLP) | |
| SEQ ID NO: 38 | rs11881222 Loop primer FB (LAMP-FLP) | |
| SEQ ID NO: 39 | rs11881222 Quencher probe (LAMP-FLP) | |
| SEQ ID NO: 40 | rs8103142 FIP (LAMP-QP) | |
| SEQ ID NO: 41 | rs8103142 BIP (LAMP-QP) | |
| SEQ ID NO: 42 | rs8103142 F3 primer (LAMP-QP) | |
| SEQ ID NO: 43 | rs8103142 B3 primer (LAMP-QP) | |
| SEQ ID NO: 44 | rs8103142 Loop primer F (LAMP-QP) | |
| SEQ ID NO: 45 | r58103142 Quenching probe (LAMP-QP) | |
| SEQ ID NO: 46 | rs8103142 FIP (LAMP-FLP) | |
| SEQ ID NO: 47 | rs8103142 BIP (LAMP-FLP) | |
| SEQ ID NO: 48 | rs8103142 F3 primer (LAMP-FLP) | |
| SEQ ID NO: 49 | rs8103142 B3 primer (LAMP-FLP) | |
| SEQ ID NO: 50 | rs8103142 Loop primer F (LAMP-FLP) | |
| SEQ ID NO: 51 | rs8103142 Loop primer FB (LAMP-FLP) | |
| SEQ ID NO: 52 | rs8103142 Quencher probe (LAMP-FLP) | |
| SEQ ID NO: 53 | rs 1127354 FIP (LAMP-QP) | |
| SEQ ID NO: 54 | rs1127354 BIP (LAMP-QP) | |
| SEQ ID NO: 55 | rs1127354 F3 primer (LAMP-QP) | |
| SEQ ID NO: 56 | rs1127354 B3 primer (LAMP-QP) | |
| SEQ ID NO: 57 | rs1127354 Loop primer (LAMP-QP) | |
| SEQ ID NO: 58 | rs 1127354 Quenching probe (LAMP-QP) | |
| SEQ ID NO: 59 | rs1127354 FIP(LAMP-FLP) | |
| SEQ ID NO: 60 | rs1127354 BIP (LAMP-FLP) | |
| SEQ ID NO: 61 | rs1127354 F3 primer (LAMP-FLP) | |
| SEQ ID NO: 62 | rs1127354 B3 primer (LAMP-FLP) | |
| SEQ ID NO: 63 | rs1127354 Loop primer F (LAMP-FLP) | |
| SEQ ID NO: 64 | rs1127354 Loop primer FB (LAMP-FLP] | |
| SEQ ID NO: 65 | rs1127354 Quencher probe (LAMP-FLP) | |
| SEQ ID NO: 66 | rs7270101 FIP (LAMP-QP) | |
| SEQ ID NO: 67 | rs7270101 BIP (LAMP-QP) | |
| SEQ ID NO: 68 | rs7270101 F3 primer (LAMP-QP) | |
| SEQ ID NO: 69 | rs7270101 B3 primer (LAMP-QP) | |
| SEQ ID NO: 70 | rs7270101 Loop primer F (LAMP-QP) | |
| SEQ ID NO: 71 | rs7270101 Quenching probe (LAMP-QP) | |
| SEQ ID NO: 72 | ras7270101 FIP (LAMP-FLP) | |
| SEQ ID NO: 73 | ras7270101 BIP (LAMP-FLP) | |
| SEQ ID NO: 74 | rs7270101 F3 primer (LAMP-FLP) | |
| SEQ ID NO: 75 | ras7270101 B3 primer (LAMP-FLP) | |
| SEQ ID NO: 76 | rs7270101 Loop primer F (LAMP-FLP) | |
| SEQ ID NO: 77 | rs7270101 Loop primer FB (LAMP-FLP) | |
| SEQ ID NO: 78 | rs7270101 Quencher probe (LAMP-FLP) | |
| SEQ ID NO: 79 | rs1229984 FIP (LAMP-FLP) | |
| SEQ ID NO: 80 | rs1229984 BIP (LAMP-FLP) | |
| SEQ ID NO: 81 | rs1229984 F3 primer (LAMP-FLP) | |
| SEQ ID NO: 82 | ras122998483 primer (LAMP-FLP) | |
| SEQ ID NO: 83 | rs1229984 Loop primer F (LAMP-FLP) | |
| SEQ ID NO: 84 | rs1229984 Loop primer FB (LAMP-FLP) | |
| SEQ ID NO: 85 | rs1229984 Quencher probe (LAMP-FLP) | |
| SEQ ID NO: 86 | rs671 FIP (LAMP-FLP) | |
| SEQ ID NO: 87 | rs671 BIP (LAMP-FLP) | |
| SEQ ID NO: 88 | rs671 F3 primer (LAMP-FLP) | |
| SEQ ID NO: 89 | rs671 B3 primer (LAMP-FLP) | |
| SEQ ID NO: 90 | rs671 Loop primer F (LAMP-FLP) | |
| SEQ ID NO: 91 | rs671 Loop primer FB (LAMP-FLP) | |
| SEQ ID NO: 92 | rs671 Quencher probe (LAMP-FLP) | |
| SEQ ID NO: 93 | rs1800592 FIP (LAMP-FLP) | |
| SEQ ID NO: 94 | rs1800592 BIP (LAMP-FLP) | |
| SEQ ID NO: 95 | rs1800592 F3 primer (LAMP-FLP) | |
| SEQ ID NO: 96 | rs1800592 B3 primer (LAMP-FLP) | |
| SEQ ID NO: 97 | rs1800592 Loop primer F (LAMP-FLP) | |
| SEQ ID NO: 98 | rs1800592 Loop primer FB (LAMP-FLP) | |
| SEQ ID NO: 99 | rs1800592 Quencher probe (LAMP-FLP) | |
| SEQ ID NO: 100 | rs1042713 FIP (LAMP-FLP) | |
| SEQ ID NO: 101 | rs1042713 BIP (LAMP-FLP) | |
| SEQ ID NO: 102 | rs1042713 F3 primer (LAMP-FLP) | |
| SEQ ID NO: 103 | ras1042713 B3 primer (LAMP-FLP) | |
| SEQ ID NO: 104 | rs1042713 Loop primer F (LAMP-FLP) | |
| SEQ ID NO: 105 | rs1042713 Loop primer FB (LAMP-FLP) | |
| SEQ ID NO: 106 | rs1042713 Quencher probe (LAMP-FLP) | |
| SEQ ID NO: 107 | rs4994 FIP (LAMP-FLP) | |
| SEQ ID NO: 108 | rs4994 BIP (LAMP-FLP) | |
| SEQ ID NO: 109 | rs4994 F3 primer (LAMP-FLP) | |
| SEQ ID NO: 110 | rs4994 B3 primer (LAMP-FLP) | |
| SEQ ID NO: 111 | rs4994 Loop primer F (LAMP-FLP) | |
| SEQ ID NO: 112 | rs4994 Loop primer FB (LAMP-FLP) | |
| SEQ ID NO: 113 | rs4994 Quencher probe (LAMP-FLP) | |
| SEQ ID NO: 114 | rs1057910 FIP (LAMP-FLP) | |
| SEQ ID NO: 115 | rs 1057910 BIP (LAMP-FLP) | |
| SEQ ID NO: 116 | rs1057910 F3 primer (LAMP-FLP) | |
| SEQ ID NO: 117 | rs057910 B3 primer (LAMP-FLP) | |
| SEQ ID NO: 118 | rs1057910 Loop primer F (LAMP-FLP) | |
| SEQ ID NO: 119 | rs1057910 Loop primer FB (LAMP-FLP) | |
| SEQ ID NO: 120 | rs1057910 Quencher probe (LAMP-FLP) | |
| SEQ ID NO: 121 | rs9934438 FIP (LAMP-FLP) | |
| SEQ ID NO: 122 | rs9934438 BIP (LAMP-FLP) | |
| SEQ ID NO: 123 | rs9934438 F3 primer (LAMP-FLP) | |
| SEQ ID NO: 124 | rs9934438 F3 primer (LAMP-FLP) | |
| SEQ ID NO: 125 | rs9934438 Loop primer F (LAMP-FLP) | |
| SEQ ID NO: 126 | rs9934438 Loop primer FB (LAMP-FLP) | |
| SEQ ID NO: 127 | rs9934438 Quencher probe (LAMP-FLP) | |
| SEQ ID NO: 128 | rs9923231 FIP (LAMP-FLP) | |
| SEQ ID NO: 129 | rs9923231 BIP (LAMP-FLP) | |
| SEQ ID NO: 130 | rs9923231 F3 primer (LAMP-FLP) | |
| SEQ ID NO: 131 | rs9923231 B3 primer (LAMP-FLP) | |
| SEQ ID NO: 132 | rs9923231 Loop primer F (LAMP-FLP) | |
| SEQ ID NO: 133 | rs9923231 Loop primer FB (LAMP-FLP) | |
| SEQ ID NO: 134 | rs992323 Quencher probe (LAMP-FLP) | |
| SEQ ID NO: 135 | Dab3'-3 | |
| SEQ ID NO: 136 | Dab3'-2 | |
| SEQ ID NO: 137 | Dab3'-1 | |
| SEQ ID NO: 138 | Dab-6 | |
| SEQ ID NO: 139 | Dab-4 | |
| SEQ ID NO: 140 | Dabcyl 1 | |
| SEQ ID NO: 141 | Dab+half | |
| SEQ ID NO: 142 | Dab+full | |
| SEQ ID NO: 143 | misDab | |
| SEQ ID NO: 144 | DNA fragment containing ras8099917 | |
| SEQ ID NO: 145 | DNA fragment containing rs12979860 | |
| SEQ ID NO: 146 | DNA fragment containing rs11881222 | |
| SEQ ID NO: 147 | DNA fragment containing rs8103142 | |
| SEQ ID NO: 148 | DNA fragment containing rs1127354 | |
| SEQ ID NO: 149 | DNA fragment containing rs7270101 | |
| SEQ ID NO: 150 | DNA ADH1B fragment containing rs1229984 | |
| SEQ ID NO: 151 | DNA ALDH2 fragment containing rs671 | |
| SEQ ID NO: 152 | UCP1 DNA fragment containing rs1800592 | |
| SEQ ID NO: 153 | ADRB2 DNA fragment containing rs1042713 | |
| SEQ ID NO: 154 | ADRB3 DNA fragment containing rs4994 | |
| SEQ ID NO: 155 | CYP2C9*3 DNA fragment containing rs1057910 | |
| SEQ ID NO: 156 | VKORC1 #1 DNA fragment containing rs9934438 | |
| SEQ ID NO: 157 | VKORC1 #2 DNA fragment containing rs9923231 | |

## Claims

1. A method for detecting a target site, which is an LAMP method using four kinds of primers, FIP, F3 primer, BIP, and B3 primer, which are designed on the basis of six regions of template polynucleotide, F1 region, F2 region, F3 region, B1 region, B2 region, and B3 region:
wherein:
the template polynucleotide contains the target site, the four kinds of primers are designed so that the target site exists between the F1 region and the F2 region, or between the B1 region and the B2 region;
a loop primer designed on the basis of a region between the F1 region and the F2 region or between the B1 region and the B2 region on the side on which the target site exists, and an oligonucleotide probe that can associate with a region including the target site are used,
the loop primer and the probe are designed so that the 5' end of the loop primer and the 3' end of the probe associate with the template polynucleotide at positions close to each other,
one of the loop primer and the probe is modified with a fluorescent molecule around the 5' end in the case of the loop primer or the 3' end in the case of the probe,
and the other is modified with a quenching molecule.

2. The method according to claim 1, wherein the target site is designed so as to locate 3 to 20 nucleotides upstream from the 5' end of the associated loop primer.

3. The method according to claim 1 or 2, wherein the target site is a single nucleotide polymorphism (SNP).

4. The method according to claim 3, wherein SNP is selected from the group consisting of rs8099917, rs12978960, rs11881222, rs8103142, rs1127354, rs7270101, rs1229984, rs671, rs1800592, rs1042713, rs4994, rs1057910, rs9934438, and rs9923231.

5. The method according to any one of claims 1 to 4, which comprises the step of changing temperature of system within a temperature range where association or dissociation of the probe occurs, and monitoring change of fluorescence occurring during the temperature change.

6. The method according to claim 5, wherein, after an isothermal reaction, temperature of the system is raised and then lowered, during which occurring change of fluorescence is monitored.

7. The method according to claim 5 or 6, which comprises the step of calculating ratio of change of fluorescence by primary differentiation.

8. The method according to claim 7, which comprises the step of further differentiating a value of ratio of change of fluorescence calculated by the primary differentiation.

9. The method according to any one of claims 1 to 8, wherein the probe is modified with the fluorescence molecule around the 3' end thereof, and the loop primer is modified with the quenching molecule around the 5' end thereof.

10. The method according to any one of claims 1 to 9, wherein DNA contained in a sample obtained by treating a specimen derived from a living organism with a protease is used as the template polynucleotide.

11. The method according to claim 10, wherein the sample is subjected to a treatment for inactivating the protease.

12. The method according to claim 10 or 11, wherein the specimen is selected from the group consisting of hair, blood, oral mucosa, nail, bloodstain, and umbilical cord.

13. The method according to any one of claims 1 to 12, wherein DNA synthetase used for the amplification reaction is a DNA polymerase I derived from a *Geobacillus* bacterium, or a Csa DNA polymerase or 96-7 DNA polymerase isolated from compost obtained by fermentation at a temperature of 85°C or higher.

14. A kit for carrying out the method according to any one of claims 1 to 13, which comprises:
four kinds of primers (FIP, F3 primer, BIP, and B3 primer) designed on the basis of six regions of a template polynucleotide (F1 region, F2 region, F3 region, B1 region, B2 region, and B3 region);
a loop primer designed on the basis of a region between the F 1 region and the F2 region or between the B 1 region and the B2 region on the side on which the target site exists, and
an oligonucleotide probe that can associate with a region including the target site, and
wherein:
the four kinds of the primers are designed so that the target site locates between the F1 region and the F2 region or between the B1 region and the B2 region;
the loop primer and the probe are designed so that the 5' end of the loop primer and the 3' end of the probe associate with the template polynucleotide at positions close to each other; and
one of the loop primer and the probe is modified with a fluorescent molecule around the 5' end in the case of the loop primer or the 3' end in the case of the probe, and the other is modified with a quenching molecule.

15. The kit according to claim 14, which is for detecting rs8099917, and is used for predicting effect of the combined therapy of peginterferon and ribavirin in a therapeutic treatment of hepatitis C.

16. A method for treating a specimen for a gene amplification reaction, which comprises the step of treating the specimen with a protease.

17. The method according to claim 16, wherein the gene amplification reaction is performed by the PCR method, SDA method, TMA method, NASBA method, TRC method, ICAN method, SmartAmp method, or RCA method.


### Sequence Listing

SEQUENCE LISTING

<110>  NIPPON GENE CO., LTD.

<120>  Primer-Probe Sets for Genetic Polymorphism Typing, and Use thereof

<130>  145015H

<150>  JP 2013-064226
<151>  2013-03-26

<160>  157

<170>  PatentIn version 3.5

<210>  1
<211>  50
<212>  DNA
<213>  Artificial Sequence

<220>
<223>  rs8099917 FIP

<400>  1
tacctccagg aggtgaaata caagagttcc ttgtaaaaga ttccatccat                50


<210>  2
<211>  48
<212>  DNA
<213>  Artificial Sequence

<220>
<223>  rs8099917 BIP

<400>  2
tgtcactgtt cctccttttg ttttcccaaa ctgtatacag catggttc                  48


<210>  3
<211>  24
<212>  DNA
<213>  Artificial Sequence

<220>
<223>  rs8099917 F3 Primer

<400>  3
ttgtgcatat gttttctgac tacc                                            24


<210>  4
<211>  23
<212>  DNA
<213>  Artificial Sequence

<220>
<223>  rs8099917 B3 Primer

<400>  4
atggtaagac ataaaaagcc agc                                             23

<210>  5
<211>  25
<212>  DNA
<213>  Artificial Sequence

<220>
<223>  rs8099917 Loop Primer F

<400>  5
aactctccat gttgtatgtt tttgt                                              25


<210>  6
<211>  18
<212>  DNA
<213>  Artificial Sequence

<220>
<223>  rs8099917 Quenching Probe

<400>  6
ctgtgagcaa tgtcaccc                                                      18


<210>  7
<211>  47
<212>  DNA
<213>  Artificial Sequence

<220>
<223>  rs8099917 FIP

<400>  7
cctccaggag gtgaaataca agaccttgtt ccttgtaaaa gattcca                      47


<210>  8
<211>  45
<212>  DNA
<213>  Artificial Sequence

<220>
<223>  rs8099917 BIP

<400>  8
tcactgttcc tccttttgtt ttcctgacat aaaaagccag ctacc                        45


<210>  9
<211>  23
<212>  DNA
<213>  Artificial Sequence

<220>
<223>  rs8099917 F3 Primer

<400>  9
   atatgttttc tgactaccaa agt                                                 23


<210>  10
<211>  20
<212>  DNA

<213>  Artificial Sequence

<220>
<223>  rs8099917 B3 Primer

<400>  10
gggagaatgc aaatgagaga                                                    20


<210>  11
<211>  25
<212>  DNA
<213>  Artificial Sequence

<220>
<223>  rs8099917 Loop Primer F

<400>  11
tccatgttgt atgtttttgt atgga                                              25


<210>  12
<211>  25
<212>  DNA
<213>  Artificial Sequence

<220>
<223>  rs8099917 Loop Primer FB

<400>  12
aaattggaac catgctgtat acagt                                              25


<210>  13
<211>  20
<212>  DNA
<213>  Artificial Sequence

<220>
<223>  rs8099917 Quencher Probe, Dabcyl 2

<400>  13
ttctgtgagc aatgtcaccc                                                    20


<210>  14
<211>  41
<212>  DNA
<213>  Artificial Sequence

<220>
<223>  rs12979860 FIP

<400>  14
ggaatcccag actgtgcaga ggcggcgctt atcgcatacg g                            41


<210>  15
<211>  36
<212>  DNA
<213>  Artificial Sequence

<220>

<223>  rs12979860 BIP

<400>  15
cacggtcgtg cctgtcgtgg agcgcggagt gcaatt                                36


<210>  16
<211>  17
<212>  DNA
<213>  Artificial Sequence

<220>
<223>  rs12979860 F3 Primer

<400>  16
tcctcctgcg ggacaag                                                     17


<210>  17
<211>  16
<212>  DNA
<213>  Artificial Sequence

<220>
<223>  rs12979860 B3 Primer

<400>  17
actcggctcc aggtcg                                                      16


<210>  18
<211>  18
<212>  DNA
<213>  Artificial Sequence

<220>
<223>  rs12979860 Loop Primer F

<400>  18
ttaggggccc tggcgagg                                                    18


<210>  19
<211>  18
<212>  DNA
<213>  Artificial Sequence

<220>
<223>  rs12979860 Quenching Probe

<400>  19
tccccgaagg cgcgaacc                                                    18


<210>  20
<211>  40
<212>  DNA
<213>  Artificial Sequence

<220>
<223>  rs12979860 FIP

<400>  20

ccagtaccca tccacgtcca ggggcgctta tcgcatacgg                                40

<210>  21
<211>  35
<212>  DNA
<213>  Artificial Sequence

<220>
<223>  rs12979860 BIP

<400>  21
gcacggtcgt gcctgtcgtg cgagggcctt tgctg                                     35

<210>  22
<211>  17
<212>  DNA
<213>  Artificial Sequence

<220>
<223>  rs12979860 F3 Primer

<400>  22
tcctcctgcg ggacaag                                                         17

<210>  23
<211>  16
<212>  DNA
<213>  Artificial Sequence

<220>
<223>  rs12979860 B3 Primer

<400>  23
actcggctcc aggtcg                                                          16

<210>  24
<211>  18
<212>  DNA
<213>  Artificial Sequence

<220>
<223>  rs12979860 Loop Primer F

<400>  24
tgtgcagagg ttaggggc                                                        18

<210>  25
<211>  18
<212>  DNA
<213>  Artificial Sequence

<220>
<223>  rs12979860 Loop Primer FB

<400>  25
aattgcactc cgcgctcc                                                        18

<210>  26
<211>  19
<212>  DNA
<213>  Artificial Sequence

<220>
<223>  rs12979860 Quencher Probe

<400>  26
gaaggcgcga accagggtt                                                    19


<210>  27
<211>  44
<212>  DNA
<213>  Artificial Sequence

<220>
<223>  rs11881222 FIP

<400>  27
ttcctctcta tcctcctccc ccatgaggag aggtgagaaa gagc                        44


<210>  28
<211>  37
<212>  DNA
<213>  Artificial Sequence

<220>
<223>  rs11881222 BIP

<400>  28
tgacaggcac aggggagagg tccccacacc tgctacc                                37


<210>  29
<211>  16
<212>  DNA
<213>  Artificial Sequence

<220>
<223>  rs11881222 F3 Primer

<400>  29
gcctccaaag ccacgg                                                       16


<210>  30
<211>  20
<212>  DNA
<213>  Artificial Sequence

<220>
<223>  rs11881222 B3 Primer

<400>  30
ccccttcacc ttctccttct                                                   20


<210>  31
<211>  18
<212>  DNA

<213>  Artificial Sequence

<220>
<223>  rs11881222 Loop Primer F

<400>  31
acctgttccc ctcacctc                                                    18


<210>  32
<211>  20
<212>  DNA
<213>  Artificial Sequence

<220>
<223>  rs11881222 Quenching Probe

<400>  32
ctctgctccc acctgaccac                                                  20


<210>  33
<211>  40
<212>  DNA
<213>  Artificial Sequence

<220>
<223>  rs11881222 FIP

<400>  33
gagggacaat ggagaaggag aaggctccca gccctgctca                            40


<210>  34
<211>  44
<212>  DNA
<213>  Artificial Sequence

<220>
<223>  rs11881222 BIP

<400>  34
ccccacacct gctacccctt cgaggaggat agagaggaac aagt                       44


<210>  35
<211>  15
<212>  DNA
<213>  Artificial Sequence

<220>
<223>  rs11881222 F3 Primer

<400>  35
ggagtcaggc ccacc                                                       15


<210>  36
<211>  17
<212>  DNA
<213>  Artificial Sequence

<220>

<223>  rs11881222 B3 Primer

<400>  36
gaggtgaggg gaacagg                                                    17


<210>  37
<211>  19
<212>  DNA
<213>  Artificial Sequence

<220>
<223>  rs11881222 Loop Primer F

<400>  37
gggccactac agagccagg                                                  19


<210>  38
<211>  19
<212>  DNA
<213>  Artificial Sequence

<220>
<223>  rs11881222 Loop Primer FB

<400>  38
gctgtgccct ctcccctgt                                                  19


<210>  39
<211>  20
<212>  DNA
<213>  Artificial Sequence

<220>
<223>  rs11881222 Quencher Probe

<400>  39
tgctcccacc tgaccacact                                                 20


<210>  40
<211>  37
<212>  DNA
<213>  Artificial Sequence

<220>
<223>  rs8103142 FIP

<400>  40
ttagccccag caggagggct cccctatcct gttgtca                              37


<210>  41
<211>  39
<212>  DNA
<213>  Artificial Sequence

<220>
<223>  rs8103142 BIP

<400>  41

cctttgctgt ctaggaagag tcgtcaggtc ccaggtcct                              39

<210>  42
<211>  17
<212>  DNA
<213>  Artificial Sequence

<220>
<223>  rs8103142 F3 Primer

<400>  42
ccatcctccc agcagtt                                                     17

<210>  43
<211>  16
<212>  DNA
<213>  Artificial Sequence

<220>
<223>  rs8103142 B3 Primer

<400>  43
gactccccct ctcacc                                                      16

<210>  44
<211>  20
<212>  DNA
<213>  Artificial Sequence

<220>
<223>  rs8103142 Loop Primer F

<400>  44
tgatgggatt ggaggatggc                                                  20

<210>  45
<211>  20
<212>  DNA
<213>  Artificial Sequence

<220>
<223>  rs8103142 Quenching Probe

<400>  45
agcggcacct gcagtccttc                                                  20

<210>  46
<211>  40
<212>  DNA
<213>  Artificial Sequence

<220>
<223>  rs8103142 FIP

<400>  46
agtggcccct tcaccttctc cttagaggga aggggtagca                            40

<210>  47
<211>  37
<212>  DNA
<213>  Artificial Sequence

<220>
<223>  rs8103142 BIP

<400>  47
ggtgggcctg actcccccgg ctaacctgtg cctttgc                                37


<210>  48
<211>  16
<212>  DNA
<213>  Artificial Sequence

<220>
<223>  rs8103142 F3 Primer

<400>  48
cagccagtgt ggtcag                                                       16


<210>  49
<211>  21
<212>  DNA
<213>  Artificial Sequence

<220>
<223>  rs8103142 B3 Primer

<400>  49
tcccatcaga gtggtctaac c                                                 21


<210>  50
<211>  21
<212>  DNA
<213>  Artificial Sequence

<220>
<223>  rs8103142 Loop Primer F

<400>  50
ccattgtccc tctctcctct c                                                 21


<210>  51
<211>  21
<212>  DNA
<213>  Artificial Sequence

<220>
<223>  rs8103142 Loop Primer FB

<400>  51
agcagaagcg actcttccta g                                                 21


<210>  52
<211>  20
<212>  DNA

<213>  Artificial Sequence

<220>
<223>  rs8103142 Quencher Probe

<400>  52
agcggcacct gcagtccttc                                                    20


<210>  53
<211>  44
<212>  DNA
<213>  Artificial Sequence

<220>
<223>  rs1127354 FIP

<400>  53
ggttggattt ctctgtcttc ctgtgtccgg cacttatcag ggaa                         44


<210>  54
<211>  44
<212>  DNA
<213>  Artificial Sequence

<220>
<223>  rs1127354 BIP

<400>  54
tacggtcaat tttctgtgcc accactcttg gaacaggtcg ttca                         44


<210>  55
<211>  19
<212>  DNA
<213>  Artificial Sequence

<220>
<223>  rs1127354 F3 Primer

<400>  55
tggaaatctc atccggctc                                                     19


<210>  56
<211>  23
<212>  DNA
<213>  Artificial Sequence

<220>
<223>  rs1127354 B3 Primer

<400>  56
tcacatggag aatcactaga tgg                                                23


<210>  57
<211>  22
<212>  DNA
<213>  Artificial Sequence

<220>

<223>  rs1127354 Loop Primer F

<400>  57
acctgacttt ctgtgtgtct gt                                                22


<210>  58
<211>  20
<212>  DNA
<213>  Artificial Sequence

<220>
<223>  rs1127354 Quenching Probe

<400>  58
ggagataagt ttccatgcac                                                   20


<210>  59
<211>  38
<212>  DNA
<213>  Artificial Sequence

<220>
<223>  rs1127354 FIP

<400>  59
tctccatgtg agcacgtgag cgcctgagtt ggtaagct                               38


<210>  60
<211>  45
<212>  DNA
<213>  Artificial Sequence

<220>
<223>  rs1127354 BIP

<400>  60
ctcttggaac aggtcgttca gattcaacaa aacagagaca tacgg                       45


<210>  61
<211>  17
<212>  DNA
<213>  Artificial Sequence

<220>
<223>  rs1127354 F3 Primer

<400>  61
   ctgaaagccg gggtgag                                                       17


<210>  62
<211>  20
<212>  DNA
<213>  Artificial Sequence

<220>
<223>  rs1127354 B3 Primer

<400>  62

acagagaaat ccaaccatct                                                    20

<210>  63
<211>  16
<212>  DNA
<213>  Artificial Sequence

<220>
<223>  rs1127354 Loop Primer F

<400>  63
tctgctgccc atctcc                                                        16

<210>  64
<211>  20
<212>  DNA
<213>  Artificial Sequence

<220>
<223>  rs1127354 Loop Primer FB

<400>  64
tttggtggca cagaaaattg                                                    20

<210>  65
<211>  20
<212>  DNA
<213>  Artificial Sequence

<220>
<223>  rs1127354 Quencher Probe

<400>  65
ggagataagt ttccatgcac                                                    20

<210>  66
<211>  45
<212>  DNA
<213>  Artificial Sequence

<220>
<223>  rs7270101 FIP

<400>  66
agtgcatgga aacttatctc ctagaggaga atcactagat ggtga                        45

<210>  67
<211>  43
<212>  DNA
<213>  Artificial Sequence

<220>
<223>  rs7270101 BIP

<400>  67
gtggcacaga aaattgaccg tatgtcaggt cacaggaaga cag                          43

<210>  68
<211>  18
<212>  DNA
<213>  Artificial Sequence

<220>
<223>  rs7270101 F3 Primer

<400>  68
gatgagaaag gcggatga                                                    18


<210>  69
<211>  18
<212>  DNA
<213>  Artificial Sequence

<220>
<223>  rs7270101 B3 Primer

<400>  69
gggaaacaga cacacaga                                                    18


<210>  70
<211>  25
<212>  DNA
<213>  Artificial Sequence

<220>
<223>  rs7270101 Loop Primer F

<400>  70
acgacctgtt ccaagagaaa agaga                                            25


<210>  71
<211>  21
<212>  DNA
<213>  Artificial Sequence

<220>
<223>  rs7270101 Quenching Probe

<400>  71
cttttaaaaa gaaaacaaaa c                                                21


<210>  72
<211>  42
<212>  DNA
<213>  Artificial Sequence

<220>
<223>  rs7270101 FIP

<400>  72
tccctgataa gtgccggagt acccgaactg cctcctgaca tt                         42


<210>  73
<211>  46
<212>  DNA

<213>  Artificial Sequence

<220>
<223>  rs7270101 BIP

<400>  73
agtcaggtca caggaagaca gagataagtt tccatgcact ttggtg                    46


<210>  74
<211>  19
<212>  DNA
<213>  Artificial Sequence

<220>
<223>  rs7270101 F3 Primer

<400>  74
gagcaagtgt gggacaagg                                                  19


<210>  75
<211>  22
<212>  DNA
<213>  Artificial Sequence

<220>
<223>  rs7270101 B3 Primer

<400>  75
aacaggtcgt tcagattcta gg                                              22


<210>  76
<211>  23
<212>  DNA
<213>  Artificial Sequence

<220>
<223>  rs7270101 Loop Primer F

<400>  76
gccggatgag atttccatac aga                                             23


<210>  77
<211>  20
<212>  DNA
<213>  Artificial Sequence

<220>
<223>  rs7270101 Loop Primer FB

<400>  77
atacggtcaa ttttctgtgc                                                 20


<210>  78
<211>  22
<212>  DNA
<213>  Artificial Sequence

<220>

<223>  rs7270101 Quencher Probe

<400>  78
aaaaagaaaa caaaacagag ac                                                22


<210>  79
<211>  38
<212>  DNA
<213>  Artificial Sequence

<220>
<223>  rs1229984 FIP

<400>  79
aggggtgact acagtcaaac cggatggtga accacacg                               38


<210>  80
<211>  41
<212>  DNA
<213>  Artificial Sequence

<220>
<223>  rs1229984 BIP

<400>  80
atcacaggaa ggggggtcaa tcttttctga atctgaacag c                            41


<210>  81
<211>  19
<212>  DNA
<213>  Artificial Sequence

<220>
<223>  rs1229984 F3 Primer

<400>  81
tggtttcctt atccaggct                                                    19


<210>  82
<211>  23
<212>  DNA
<213>  Artificial Sequence

<220>
<223>  rs1229984 B3 Primer

<400>  82
cacaatttca ggaatttggg tat                                               23


<210>  83
<211>  22
<212>  DNA
<213>  Artificial Sequence

<220>
<223>  rs1229984 LooP Primer F

<400>  83

acaggattca cactcaggga ac                                                22

<210>  84
<211>  25
<212>  DNA
<213>  Artificial Sequence

<220>
<223>  rs1229984 LooP Primer FB

<400>  84
agccaccatc tacagaataa agaga                                             25

<210>  85
<211>  23
<212>  DNA
<213>  Artificial Sequence

<220>
<223>  rs1229984 Quencher Probe

<400>  85
tcatctgtgc gacagattcc tac                                               23

<210>  86
<211>  39
<212>  DNA
<213>  Artificial Sequence

<220>
<223>  rs671 FIP

<400>  86
tcggagcctg ctgggggatt gcaggtcctg aacctctgg                               39

<210>  87
<211>  40
<212>  DNA
<213>  Artificial Sequence

<220>
<223>  rs671 BIP

<400>  87
caagccccaa caggccctga tacaagatgt cggggagtgg                             40

<210>  88
<211>  18
<212>  DNA
<213>  Artificial Sequence

<220>
<223>  rs671 F3 Primer

<400>  88
gcaggtcctg aacttcca                                                     18

<210>  89
<211>  22
<212>  DNA
<213>  Artificial Sequence

<220>
<223>  rs671 B3 Primer

<400>  89
aactgctatg atgtgtttgg ag                                                22


<210>  90
<211>  16
<212>  DNA
<213>  Artificial Sequence

<220>
<223>  rs671 LooP Primer F

<400>  90
tctgttgggg gctcgg                                                       16


<210>  91
<211>  16
<212>  DNA
<213>  Artificial Sequence

<220>
<223>  rs671 LooP Primer FB

<400>  91
tgcagcccgt actcgc                                                       16


<210>  92
<211>  22
<212>  DNA
<213>  Artificial Sequence

<220>
<223>  rs671 Quencher Probe

<400>  92
agttttcact tcagtgtatg cc                                                22


<210>  93
<211>  44
<212>  DNA
<213>  Artificial Sequence

<220>
<223>  rs1800592 FIP

<400>  93
acagtgcttt gcatttgaat agtgtaacag ggtatttccc agtg                        44


<210>  94
<211>  49
<212>  DNA

<213>  Artificial Sequence

<220>
<223>  rs1800592 BIP

<400>  94
gaagacattt tgtgcagcga tttctctgaa tgtaacaaat tctcctttc                    49


<210>  95
<211>  22
<212>  DNA
<213>  Artificial Sequence

<220>
<223>  rs1800592 F3 Primer

<400>  95
tgtcatttgc acatttatct gt                                                 22


<210>  96
<211>  19
<212>  DNA
<213>  Artificial Sequence

<220>
<223>  rs1800592 B3 Primer

<400>  96
tgacccttta tgacgtagc                                                     19


<210>  97
<211>  22
<212>  DNA
<213>  Artificial Sequence

<220>
<223>  rs1800592 LooP Primer F

<400>  97
ccataattct ctcattagcc ac                                                 22


<210>  98
<211>  24
<212>  DNA
<213>  Artificial Sequence

<220>
<223>  rs1800592 LooP Primer FB

<400>  98
aatgtgttct acattttcaa aaag                                               24


<210>  99
<211>  23
<212>  DNA
<213>  Artificial Sequence

<220>

<223>  rs1800592 Quencher Probe

<400>  99
cagtttgatc aagtgcattt gtt                                              23


<210>  100
<211>  36
<212>  DNA
<213>  Artificial Sequence

<220>
<223>  rs1042713 FIP

<400>  100
gtaagcgcac tggctgggga tgaggcttcc aggcgt                                36


<210>  101
<211>  34
<212>  DNA
<213>  Artificial Sequence

<220>
<223>  rs1042713 BIP

<400>  101
atggggcaac ccgggaaccc caccacccac acct                                  34


<210>  102
<211>  16
<212>  DNA
<213>  Artificial Sequence

<220>
<223>  rs1042713 F3 Primer

<400>  102
ccacacccac accaca                                                      16


<210>  103
<211>  20
<212>  DNA
<213>  Artificial Sequence

<220>
<223>  rs1042713 B3 Primer

<400>  103
gatgagagac atgacgatgc                                                  20


<210>  104
<211>  16
<212>  DNA
<213>  Artificial Sequence

<220>
<223>  rs1042713 LooP Primer F

<400>  104

tcagcgggcg gaccca                                                    16


<210>  105
<211>  15
<212>  DNA
<213>  Artificial Sequence

<220>
<223>  rs1042713 LooP Primer FB

<400>  105
gccggaccac gacgt                                                     15


<210>  106
<211>  19
<212>  DNA
<213>  Artificial Sequence

<220>
<223>  rs1042713 Quencher Probe

<400>  106
cacccaatag aagccatgc                                                 19


<210>  107
<211>  36
<212>  DNA
<213>  Artificial Sequence

<220>
<223>  rs4994 FIP

<400>  107
tattgggcgc cagggtggga tggctccgtg gcctca                              36


<210>  108
<211>  40
<212>  DNA
<213>  Artificial Sequence

<220>
<223>  rs4994 BIP

<400>  108
gaggcaacct gctggtcatc gtccatcacc aggtcggctg                          40


<210>  109
<211>  20
<212>  DNA
<213>  Artificial Sequence

<220>
<223>  rs4994 F3 Primer

<400>  109
cttccttctt tccctaccgc                                                20

<210>  110
<211>  16
<212>  DNA
<213>  Artificial Sequence

<220>
<223>  rs4994 B3 Primer

<400>  110
cggcaccacc aggagt                                                        16


<210>  111
<211>  20
<212>  DNA
<213>  Artificial Sequence

<220>
<223>  rs4994 LooP Primer F

<400>  111
gggcaagaga gctgttctcg                                                    20


<210>  112
<211>  20
<212>  DNA
<213>  Artificial Sequence

<220>
<223>  rs4994 LooP Primer FB

<400>  112
ctccagacca tgaccaacgt                                                    20


<210>  113
<211>  22
<212>  DNA
<213>  Artificial Sequence

<220>
<223>  rs4994 Quencher Probe

<400>  113
ggccatcgcc cggactccga ga                                                 22


<210>  114
<211>  47
<212>  DNA
<213>  Artificial Sequence

<220>
<223>  rs1057910 FIP

<400>  114
ctcctgtctt gcatgcaggg gcgctaaagt ccaggaagag attgaac                      47


<210>  115
<211>  47
<212>  DNA

<213>  Artificial Sequence

<220>
<223>  rs1057910 BIP

<400>  115
ccacatgccc tacacagatg ctgtctgaat ttaatgtcac aggtcac                47


<210>  116
<211>  24
<212>  DNA
<213>  Artificial Sequence

<220>
<223>  rs1057910 F3 Primer

<400>  116
tccatcagtt tttacttgtg tctt                                         24


<210>  117
<211>  23
<212>  DNA
<213>  Artificial Sequence

<220>
<223>  rs1057910 B3 Primer

<400>  117
caaacttacc ttgggaatga gat                                          23


<210>  118
<211>  18
<212>  DNA
<213>  Artificial Sequence

<220>
<223>  rs1057910 LooP Primer F

<400>  118
ggtttctgcc aatcacac                                                18


<210>  119
<211>  15
<212>  DNA
<213>  Artificial Sequence

<220>
<223>  rs1057910 LooP Primer FB

<400>  119
cccaccagcc tgccc                                                   15


<210>  120
<211>  20
<212>  DNA
<213>  Artificial Sequence

<220>

<223>  rs1057910 Quencher Probe

<400>  120
cagagatacc ttgaccttct                                                    20


<210>  121
<211>  43
<212>  DNA
<213>  Artificial Sequence

<220>
<223>  rs9934438 FIP

<400>  121
ttggaaatca cctttctcgg gcatggaacc aggttaggac tgt                          43


<210>  122
<211>  44
<212>  DNA
<213>  Artificial Sequence

<220>
<223>  rs9934438 BIP

<400>  122
aagccacctg ggctatcctc tgttgtgaca tggaatcctg acgt                         44


<210>  123
<211>  16
<212>  DNA
<213>  Artificial Sequence

<220>
<223>  rs9934438 F3 Primer

<400>  123
cacctggcag aggggt                                                        16


<210>  124
<211>  19
<212>  DNA
<213>  Artificial Sequence

<220>
<223>  rs9934438 F3 Primer

<400>  124
agaggggagg atagggtca                                                     19


<210>  125
<211>  19
<212>  DNA
<213>  Artificial Sequence

<220>
<223>  rs9934438 LooP Primer F

<400>  125

ggtccaaggc actgggttg                                                    19

<210>  126
<211>  19
<212>  DNA
<213>  Artificial Sequence

<220>
<223>  rs9934438 LooP Primer FB

<400>  126
ctggcaccgg gcacctttg                                                    19

<210>  127
<211>  20
<212>  DNA
<213>  Artificial Sequence

<220>
<223>  rs9934438 Quencher Probe

<400>  127
gtccaagagt cgatgatctc                                                   20

<210>  128
<211>  38
<212>  DNA
<213>  Artificial Sequence

<220>
<223>  rs9923231 FIP

<400>  128
caagcctggc caacatggtg agcctgccac catgtctg                               38

<210>  129
<211>  46
<212>  DNA
<213>  Artificial Sequence

<220>
<223>  rs9923231 BIP

<400>  129
cctcccaaaa tgctaggatt ataggcgggg atccctctgg gaagtc                      46

<210>  130
<211>  21
<212>  DNA
<213>  Artificial Sequence

<220>
<223>  rs9923231 F3 Primer

<400>  130
cccaagtagt ttggactaca g                                                 21

<210>  131
<211>  19
<212>  DNA
<213>  Artificial Sequence

<220>
<223>  rs9923231 B3 Primer

<400>  131
aagggtaggt gcaacagta                                                    19


<210>  132
<211>  25
<212>  DNA
<213>  Artificial Sequence

<220>
<223>  rs9923231 Loop Primer F

<400>  132
accctgtctc tactaaaaat acaaa                                             25


<210>  133
<211>  25
<212>  DNA
<213>  Artificial Sequence

<220>
<223>  rs9923231 Loop Primer FB

<400>  133
ggttgttttt caggtcttct cttgc                                             25


<210>  134
<211>  15
<212>  DNA
<213>  Artificial Sequence

<220>
<223>  rs9923231 Quencher Probe

<400>  134
ccgcacccgg ccaat                                                        15


<210>  135
<211>  17
<212>  DNA
<213>  Artificial Sequence

<220>
<223>  Dab3'-3

<400>  135
ttctgtgagc aatgtca                                                      17


<210>  136
<211>  18
<212>  DNA

<213>  Artificial Sequence

<220>
<223>  Dab3'-2

<400>  136
ttctgtgagc aatgtcac                                                    18


<210>  137
<211>  19
<212>  DNA
<213>  Artificial Sequence

<220>
<223>  Dab3'-1

<400>  137
ttctgtgagc aatgtcacc                                                   19


<210>  138
<211>  14
<212>  DNA
<213>  Artificial Sequence

<220>
<223>  Dab-6

<400>  138
gagcaatgtc accc                                                        14


<210>  139
<211>  16
<212>  DNA
<213>  Artificial Sequence

<220>
<223>  Dab-4

<400>  139
gtgagcaatg tcaccc                                                      16


<210>  140
<211>  18
<212>  DNA
<213>  Artificial Sequence

<220>
<223>  Dabcyl1

<400>  140
ctgtgagcaa tgtcaccc                                                    18


<210>  141
<211>  33
<212>  DNA
<213>  Artificial Sequence

<220>

<223>  Dab+half

<400>  141
cttttgtttt cctttctgtg agcaatgtca ccc                                    33


<210>  142
<211>  45
<212>  DNA
<213>  Artificial Sequence

<220>
<223>  Dab+full

<400>  142
tcactgttcc tccttttgtt ttcctttctg tgagcaatgt caccc                       45


<210>  143
<211>  20
<212>  DNA
<213>  Artificial Sequence

<220>
<223>  misDab

<400>  143
ttctgttagc aatgtcaccc                                                   20


<210>  144
<211>  803
<212>  DNA
<213>  Homo sapiens

<220>
<223>  DNA Fragment contaning rs8099917

<400>  144
tctgttcctg tctctgtctc tggcgtgact ccatctttct tattcatttt tccaacaagc      60

atcctgcccc aggtcgctct gtctgtctca atcaatctct ttttgtttaa acataaaatc     120

tacttctttt tctgataaaa ttagccatgc atgttcagtg gataaagctt gaaaaataca     180

caaaaacata aaagaaaaaa aacatcacct ataacttcac catcctcctc tcatccctca     240

tcccacttct ggaacaaatc gtcccaatac ataggaattt tccatgtgtt tatttgtgca     300

tatgttttct gactaccaaa gtaacacttg ttccttgtaa aagattccat ccatacaaaa     360

acatacaaca tggagagtta aagtaagtct tgtatttcac ctcctggagg taaatatttt     420

ttaacaattt gtcactgttc ctccttttgt tttcctttct gtgagcaatt tcacccaaat     480

tggaaccatg ctgtatacag tttggtagct ggctttttat gtcttaccat tatctctcat     540

ttgcattctc ccacatcttt aattatagcg tatcagttag ggcccagcag gaaacagatg     600

gcccgtcaat ttaggataat ttgaggtggg gttgatacca gaagccttta taaagcgatt     660

ggatagtaaa ggcaaatgac aagagctagt gcaagctcct ggggccagca tgggtgaggg     720

gcctcatcca caggcctaaa ggaaggggag agggttgagg gtgtgcatgt ggttgcctga 780

cttggggagg agagagtcac tta 803

<210> 145
<211> 583
<212> DNA
<213> Homo sapiens

<220>
<223> DNA Fragment contaning rs12979860

<400> 145
ggacgagagg gcgttagagc gggccgcgcc cgggccatgc ctctcccgcc cactcccggg 60

cctcaccgat ggccgcggag gatccctcct ggggcggaag gagcagttgc gctgccccca 120

gctcagcgcc tcttcctcct gcgggacaag cggcgcttat cgcatacggc taggccccct 180

cgccagggcc cctaacctct gcacagtctg ggattcctgg acgtggatgg gtactggcag 240

cgcacggtcg tgcctgtcgt gtactgaacc agggagctcc ccgaaggcgt gaaccagggt 300

tgaattgcac tccgcgctcc cccagcaaag cccctcgccc cgacctggag ccgagtcctc 360

ccggcagggc tcccttctgt gattgaccct gagcctgcgt tcgcgctgac gacggggact 420

gcgggggtct cgtggtggga attgtgggcg ctgacatagg agaggcgcct gctgggcgct 480

aggacgcagg accccttggg acaggaacgg gtgtatggga acccggtggg gccagggtcc 540

cagggggcac aggggctggg cggtgactta cgtagcggtc cct 583

<210> 146
<211> 1265
<212> DNA
<213> Homo sapiens

<220>
<223> DNA Fragment contaning rs11881222

<400> 146
gtgctcagct cctgcagccc ctgccctcag tgggcagcct ctgcattccc tcagctccct 60

ttctctctgt gacacagaca tgaccgggga ctgcatgcca gtgctggtgc tgatggccgc 120

agtgctgacc gtgactggag cagttcctgt cgccaggctc cgcggggctc tcccggatgc 180

aaggggctgc cacatagccc agttcaagtc cctgtctcca caggagctgc aggcctttaa 240

gagggccaaa gatgccttag tgagtctccc cctgccctcc tgccatggac tagcctccac 300

ccgcactcca agggtcacca tgctttccca ctcccagctt ccttcactgg gctagcctcc 360

accctccctg cagtgggcta tctcatgctc ctactgcagg gactgactca tgttttcctg 420

aagaagaggg tcctctacca tcctcccagc agttaacctc ccctatcctg ttgtcagcca 480

tcctccaatc ccatcagagt ggtctaacct ccacccctcc tgctggggct aacctgtgcc 540

tttgctgtct aggaagagtc gcttctgctg aaggactgca agtgccgctc ccgcctcttc 600

cccaggacct gggacctgag gcagctgcag gtgagagggg gagtcaggcc cacccctgcc 660

ctcccagccc tgctcacctg gctctgtagt ggccccttca ccttctcctt ctccattgtc 720

cctctctcct ctccccacac ctgctacccc ttccctctgc tcctacctga ccacactggc 780

tgtgccctct cccctgtgcc tgtcaccttc acttgttcct ctctatcctc ctcccccaac 840

ctgttcccct cacctccccc ctcacctgct ctttctcacc tctcctcagg tgagggagcg 900

ccccgtggct ttggaggctg agctggccct gacgctgaag gttctggagg ccaccgctga 960

cactgaccca gccctggggg atgtcttgga ccagcccctt cacaccctgc accatatcct 1020

ctcccagctc cgggcctgtg tgagtcgtca gggcccgggc acccaggtct gtgagctctg 1080

agcagcgtcc ttcccctggc caaggccccg gctcacacac cgccctcctc tgcccacaga 1140

tccagcctca gcccacggca gggcccagga cccggggccg cctccaccat tggctgcacc 1200

ggctccagga ggccccaaaa aaggtgagtg acccgggaag agagggactg aggtctgggg 1260

agcca 1265

<210> 147
<211> 1265
<212> DNA
<213> Homo sapiens

<220>
<223> DNA Fragment contaning rs8103142

<400> 147
tggctcccca gacctcagtc cctctcttcc cgggtcactc accttttttg gggcctcctg 60

gagccggtgc agccaatggt ggaggcggcc ccgggtcctg ggccctgccg tgggctgagg 120

ctggatctgt gggcagagga gggcggtgtg tgagccgggg ccttggccag gggaaggacg 180

ctgctcagag ctcacagacc tgggtgcccg ggccctgacg actcacacag gcccggagct 240

gggagaggat atggtgcagg gtgtgaaggg gctggtccaa gacatccccc agggctgggt 300

cagtgtcagc ggtggcctcc agaaccttca gcgtcagggc cagctcagcc tccaaagcca 360

cggggcgctc cctcacctga ggagaggtga gaaagagcag gtgagggggg aggtgagggg 420

aacaggttgg gggaggagga tagagaggaa caagtgaagg tgacaggcac aggggagagg 480

gcacagccag tgtggtcagg taggagcaga gggaaggggt agcaggtgtg gggagaggag 540

agagggacaa tggagaagga gaaggtgaag gggccactac agagccaggt gagcagggct 600

gggagggcag gggtgggcct gactccccct ctcacctgca gctgcctcag gtcccaggtc 660

ctggggaaga ggcgggagcg gcacttgcag tccttcagca gaagcgactc ttcctagaca 720

gcaaaggcac aggttagccc cagcaggagg ggtggaggtt agaccactct gatgggattg 780

gaggatggct gacaacagga taggggaggt taactgctgg gaggatggta gaggaccctc 840

ttcttcagga aaacatgagt cagtccctgc agtaggagca tgagatagcc cactgcaggg 900

agggtggagg ctagcccagt gaaggaagct gggagtggga aagcatggtg acccttggag 960

tgcgggtgga ggctagtcca tggcaggagg gcagggggag actcactaag gcatctttgg 1020

ccctcttaaa ggcctgcagc tcctgtggag acagggactt gaactgggct atgtggcagc 1080

cccttgcatc cgggagagcc ccgcggagcc tggcgacagg aactgctcca gtcacggtca 1140

gcactgcggc catcagcacc agcactggca tgcagtcccc ggtcatgtct gtgtcacaga 1200

gagaaaggga gctgagggaa tgcagaggct gcccactgag ggcaggggct gcaggagctg 1260

agcac 1265

<210> 148
<211> 1001
<212> DNA
<213> Homo sapiens

<220>
<223> DNA Fragment contaning rs1127354

<400> 148
tgtgatttta gtagggttaa aagtcacagg cactgctagt aatattttgg tttgttacct 60

atattcataa ttgaaaaagt tactacattt caattaaggg ttactaagaa taaagttttt 120

tttcccaatc catgttcata ggccttctga attctgggta tcttctgtta gagaattcct 180

ggtctaggag gatggctagg gtttctggga gcattgagaa gagaagagtg tgttctggat 240

gggggcttag cacaagcaga gacctgacgt agaagagata gagaagcaag gagatggaag 300

gggctggctt gctggggtgg gaccctgaaa gccggggtga ggcccacagg cctgagttgg 360

taagctttag gagatgggca gcagagttat cgatgagaaa ggcggatgac agctcacgtg 420

ctcacatgga gaatcactag atggtgataa gtgttctctt ttctcttgga acaggtcgtt 480

cagattctag gagataagtt tccatgcact ttggtggcac agaaaattga ccgtatgtct 540

ctgttttgtt ttatttttaa aagatggttg gatttctctg tcttcctgtg acctgacttt 600

ctgtgtgtct gtttccctga taagtgccgg agtaccaggg ggagccggat gagatttcca 660

tacagaaatg tcaggaggca gttcgccagg tgcttgccct gcccttgtcc cacacttgct 720

cttcttgtcc aggtagcttc cagggcctgc gcctgctaga aacaatagag tagacacagt 780

ttgttgagct ttctaggacc ggcccagagt cctctagggt tcagttaata ttggctttgg 840

aacactgcct tcctttgcca gccttgaata catggtatct cccccagacc accaccatac 900

actcccacgt acacacagac tccccttccc ccaaatgctc agacaatgga ttccagccct 960

acatcctccc cacacacatc caccgctgca ttcacacatg t 1001

<210> 149
<211> 1001

<212>  DNA
<213>  Homo sapiens

<220>
<223>  DNA Fragment containing rs7270101

<400>  149
acatgtgtga atgcagcggt ggatgtgtgt ggggaggatg tagggctgga atccattgtc      60

tgagcatttg ggggaagggg agtctgtgtg tacgtgggag tgtatggtgg tggtctgggg     120

gagataccat gtattcaagg ctggcaaagg aaggcagtgt tccaaagcca atattaactg     180

aaccctagag gactctgggc cggtcctaga aagctcaaca aactgtgtct actctattgt     240

ttctagcagg cgcaggccct ggaagctacc tggacaagaa gagcaagtgt gggacaaggg     300

cagggcaagc acctggcgaa ctgcctcctg acatttctgt atggaaatct catccggctc     360

cccctggtac tccggcactt atcagggaaa cagacacaca gaaagtcagg tcacaggaag     420

acagagaaat ccaaccatct tttaaaaata aaacaaaaca gagacatacg gtcaattttc     480

tgtgccacca aagtgcatgg aaacttatct cctagaatct gaacgacctg ttccaagaga     540

aaagagaaca cttatcacca tctagtgatt ctccatgtga gcacgtgagc tgtcatccgc     600

ctttctcatc gataactctg ctgcccatct cctaaagctt accaactcag gcctgtgggc     660

ctcaccccgg ctttcagggt cccaccccag caagccagcc ccttccatct ccttgcttct     720

ctatctcttc tacgtcaggt ctctgcttgt gctaagcccc catccagaac acactcttct     780

cttctcaatg ctcccagaaa ccctagccat cctcctagac caggaattct ctaacagaag     840

atacccagaa ttcagaaggc ctatgaacat ggattgggaa aaaaaacttt attcttagta     900

acccttaatt gaaatgtagt aactttttca attatgaata taggtaacaa accaaaatat     960

tactagcagt gcctgtgact ttttaaccta ctaaaatcac a                        1001


<210>  150
<211>  756
<212>  DNA
<213>  Homo sapiens

<220>
<223>  DNA ADH1B fragment containing rs1229984

<400>  150
ctatgaatgc cacactggta ggcactgtgt ctcttttgat cctcacatat ctccaggctc      60

taacctggtg cctggcttct agtagatact cggtacataa tggttgaagg gtacaataca     120

tgagtgcctg aatgcataca tgcttgggtc aggcaggcag agagggaaag aggaaactcc     180

tgaagtcctg gctgcgcggt gaccttgtgc aagcactttc gtctctcatt gccttggttt     240

ccttatccag gctgggaaat cctggatggt gaaccacacg tgttccctga gtgtgaatcc     300

tgtacctggt ttgactgtag tcaccccttc tccaacactc tccacgatgc cggctgcctc     360

atggcctaaa atcacaggaa ggggggtcac caggttgcca ctaaccacgt ggtcatctgt 420

gtgacagatt cctacagcca ccatctacag aataaagaga agctgttcag attcagaaaa 480

gattgtaact agatgaattt aacataccca aattcctgaa attgtgtttc taaaatgtat 540

ttgagggttt tgctactaat ccctactatt cctaaatatg aaagattcag tttatcccca 600

aggttattca gtctaaagcc cttctctacc ttcccttctt gaattaaaca atttagaata 660

atctgtgaaa taattgattc tgaaatattt aagtcttatg taagaatgca cgctttaaaa 720

aatagaagta gaaatagcaa agtaacacat ggaaaa 756

<210> 151
<211> 872
<212> DNA
<213> Homo sapiens

<220>
<223> DNA ALDH2 fragment contaning rs671

<400> 151
   cccctgaagt ctctccctct tctcaggcct acaggccttg gcgtataacg aagcccagca 60
   aatgaccgca taggcctgat gggggcacgt ccctcagtat ttctcatggg acagagcaga 120
   ggctgggtct ttaccctctc ttgtcacttc tcaggcttaa aatgggaaat tagtaggaaa 180
   cactgatggc ctcaagcatg agggagaggc caaaaggtgg cccgcccagc agaccctaaa 240
   tccctggcag gccctgatcc ccagcaggtc ctgaacttcc agcaggccct gagtccccgg 300
   caggtcctga acctctggca ggccccgagc ccccaacaga ccccaatccc ccagcaggct 360
   ccgagccacc agcagaccct caagccccaa caggccctga gcccccagca ggtcccacac 420
   tcacagtttt cacttcagtg tatgcctgca gcccgtactc gcccaactcc cggccactcc 480
   ccgacatctt gtagccacca aagggtgact gggctccaaa cacatcatag cagttgaccc 540
   tgtaatttga aacgagattg cagaaatcac tcttgggggt tatgggttac actcccagga 600
   ccccctgtat tgccccaaag tctttatttt atttttaact taaaaaaaaa tttttttttt 660
   tgagatagaa tctttctctg ttgcccaggc tggagttgcc atggcatgat cttggctcgt 720
   tgcaacctcc acatctgggg ttcaagagat tctcctgcct cagcttcctg agtagctgga 780
   ctaccagcac gtgcaaccac gcctggcaat aaattttttt ttaagagaca gggtctgatt 840
   atgttaccca ggctggtctc caactcctgg tc 872

<210> 152
<211> 914
<212> DNA
<213> Homo sapiens

<220>
<223> UCP1 DNA fragment contaning rs1800592

<400>  152
aaatagactt tctcttggct cagtgtatac tgccaaattg ttttccaaaa aaattgtgtc      60

aatgtataac accatcacta atatagtatt gatattatgg ttattacatt ttaaaattca     120

taatttgtaa ttataacatt cataatttat tactatttat aatattaatg taaatgtata     180

ttatatataa atgttatagt aattataact ttggtagtga caaagtatta atttattagg     240

tgaagtatat gcttttttat tagtgataat aaatatatcc tctctcccat tataaaagtt     300

tgtatttctt cttttagaaa ttgattcttc tgtcatttgc acatttatct gtataattat     360

aacagggtat ttcccagtgg tggctaatga gagaattatg ggaaagtata gaacactatt     420

caaatgcaaa gcactgtatg atttttattt aataggaaga cattttgtgc agcgatttct     480

gattgaccac agtttgatcg agtgcatttg ttaatgtgtt ctacattttc aaaaaggaaa     540

ggagaatttg ttacattcag aacttgctgc cactcctttg ctacgtcata aagggtcagt     600

tgcccttgct catactgacc tattctttac ctctctgctt cttctttgtg ccagaagagt     660

agaaatctga ccctttgggg ataccaccct ctcccctact gctctctcca acctgaggca     720

aactttctcc tacttcccag agcctgtcag aagtggtgaa gccagcctgc tccttggaat     780

ccagaactac tttcagaatc ttgaacttct gtgacctctc agggtcccct tgtgtgaagt     840

ttttgacgtc agcttctcct gtgaccctta gaagtcactc ttgtgtctag cacatcccag     900

gtgctcagtc acca                                                       914


<210>  153
<211>  2000
<212>  DNA
<213>  Homo sapiens

<220>
<223>  ADRB2 DNA fragment contaning rs1042713

<400>  153
tgtatgttgg gaggggagca ttatcagtgc tcgggtgagg caagttcgga gtacccagat      60

ggagacatcc gtgtctgtgt cgctctggat gcctccaagc cagcgtgtgt ttactttctg     120

tgtgtgtcac catgtctttg tgcttctggg tgcttctgtg tttgtttctg gccgcgtttc     180

tgtgttggac aggggtgact ttgtgccgga tggcttctgt gtgagagcgc gcgcgagtgt     240

gcatgtcggt gagctgggag ggtgtgtctc agtgtctatg gctgtggttc ggtataagtc     300

tgagcatgtc tgccagggtg tatttgtgcc tgtatgtgcg tgcctcggtg ggcactctcg     360

tttccttccg aatgtggggc agtgccggtg tgctgccctc tgccttgaga cctcaagccg     420

cgcaggcgcc cagggcaggc aggtagcggc cacagaagag ccaaaagctc ccgggttggc     480

tggtaaggac accacctcca gctttagccc tctggggcca gccagggtag ccgggaagca     540

gtggtggccc gccctccagg gagcagttgg gccccgcccg ggccagcccc aggagaagga     600

gggcgagggg aggggaggga aaggggagga gtgcctcgcc ccttcgcggc tgccggcgtg 660

ccattggccg aaagttcccg tacgtcacgg cgagggcagt tcccctaaag tcctgtgcac 720

ataacgggca gaacgcactg cgaagcggct tcttcagagc acgggctgga actggcaggc 780

accgcgagcc cctagcaccc gacaagctga gtgtgcagga cgagtcccca ccacacccac 840

accacagccg ctgaatgagg cttccaggcg tccgctcgcg gcccgcagag ccccgccgtg 900

ggtccgcccg ctgaggcgcc cccagccagt gcgcttacct gccagactgc gcgccatggg 960

gcaacccggg aacggcagcg ccttcttgct ggcacccaat ggaagccatg cgccggacca 1020

cgacgtcacg caggaaaggg acgaggtgtg ggtggtgggc atgggcatcg tcatgtctct 1080

catcgtcctg gccatcgtgt ttggcaatgt gctggtcatc acagccattg ccaagttcga 1140

gcgtctgcag acggtcacca actacttcat cacttcactg gcctgtgctg atctggtcat 1200

gggcctggca gtggtgccct ttggggccgc ccatattctt atgaaaatgt ggacttttgg 1260

caacttctgg tgcgagtttt ggacttccat tgatgtgctg tgcgtcacgg ccagcattga 1320

gaccctgtgc gtgatcgcag tggatcgcta ctttgccatt acttcacctt tcaagtacca 1380

gagcctgctg accaagaata aggcccgggt gatcattctg atggtgtgga ttgtgtcagg 1440

ccttacctcc ttcttgccca ttcagatgca ctggtaccgg gccacccacc aggaagccat 1500

caactgctat gccaatgaga cctgctgtga cttcttcacg aaccaagcct atgccattgc 1560

ctcttccatc gtgtccttct acgttcccct ggtgatcatg gtcttcgtct actccagggt 1620

ctttcaggag gccaaaaggc agctccagaa gattgacaaa tctgagggcc gcttccatgt 1680

ccagaacctt agccaggtgg agcaggatgg gcggacgggg catggactcc gcagatcttc 1740

caagttctgc ttgaaggagc acaaagccct caagacgtta ggcatcatca tgggcacttt 1800

caccctctgc tggctgccct tcttcatcgt taacattgtg catgtgatcc aggataacct 1860

catccgtaag gaagtttaca tcctcctaaa ttggataggc tatgtcaatt ctggtttcaa 1920

tccccttatc tactgccgga gcccagattt caggattgcc ttccaggagc ttctgtgcct 1980

gcgcaggtct tctttgaagg 2000

<210> 154
<211> 2000
<212> DNA
<213> Homo sapiens

<220>
<223> ADRB3 DNA fragment contaning rs4994

<400> 154
aggaggctgg gacaggaggg tggcttgagc ccgggagttc aaagccagcc tgggaaaaat 60

agagagagag agagacttga aatatggtaa atttcctcag tacctgaaaa tattaaaaaa 120

aacaaaaagc tagaaggaaa gagaaggtaa ctgaaacaga agggaaaggg gacagatctc 180

accaagctga ggtcttggga gaggagatac tggctgagcc ctattactta atttaaaata 240

ccttagggga ggccacccaa gtggatgcgg ggctcctgtg aatcctttgc ttgactccag 300

cgggttacct ttgcctctga tacataaagg gtggggatgg gagcgctctc ctctctcctt 360

cccctgcctt gctgtgggaa cttctgggaa aggaggtgca gggctccagg aagccagtgc 420

ccagggagtg ctatgctgag tccaggagcc tggccacggc aggggtggac agatggtggc 480

agaggaacca cggtgtccct tcctccagat ttagctaaag gaaacgtgga gcatcccatt 540

ggccatcctc cccactctcc aattcggctc cagaggcccc tccagactat aggcagctgc 600

ccctttaagc gtcgctactc ctcccccaag agcggtggca ccgagggagt tggggtgggg 660

ggaggctgag cgctctggct gggacagcta gagaagatgg cccaggctgg ggaagtcgct 720

ctcatgcctt gctgtcccct cccctgagcc aggtgatttg ggagaccccc tccttccttc 780

tttccctacc gccccacgcg cgacccgggg atggctccgt ggcctcacga gaacagctct 840

cttgccccat ggccggacct ccccaccctg gcgcccaata ccgccaacac cagtgggctg 900

ccaggggttc cgtgggaggc ggccctagcc ggggccctgc tggcgctggc ggtgctggcc 960

accgtgggag gcaacctgct ggtcatcgtg gccatcgcct ggactccgag actccagacc 1020

atgaccaacg tgttcgtgac ttcgctggcc gcagccgacc tggtgatggg actcctggtg 1080

gtgccgccgg cggccacctt ggcgctgact ggccactggc cgttgggcgc cactggctgc 1140

gagctgtgga cctcggtgga cgtgctgtgt gtgaccgcca gcatcgaaac cctgtgcgcc 1200

ctggccgtgg accgctacct ggctgtgacc aacccgctgc gttacggcgc actggtcacc 1260

aagcgctgcg cccggacagc tgtggtcctg gtgtgggtcg tgtcggccgc ggtgtcgttt 1320

gcgcccatca tgagccagtg gtggcgcgta ggggccgacg ccgaggcgca gcgctgccac 1380

tccaacccgc gctgctgtgc cttcgcctcc aacatgccct acgtgctgct gtcctcctcc 1440

gtctccttct accttcctct tctcgtgatg ctcttcgtct acgcgcgggt tttcgtggtg 1500

gctacgcgcc agctgcgctt gctgcgcggg gagctgggcc gctttccgcc cgaggagtct 1560

ccgccggcgc cgtcgcgctc tctggccccg gccccggtgg ggacgtgcgc tccgcccgaa 1620

ggggtgcccg cctgcggccg gcggcccgcg cgcctcctgc ctctccggga acaccgggcc 1680

ctgtgcacct tgggtctcat catgggcacc ttcactctct gctggttgcc cttctttctg 1740

gccaacgtgc tgcgcgccct gggggccccc tctctagtcc cgggcccggc tttccttgcc 1800

ctgaactggc taggttatgc caattctgcc ttcaacccgc tcatctactg ccgcagcccg 1860

gactttcgca gcgccttccg ccgtcttctg tgccgctgcg gccgtcgcct gcctccggag 1920

ccctgcgccg ccgcccgccc ggccctcttc ccctcgggcg ttcctgcggc ccggagcagc 1980

ccagcgcagc ccaggctttg 2000

<210>  155
<211>  899
<212>  DNA
<213>  Homo sapiens

<220>
<223>  CYP2C9*3 DNA fragment contaning rs1057910

<400>  155
gtcttgtttt tccatccagg tcagtaacag gtcagtatgt cttttgattg gagattttat      60

tccatttaca ttcagtgtta ttattgataa gtaaggactt acccatgccc ctttgttatt     120

tgttttctgg ttgttttgtg gacttctctt ccttctttca tttcttcctg tcttccttta     180

ttgaagagaa ttttctccac ttatatgtgt acagattttt cttaatatct ggtttatggc     240

agttacacat ttgtgcatct gtaaccatcc tctctttaag tttgcatata cttccagcac     300

tataatttaa atttataatg atgtttggat accttcatga ttcatatacc cctgaattgc     360

tacaacaaat gtgccatttt tctccttttc catcagtttt tacttgtgtc ttatcagcta     420

aagtccagga agagattgaa cgtgtgattg gcagaaaccg gagcccctgc atgcaagaca     480

ggagccacat gccctacaca gatgctgtgg tgcacgaggt ccagagatac attgaccttc     540

tccccaccag cctgccccat gcagtgacct gtgacattaa attcagaaac tatctcattc     600

ccaaggtaag tttgtttctc ctacactgca actccatgtt ttcgaagtcc ccaaattcat     660

agtatcattt ttaaacctct accatcaccg ggtgagagaa gtgcataact catatgtatg     720

gcagtttaac tggactttct cttgtttcca gtttggggct ataaaggttt gtaacaggtc     780

ctagtgtctg gcagtgtgtg ttctccagat ttattatctt tcttcaagat tggtttggct     840

actcttaggt gcttatattt ccaaataatt tttaaaggta ttagtttgtc aatttccca      899


<210>  156
<211>  807
<212>  DNA
<213>  Homo sapiens

<220>
<223>  VKORC1_1 DNA fragment contaning rs9934438

<400>  156
ggagtggggc tgagctgacc aagggggatg aggggcgggg cggggcgggc agggaggggg      60

cggagccact cacctaacaa tagctgtagt gtgtagaaga tgcaaccgaa tatgctgttg     120

gattgattga ggatgctgtc ctgtcccagc acatgctcca ccagcccgaa acccctgccc     180

cacctggcag aggggtgggg tggggtggaa ccaggttagg actgtcaacc cagtgccttg     240

gaccctgccc gagaaaggtg atttccaaga agccacctgg gctatcctct gttccccgac     300

ctcccatcct agtccaaggg tcgatgatct cctggcaccg ggcacctttg gccacgtcag     360

gattccatgt cactgaccct atcctcccct ctccccagac caggcccgga cgtggctact     420

ccgtaggccc tgcttttcat cttagacctt aagtaagtct cttttttttt tttttttttt 480

ttttttttga gacggagtct cactctggcc caggctggag tgcagtggcg cgatctcggc 540

tcactgaaac ctccgcctcc cgggttcaag cgattctcct gccttagccc ccccgagtag 600

ctgggattaa ggcacccgcc atagcgcccg attaattttt ctatttttgg tagagacagg 660

ctttcaccat gttggccagg ctggtcaact cctgacttca agtgatccat ccgcctcggc 720

ctcccaaagt gctgggatta caggagtggg ccaccgcgcc cggcccttaa gtaattctta 780

aaatggcaag gctggtataa cggttca 807

<210> 157
<211> 748
<212> DNA
<213> Homo sapiens

<220>
<223> VKORC1_2 DNA fragment containing rs9923231

<400> 157
ctcccggcat tatcccatct acccctccac attcaagttt ttggaaagat tctacactcc 60

cagtctctac ttcctcactt cttccttgct gcccacgcca taaactagct gctgcctcca 120

gcattgccct gacacctagt ggctggtgtc accaagacgc tagacccaat ggttatttat 180

ttatttattt acttattttg agacggagtc tcactctgtc gcccaggctg gagtgcagcg 240

gtgccatctc ggctcactgc aacttccgcc tccagggttc aagtggttct cgtgcctcag 300

cctcccaagt agtttggact acaggtgcct gccaccatgt ctggctaatt tttgtatttt 360

tagtagagac agggtttcac catgttggcc aggcttgtct taaactcctg acctcaagtg 420

atccacccac ctcggcctcc caaaatgcta ggattatagg cgtgagccac cgcacccggc 480

caatggttgt ttttcaggtc ttctcttgct tgacttccca gagggatccc ttactgttgc 540

acctaccctt ctgggaactc tcttcctctg gcgtctgtga tatttccctc tcctgctggc 600

tcctccctct ccagatgctg tttctcacat ctactctctt ctagagagtg tggtagacag 660

aataatggtc accaaagatg tccctgcatg aatccctgga acttgtgaat atgataggtt 720

aaatggccaa aagggaatta aggttgca 748